# FASCICULE DE BREVET EUROPEEN

(11) **EP 1 953 161 B1**
(45) Date de publication et mention de la délivrance du brevet: **26.08.2009**
(21) Numéro de dépôt: 08290088.7
(22) Date de dépôt: 01.02.2008
(51) Int. Cl.: C07D 471/04, C07D 487/04, A61K 31/498, A61P 35/00

(54) **Nouveaux dérivés tricycliques, leur procédé de préparation et les compositions pharmaceutiques qui les contiennent**
Neue trizyklische Derivate, ihr Herstellungsverfahren und pharmazeutische Zusammensetzungen, die sie enthalten
New tricyclic derivatives, method of preparing same and pharmaceutical compositions containing them

(30) Priorité: 02.02.2007 FR 0700741
(43) Date de publication de la demande: 06.08.2008
(73) Titulaire: Les Laboratoires Servier, 92415 Courbevoie Cedex (FR)
(72) Inventeur: Casara, Patrick, 78670 Villennes sur Seine (FR); Le Diguarher, Thierry, 92500 Rueil Malmaison (FR); Geneste, Olivier, 92500 Rueil Malmaison (FR); Hickman, John, 75017 Paris (FR)

(56) Documents cités:
- WO-A-97/40015
- WO-A-20/04000834
- WO-A-20/04055163
- US-B1- 6 180 629

## Description

La présente invention concerne de nouveaux dérivés tricycliques, leur procédé de préparation et les compositions pharmaceutiques qui les contiennent.

Les composés de la présente invention sont nouveaux et présentent des caractéristiques pharmacologiques très intéressantes dans le domaine de l'apoptose et de la cancérologie.

L'apoptose, ou mort cellulaire programmée, est un processus physiologique crucial pour le développement embryonnaire et le maintien de l'homéostasie tissulaire.

La mort cellulaire de type apoptotique fait intervenir des changements morphologiques, tels que la condensation du noyau, la fragmentation de l'ADN, ainsi que des phénomènes biochimiques, tels que l'activation des caspases qui vont dégrader des composants structuraux clés de la cellule pour induire son désassemblage et sa mort. La régulation du processus d'apoptose est complexe et implique l'activation ou la répression de plusieurs voies de signalisation intracellulaire (Cory S. et al., Nature Review Cancer, 2002, 2, 647-656).

Des dérégulations de l'apoptose sont impliquées dans certaines pathologies. Une apoptose accrue est liée aux maladies neurodégénératives telles que la maladie de Parkinson, la maladie d'Alzheimer et l'ischémie. Inversement, des déficiences dans l'exécution de l'apoptose jouent un rôle important dans le développement des cancers et leur chimiorésistance, des maladies auto-immunes, des maladies inflammatoires et des infections virales. Ainsi, l'échappement à l'apoptose fait partie des signatures phénotypiques du cancer (Hanahan D. et al., Cell 2000, 100, 57-70).

US 6,180,629, WO 2004/000834 et WO 2004/055163 décrivent des composés ayant des propriétés pro-apoptotiques.

Les composés de la présente invention outre leur nouveauté, présentent des propriétés pro-apoptotiques permettant de les utiliser dans les pathologies impliquant un défaut d'apoptose, comme par exemple dans le traitement du cancer.

La présente invention concerne plus particulièrement les composés de formule (I) : 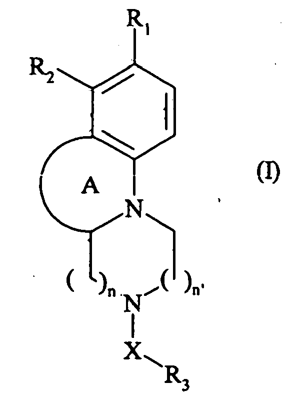 dans laquelle :
◆ A représente un cycle aromatique ou non contenant 5, 6 ou 7 chaînons, et pouvant contenir 1 ou 2 hétéroatomes choisis parmi oxygène, soufre et azote, ce dernier pouvant être substitué par un groupement alkyle (C₁-C₆) linéaire ou ramifié, étant entendu que le cycle A ainsi défini ne peut contenir 2 atomes de soufre ni 2 atomes d'oxygène et que l'un des chaînons peut représenter un groupement C=O,
◆ n et n', identiques ou différents, représentent 0, 1 ou 2, avec 0 < n +n' < 4,
◆ R₃ représente un groupement aryle ou hétéroaryle,
◆ X représente une chaîne alkylène linéaire ou ramifiée contenant de 1 à 6 atomes de carbone, dont un ou deux des atomes de carbone peut(vent) être remplacé(s) par un atome d'oxygène, un groupement cycloalkylène, un groupement arylène, un groupement hétéroarylène ou un groupement SO₂,
◆ un des groupements R₁ ou R₂ représente un atome d'hydrogène et l'autre représente un groupement de formule (II) : 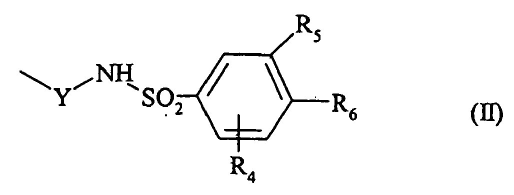
dans laquelle :
- Y représente un groupement C=O ou CH₂,
- R₅ représente un atome d'hydrogène et dans ce cas R₆ représente un atome d'hydrogène ou un groupement -NR₇R'₇ ou -CH₂-NR₇R'₇ dans lesquels R₇ et R'₇, identiques ou différents, représentent chacun indépendamment un atome d'hydrogène ou un groupement alkyle (C₁-C₆) linéaire ou ramifié substitué par un ou plusieurs groupements aryle, hétéroaryle, aryloxy, hétéroaryloxy, arylthio, hétéroarylthio, hétérocycloalkyle, ou -NR₁₀R'₁₀ dans lequel :
   * R₁₀ et R'₁₀, identiques ou différents, sont choisis parmi hydrogène, alkyle (C₁-C₆) linéaire ou ramifié, alkoxy (C₁-C₆) linéaire ou ramifié, aryle et hétéroaryle,
   * ou bien R₁₀ et R'₁₀ forment un groupement cyclique ou bicyclique, saturé ou non, éventuellement substitué par un hétéroatome choisi parmi oxygène, azote ou soufre, étant entendu que un ou plusieurs des chaînons peut (peuvent) représenter un groupement C=O ou peut (peuvent) être substitué(s) comme le prévoit la définition de l'hétérocycle ci-dessous,
   ou R₅ et R₆ forment avec les deux atomes de carbone qui les portent, un cycle aromatique ou non contenant 5 ou 6 chaînons dont un atome d'azote en para du groupement SO₂, et pouvant contenir en plus de l'atome d'azote un autre atome d'azote et/ou un groupement SO₂, le cycle ainsi défini étant substitué par un groupement R₇ tel que défini précédemment,
- R₄ représente un atome d'halogène ou un groupement NO₂, R₈, SO₂-R₉, alkyle (C₁-C₆) linéaire ou ramifié, ou alkoxy (C₁-C₆) linéaire ou ramifié, où R₈ peut prendre toutes les valeurs de R₇ tel que défini précédemment,
- R₉ représente un groupement amino ou un groupement alkyle (C₁-C₆) linéaire ou ramifié éventuellement substitué par un ou plusieurs atomes d'halogène,
étant entendu que :
- par "aryle", on entend un groupement phényle, naphtyle ou biphényle,
- par "hétéroaryle", on entend tout groupement mono ou bi-cyclique, possédant au moins une partie aromatique, et contenant 5 à 10 chaînons et pouvant contenir 1 à 3 hétéroatomes choisis parmi oxygène, soufre ou azote comme les groupements furane, thiophène, pyrrole, imidazoline, pyridine, quinoléine, isoquinoléine, chromane, indole, benzothiophène, benzofurane, 1,3-benzodioxole et 2,3-dihydro-1,4-benzodioxine,
- par "hétérocycloalkyle", on entend tout groupement non aromatique mono ou bicyclique contenant 4 à 10 chaînons et pouvant contenir 1 à 3 hétéroatomes choisis parmi oxygène, soufre ou azote,
- par "cycloalkyle", on entend tout groupement non aromatique mono ou bi-cyclique contenant 4 à 10 chaînons,
les groupements aryle, hétéroaryle, hétérocycloalkyle et cycloalkyle ainsi définis pouvant être substitués par 1 à 3 groupements choisis parmi alkyle (C₁-C₆) linéaire ou ramifié éventuellement substitué par un groupement hydroxy ou amino, alkoxy (C₁-C₆) linéaire ou ramifié, hydroxy, carboxy, formyle, nitro, cyano, amino, polyhalogénoalkyle (C₁-C₆) linéaire ou ramifié, alkyloxycarbonyle, ou atomes d'halogène,
- par "arylène", "hétéroarylène"et "cycloalkylène", on entend un groupement aryle, hétéroaryle et cycloalkyle respectivement tels que définis précédemment, insérés à la place d'un atome de carbone de la chaîne alkylène,
leurs énantiomères et diastéréoisomères, ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Y représente avantageusement un groupement C=O.

La valeur préférée pour n et n' est 1.

Les groupements R₄ préférés sont les groupements NO₂ et SO₂CF₃.

Les groupements X-R₃ préférés sont les groupements ([1,1'-biphényl]-2-yl)méthyl éventuellement substitués par un ou plusieurs groupements choisis parmi halogène, cyano, amino, aminométhyl, trifluorométhyl.

R₅ représente de façon préférentielle un atome d'hydrogène.

Les groupements R₇ préférés sont les groupements 1-(*N*,*N*-diméthylamino)-4-(phénylsulfanyl)-butan-3-yle et 1-(NR₁₀R'₁₀)-4-(phénylsulfanyl)-butan-3-yle, R₁₀ et R'₁₀ étant tels qu'ils forment un groupement cyclique ou bicyclique, saturé ou non, éventuellement substitué par un hétéroatome choisi parmi oxygène, azote ou soufre.

R'₇ représente avantageusement un atome d'hydrogène.

Le groupement 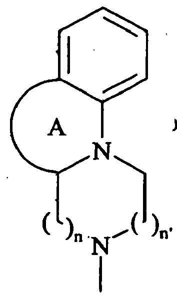 préféré est le groupement

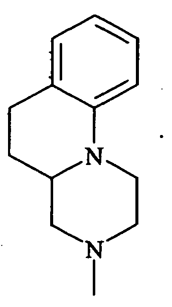

Encore plus particulièrement, l'invention concerne les composés de formule (I) qui sont :
- le *N*-({(4a*S*,*R*)-3-[(4'-chloro[1,1'-biphényl]-2-yl)méthyl]-2,3,4,4a,5,6-hexahydro-1*H-*pyrazino[1,2-*a*]quinolin-8-yl}carbonyl)-4-({(1*R*)-3-(diméthylamino)-1-[(phényl sulfanyl)méthyl]propyl}amino)-3-nitrobenzènesulfonamide,
- le *N*-({(4a*S*,*R*)-3-[(4'-chloro[1,1'-biphényl]-2-yl)méthyl]-2,3,4,4a,5,6-hexahydro-1*H-*pyrazino[1,2-*a*]quinolin-8-yl}carbonyl)-4-({(1*R*)-3-(diméthylamino)-1-[(phényl sulfanyl)méthyl]propyl}amino)-3-nitrobenzènesulfonamide, bistrifluoroacétate,
- le *N*-({(4a*S*,*R*)-3-[(4'-chloro[1,1'-biphényl]-3-yl)méthyl]-2,3,4,4a,5,6-hexahydro-1*H-*pyrazino[1,2-*a*]quinolin-8-yl}carbonyl)-3-nitro-4-{[2-(phénylsulfanyl)éthyl] amino}benzènesulfonamide,
- le *N*-({(4a*S*,*R*)-3-[(4'-chloro[1,1'-biphényl]-3-yl)méthyl]-2,3,4,4a,5,6-hexahydro-1*H-*pyrazino[1,2-*a*]quinolin-8-yl}carbonyl)-3-nitro-4-{[2-(phénylsulfanyl)éthyl] amino}benzènesulfonamide, chlorhydrate,
- le *N*-({(4a*S*,*R*)-3-[(4'-chloro[1,1'-biphényl]-4-yl)méthyl]-2,3,4,4a,5,6-hexahydro-1*H-*pyrazino[1,2-*a*]quinolin-8-yl}carbonyl)-3-nitro-4-{[2-(phénylsulfanyl)éthyl] amino}benzènesulfonamide,
- le *N*-({(4a*S,R*)-3-[(4'-chloro[1,1'-biphényl]-4-yl)méthyl]-2,3,4,4a,5,6-hexahydro-1*H-*pyrazino[1,2-*a*]quinolin-8-yl}carbonyl)-3-nitro-4-{[2-(phénylsulfanyl) éthyl]amino}benzènesulfonamide, chlorhydrate,
- le *N*-{[(4a*S*,*R*)-3-([1,1'-biphényl]-2-ylméthyl)-2,3,4,4a,5,6-hexahydro-1*H-*pyrazino[1,2-*a*]quinolin-8-yl]carbonyl}-4-({(1*R*)-3-(diméthylamino)-1-[(phényl sulfanyl)méthyl]propyl}amino)-3-nitrobenzènesulfonamide,
- le *N*-{[(4a*S*,*R*)-3-([1,1'-biphényl]-2-ylméthyl)-2,3,4,4a,5,6-hexahydro-1*H-*pyrazino[1,2-*a*]quinolin-8-yl]carbonyl}-4-({(1*R*)-3-(diméthylamino)-1-[(phényl sulfanyl)méthyl]propyl} amino)-3-nitrobenzènesulfonamide, bis(chlorhydrate),
- le *N*-{[(4a*S*,*R*)-3-(2-benzylbenzyl)-2,3,4,4a,5,6-hexahydro-1*H*-pyrazino[1,2-*a*]quinolin-8-yl]carbonyl}-3-nitro-4-{[2-(phénylsulfanyl)éthyl]amino}benzènesulfonamide,
- le *N*-{[(4a*S*,*R*)-3-(2-benzylbenzyl)-2,3,4,4a,5,6-hexahydro-1*H*-pyrazino[1,2-*a*] quinolin-8-yl]carbonyl}-3-nitro-4-{[2-(phénylsulfanyl)éthyl]amino}benzènesulfonamide, chlorhydrate,
- le 3-nitro-*N*-({(4a*S*,*R*)-3-[2-(2-phényléthyl)benzyl]-2,3,4,4a,5,6-hexahydro-1*H-*pyrazino[1,2-*a*]quinolin-8-yl}carbonyl)-4-{[2-(phénylsulfanyl)éthyl]amino} benzènesulfonamide,
- le *N*-({(4a*S,R*)-3-[(4'-chloro[1,1'-biphényl]-2-yl)méthyl]-2,3,4,4a,5,6-hexahydro-1*H-*pyrazino[1,2-*a*]quinolin-8-yl}carbonyl)-3-nitro-4-{[2-(phénylsulfanyl)éthyl] amino}benzènesulfonamide,
- le *N*-({(4a*S,R*)-3-[(4'-chloro[1,1'-biphényl]-2-yl)méthyl]-2,3,4,4a,5,6-hexahydro-1*H-*pyrazino[1,2-*a*]quinolin-8-yl}carbonyl)-3-nitro-4-[(2-phénoxyéthyl)amino] benzènesulfonamide,
- le *N*-{[(4a*S,R*)-3-([1,1'-biphényl]-2-ylméthyl)-2,3,4,4a,5,6-hexahydro-1*H*-pyrazino [1,2-*a*]quinolin-8-yl]carbonyl}-3-nitro-4-[(3-phénylpropyl)amino]benzènesulfonamide,
- le 4-[(2-anilinoéthyl)amino]-*N*-{[(4a*S,R*)-3-([1,1'-biphényl]-2-ylméthyl)-2,3,4,4a,5,6-hexahydro-1*H*-pyrazino[1,2-*a*]quinolin-8-yl]carbonyl}-3-nitrobenzènesulfonamide,
- le *N*-{[(4a*S*,*R*)-3-([1,1'-biphényl]-2-ylméthyl)-2,3,4,4a,5,6-hexahydro-1*H*-pyrazino [1,2-*a*]quinolin-8-yl]carbonyl}-4-{[3-(diméthylamino)propyl][2-(phénylsulfanyl) éthyl]amino}-3-nitrobenzènesulfonamide,
- le *N*-([(4a*S*,*R*)-3-([1,1'-biphényl]-2-ylméthyl)-2,3,4,4a,5,6-hexahydro-1*H*-pyrazino [1,2-*a*]quinolin-8-yl]carbonyl}-4-{[3-(diméthylamino)propyl][2-(phénylsulfanyl) éthyl]amino}-3-nitrobenzènesulfonamide, chlorhydrate,
- le *N*-({(4a*S,R*)-3-[(4'-chloro[1,1'-biphényl]-2-yl)méthyl]-2,3,4,4a,5,6-hexahydro-1*H-*pyrazino[1,2-*a*]quinolin-8-yl}carbonyl)-4-{[3-(diméthylamino)propyl]amino}-3-nitrobenzènesulfonamide,
- le *N*-({(4a*S,R*)-3-[(4'-chloro[1,1'-biphényl]-2-yl)méthyl]-2,3,4,4a,5,6-hexahydro-1*H-*pyrazino[1,2-*a*]quinolin-8-yl}carbonyl)benzènesulfonamide,
- le 4-{[(2-aminoéthyl)(2-phényléthyl)amino]méthyl}-*N*-({(4a*S*,*R*)-3-[(4'-chloro[1,1'-biphényl]-2-yl)méthyl]-2,3,4,4a,5,6-hexahydro-1*H*-pyrazino[1,2-*a*]quinolin-8-yl} carbonyl)benzènesulfonamide,
- le 4-{[(2-aminoéthyl)(2-phényléthyl)amino]méthyl}-*N*-({(4a*S*,*R*)-3-[(4'-chloro[1,1'-biphényl]-2-yl)méthyl]-2,3,4,4a,5,6-hexahydro-1*H*-pyrazino[1,2-*a*]quinolin-8-yl} carbonyl)benzènesulfonamide tris(trifluoroacétate),
- le *N*-({(4a*S,R*)-3-[(4'-chloro[1,1'-biphényl]-2-yl)méthyl]-2,3,4,4a,5,6-hexahydro-1*H-*pyrazino[1,2-*a*]quinolin-7-yl}carbonyl)-4-({(1*R*)-3-(diméthylamino)-1-[(phényl sulfanyl)méthyl]propyl}amino)-3-nitrobenzènesulfonamide,
- le *N*-{[(4a*S,R*)-3-([1,1'-biphényl]-2-ylméthyl)-2,3,4,4a,5,6-hexahydro-1*H*-pyrazino [1,2-*a*]quinolin-7-yl]carbonyl}-4-({(1*R*)-3-(diméthylamino)-1-[(phénylsulfanyl) méthyl]propyl}amino)-3-nitrobenzènesulfonamide,
- le *N*-{[2-([1,1'-biphényl]-2-ylméthyl)-1,2,3,4-tétrahydropyrazino[1,2-*a*]indol-8-yl] carbonyl}-4-({(1*R*)-3-(diméthylamino)-1-[(phénylsulfanyl)méthyl]propyl}amino)-3-nitrobenzènesulfonamide,
- le *N*-{[2-([1,1'-biphényl]-2-ylméthyl)-1,2,3,4-tétrahydropyrazino[1,2-*a*]indol-8-yl] carbonyl}-4-({(1*R*)-3-(diméthylamino)-1-[(phénylsulfanyl)méthyl]propyl}amino)-3-nitrobenzènesulfonamide, bis(chlorhydrate),
- le *N*-({2-[(4'-chloro[1,1'-biphényl]-2-yl)méthyl]-1,2,3,4-tétrahydro pyrazino[1,2-*a*] indol-8-yl}carbonyl)-4-({(1*R*)-3-(diméthylamino)-1-[(phénylsulfanyl)méthyl] propyl}amino)-3-nitrobenzènesulfonamide,
- le *N*-({2-[(4'-chloro[1,1'-biphényl]-2-yl)méthyl]-1,2,3,4-tétrahydro pyrazino[1,2-*a*] indol-8-yl}carbonyl)-4-({(1*R*)-3-(diméthylamino)-1-[(phénylsulfanyl)méthyl] propyl}amino)-3-nitrobenzènesulfonamide, bis(chlorhydrate),
- le *N*-({(10a*S*,*R*)-2-[(4'-chloro[1,1'-biphényl]-2-yl)méthyl]-1,2,3,4,10,10a-hexahydro pyrazino[1,2-*a*]indol-8-yl}carbonyl)-4-({(1*R*)-3-(diméthylamino)-1-[(phényl sulfanyl)méthyl]propyl}amino)-3-nitrobenzènesulfonamide,
- le *N*-({(4a*R*)-3-[(4'-chloro[1,1'-biphényl]-2-yl)méthyl]-2,3,4,4a,5,6-hexahydro-1*H-*pyrazino[1,2-*a*]quinolin-8-yl}carbonyl)-4-({(1*R*)-3-(diméthylamino)-1-[(phényl sulfanyl)méthyl]propyl}amino)-3-nitrobenzènesulfonamide,
- le *N*-({(4a*R*)-3-[(4'-chloro[1,1'-biphényl]-2-yl)méthyl]-2,3,4,4a,5,6-hexahydro-1*H-*pyrazino[1,2-*a*]quinolin-8-yl}carbonyl)-4-({(1*R*)-3-(diméthylamino)-1-[(phényl sulfanyl)méthyl]propyl}amino)-3-nitrobenzènesulfonamide, bis(chlorhydrate),
- le *N*-({(4a*S*)-3-[(4'-chloro[1,1'-biphényl]-2-yl)méthyl]-2,3,4,4a,5,6-hexahydro-1*H-*pyrazino[1,2-α]quinolin-8-yl}carbonyl)-4-({(1*R*)-3-(diméthylamino)-1-[(phényl sulfanyl)méthyl]propyl}amino)-3-nitrobenzènesulfonamide,
- le *N*-({(4a*S*)-3-[(4'-chloro[1,1'-biphényl]-2-yl)méthyl]-2,3,4,4a,5,6-hexahydro-1*H-*pyrazino[1,2-α]quinolin-8-yl}carbonyl)-4-({(1*R*)-3-(diméthylamino)-1-[(phényl sulfanyl)méthyl]propyl}amino)-3-nitrobenzènesulfonamide, bis(chlorhydrate),
- le *N*-{[(4a*S*)-3-([1,1'-biphényl]-2-ylméthyl)-2,3,4,4a,5,6-hexahydro-1*H*-pyrazino [1,2-α]quinolin-8-yl]carbonyl}-4-({(1*R*)-3-(diméthylamino)-1-[(phénylsulfanyl) méthyl]propyl} amino)-3-nitrobenzènesulfonamide,
- le *N-*{[(4a*S*)-3-([1,1'-biphényl]-2-ylméthyl)-2,3,4,4a,5,6-hexahydro-1*H*-pyrazino [1,2-*a*]quinolin-8-yl]carbonyl}-4-({(1*R*)-3-(diméthylamino)-1-[(phénylsulfanyl) méthyl]propyl}amino)-3-nitrobenzènesulfonamide, bis(chlorhydrate),
- le *N-*{[(4a*R*)-3-([1,1'-biphényl]-2-ylméthyl)-2,3,4,4a,5,6-hexahydro-1*H*-pyrazino [1,2-*a*]quinolin-8-yl]carbonyl]-4-({(1*R*)-3-(diméthylamino)-1-[(phénylsulfanyl) méthyl]propyl}amino)-3-nitrobenzènesulfonamide,
- le *N-*{[(4a*R*)-3-([1,1'-biphényl]-2-ylméthyl)-2,3,4,4a,5,6-hexahydro-1*H*-pyrazino [1,2-*a*]quinolin-8-yl]carbonyl}-4-({(1*R*)-3-(diméthylamino)-1-[(phénylsulfanyl) méthyl]propyl}amino)-3-nitrobenzènesulfonamide, tris(chlorhydrate),
- le *N*-{[(4a*S*,*R*)-3-([1,1'-biphényl]-2-ylsulfonyl)-2,3,4,4a,5,6-hexahydro-1*H-*pyrazino[ 1,2-*a*]quinolin-8-yl]carbonyl}-3-nitro-4-{[2-(phénylsulfanyl)éthyl] amino}benzènesulfonamide,
- le *N*-({(4a*S*,*R*)-3-[(4'-chloro[1,1'-biphényl]-2-yl)méthyl]-2,3,4,4a,5,6-hexahydro-1*H-*pyrazino[1,2-*a*]quinolin-8-yl)carbonyl)-4-{(1*R*)-3-(diméthylamino)-1-[(phényl sulfanyl)méthyl]propyl}-3,4-dihydro-2*H*-1,2,4-benzothiadiazine-7-sulfonamide 1,1-dioxide,
- le *N*-({(4a*S,R*)-3-[(4'-chloro[1,1'-biphényl]-2-yl)méthyl]-2,3,4,4a,5,6-hexahydro-1*H-*pyrazino[1,2-*a*]quinolin-8-yl}carbonyl)-4-{(1*R*)-3-(diméthylamino)-1-[(phényl sulfanyl)méthyl]propyl}-4*H*-1,2,4-benzothiadiazine-7-sulfonamide 1,1-dioxide,
- *N*-({(10aα)-2-[(4'-Chloro[1,1'-biphényl]-2-yl)méthyl]-1,2,3,4,10,10a-hexahydropyrazino[1,2-*a*]indol-8-yl}carbonyl)-4-({(1*R*)-3-(diméthylamino)-1-[(phénylsulfanyl)méthyl]propyl}amino)-3-nitrobenzènesulfonamide, bischlorhydrate,
- *N*-({(10aβ)-2-[(4'-Chloro[1,1'-biphényl]-2-yl)méthyl]-1,2,3,4,10,10a-hexahydropyrazino[ 1,2-α]indol-8-yl}carbonyl)-4-({(1*R*)-3-(diméthylamino)-1-[(phénylsulfanyl)méthyl]propyl}amino)-3-nitrobenzènesulfonamide, bischlorhydrate,
- *N*-({(10aα)-2-[(4'-chloro[1,1'-biphényl]-2-yl)méthyl]-1,2,3,4,10,10a-hexahydropyrazino[ 1,2-*a*]indol-8-yl}carbonyl)-4-({(1*R*)-3-(4-morpholinyl)-1-[(phénylsulfanyl)méthyl]propyl}amino)-3-nitrobenzènesulfonamide, bischlorhydrate,
- *N*-({(10aα)-2-[(4'-chloro[1,1'-biphényl]-2-yl)méthyl]-1,2,3,4,10,10a-hexahydropyrazino[1,2-*a*]indol-8-yl}carbonyl)-4-({(1*R*)-3-(4-morpholinyl)-1-[(phénylsulfanyl)méthyl]propyl}amino)-3-[(trifluorométhyl)sulfonyl]benzènesulfonamide, bischlorhydrate,
- *N*-[((4a*R*)-3-{[2-(4-chlorophényl)-5,5-diméthyl-1-cyclohexén-1-yl]méthyl}-2,3,4,4a,5,6-hexahydro-1H-pyrazino[1,2-*a*]quinolin-8-yl)carbonyl]-4-({(1*R*)-3-(4-morpholinyl)-1-[(phénylsulfanyl)méthyl]propyl}amino)-3-[(trifluorométhyl) sulfonyl]benzènesulfonamide, bischlorhydrate,
- *N*-[((10aβ)-2-{[2-(4-chlorophényl)-5,5-diméthyl-1-cyclohexén-1-yl]méthyl}-1,2,3,4,10,10a-hexahydropyrazino[1,2-*a*]indol-8-yl)carbonyl]-4-({(1*R*)-3-(4-morpholinyl)-1-[(phénylsulfanyl)méthyl]propyl}amino)-3-[(trifluorométhyl) sulfonyl]benzènesulfonamide, bischlorhydrate,
- *N*-[((4a*R*)-3-{[4-(4-chlorophényl)-3-pyridyl]méthyl}-2,3,4,4a,5,6-hexahydro-1H-pyrazino[1,2-*a*]quinolin-8-yl)carbonyl]-4-({(1*R*)-2-(diméthylamino)-1-[(phénylsulfanyl)méthyl]éthyl}amino)-3-nitrobenzènesulfonamide, trichlorhydrate,
- *N*-({(4a*R*)-3-[(4-amino-4'-chloro[1,1'-biphényl]-2-yl)méthyl]-2,3,4,4a,5,6-hexahydro-1*H*-pyrazino[1,2-*a*]quinolin-8-yl}carbonyl)-4-({(1*R*)-3-(diméthylamino)-1-[(phénylsulfanyl)méthyl]propyl} amino)-3-nitrobenzènesulfonamide, trichlorhydrate,
- *N*-[((4a*R*)-3-{[4-(aminométhyl)-4'-chloro[1,1'-biphényl]-2-yl]méthyl}-2,3,4,4a,5,6-hexahydro-1*H*-pyrazino[1,2-*a*]quinolin-8-yl)carbonyl]-4-({(1*R*)-3-(diméthylamino)-1-[(phénylsulfanyl)méthyl]propyl} amino)-3-nitrobenzènesulfonamide, trichlorhydrate,
- *N*-[((4a*R*)-3-{[3'-fluoro-4'-chloro[1,1'-biphényl]-2-yl]méthyl}-2,3,4,4a,5,6-hexahydro-1*H*-pyrazino[1,2-*a*]quinolin-8-yl)carbonyl]-4-({(1*R*)-3-(diméthylamino)-1-[(phénylsulfanyl)méthyl]propyl}amino)-3-nitrobenzènesulfonamide, bischlorhydrate,
- *N*-[((4a*R*)-3-{[4'-(trifluorométhyl)[1,1'-biphényl]-2-yl]méthyl}-2,3,4,4a,5,6-hexahydro-1*H*-pyrazino[1,2-*a*]quinolin-8-yl)carbonyl]-4-({(1*R*)-3-(diméthylamino)-1-[(phénylsulfanyl)méthyl]propyl}amino)-3-nitrobenzènesulfonamide, bischlorhydrate,
- *N*-[((4a*R*)-3-{[4'-cyano[1,1'-biphényl]-2-yl]méthyl}-2,3,4,4a,5,6-hexahydro-1*H-*pyrazino[1,2-*a*]quinolin-8-yl)carbonyl]-4-({(1*R*)-3-(diméthylamino)-1-[(phénylsulfanyl)méthyl]propyl}amino)-3-nitrobenzènesulfonamide, bischlorhydrate,
- *N*-({(4a*R*)-3-[2-(1,3-benzodioxol-5-yl)benzyl]-2,3,4,4a,5,6-hexahydro-1*H-*pyrazino[1,2-*a*]quinolin-8-yl}carbonyl)-4-({(1*R*)-3-(diméthylamino)-1-[(phénylsulfanyl)méthyl]propyl}amino)-3-nitrobenzènesulfonamide, bischlorhydrate,
- *N*-({(4a*R*)-3-[(4'-chloro[1,1'-biphényl]-2-yl)méthyl]-2,3,4,4a,5,6-hexahydro-1*H-*pyrazino[1,2-*a*]quinolin-8-yl}carbonyl)-4-({(1*R*)-3-(4-morpholinyl)-1-[(phénylsulfanyl)méthyl]propyl}amino)-3-nitrobenzènesulfonamide, bischlorhydrate,
- *N*-({(4a*R*)-3-[(4'-chloro[1,1'-biphényl]-2-yl)méthyl]-2,3,4,4a,5,6-hexahydro-1*H-*pyrazino[1,2-*a*]quinolin-8-yl}carbonyl)-4-({(1*R*)-3-(4-morpholinyl)-1-[(phénylsulfanyl)méthyl]propyl}amino)-3-[(trifluorométhyl)sulfonyl] benzènesulfonamide, bischlorhydrate,
- *N*-({(4a*R*)-3-[(4'-chloro[1,1'-biphényl]-2-yl)méthyl]-2,3,4,4a,5,6-hexahydro-1*H-*pyrazino[1,2-*a*]quinolin-8-yl}carbonyl)-4-({(1*R*)-3-(4-méthyl-l-pipérazinyl)-1-[(phénylsulfanyl)méthyl]propyl}amino)-3-nitrobenzènesulfonamide, bischlorhydrate,
- *N*-({(4a*R*)-3-[(4'-chloro[1,1'-biphényl]-2-yl)méthyl]-2,3,4,4a,5,6-hexahydro-1*H-*pyrazino[1,2-*a*]quinolin-8-yl}carbonyl)-4-({(1*R*)-3-(1-pipéridyl)-1-[(phénylsulfanyl)méthyl]propyl}amino)-3-nitrobenzènesulfonamide, bischlorhydrate,
- *N*-({(4a*R*)-3-[(4'-chloro[1,1'-biphényl]-2-yl)méthyl]-2,3,4,4a,5,6-hexahydro-1*H-*pyrazino[1,2-*a*]quinolin-8-yl}carbonyl)-4-({(1*R*)-3-(1-pyrrolidinyl)-1-[(phénylsulfanyl)méthyl]propyl}amino)-3-nitrobenzènesulfonamide, bischlorhydrate,
- *N*-({(4a*R*)-3-[(4'-chloro[1,1'-biphényl]-2-yl)méthyl]-2,3,4,4a,5,6-hexahydro-1*H-*pyrazino[1,2-*a*]quinolin-8-yl}carbonyl)-4-({(1*R*)-3-(3,6-dihydro-1(2*H*)-pyridyl)-1-[(phénylsulfanyl)méthyl]propyl}amino)-3-nitrobenzènesulfonamide, bischlorhydrate,
- *N*-({(4a*R*)-3-[(4'-chloro[1,1'-biphényl]-2-yl)méthyl]-2,3,4,4a,5,6-hexahydro-1*H-*pyrazino[1,2-*a*]quinolin-8-yl}carbonyl)-4-({(1*R*)-3-(1-azépanyl)-1-[(phénylsulfanyl)méthyl]propyl} amino)-3-nitrobenzènesulfonamide, bischlorhydrate,
- *N*-({(4a*R*)-3-[(4'-chloro[1,1'-biphényl]-2-yl)méthyl]-2,3,4,4a,5,6-hexahydro-1*H-*pyrazino[1,2-*a*]quinolin-8-yl}carbonyl)-4-({(1*R*)-3-((1*R*,5*S*)-3-azabicyclo[3.1.0]hex-3-yl)-1-[(phénylsulfanyl)méthyl]propyl} amino)-3-nitrobenzènesulfonamide, bischlorhydrate,
- le *N*-({(4a*R*)-3-[(4'-chloro[1,1'-biphényl]-2-yl)méthyl]-2,3,4,4a,5,6-hexahydro-1*H-*pyrazino[1,2-*a*]quinolin-8-yl}carbonyl)-4-({(1*R*)-3-(diméthylamino)-1-[(Phényl sulfanyl)méthyl]propyl} amino)-3-nitrobenzènesulfonamide,sodium,

Les énantiomères, diastéréoisomères ainsi que les sels d'addition à un acide ou à une base pharmaceutiquement acceptable des composés préférés de l'invention font partie intégrante de l'invention.

L'invention s'étend également au procédé de préparation des composés de formule (I) caractérisé en ce que l'on utilise comme produit de départ le composé de formule (III) : 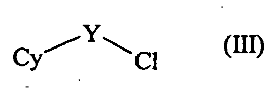 dans laquelle Y est tel que défini dans la formule (I) et Cy représente un système tricyclique fusionné de formule (IV) : 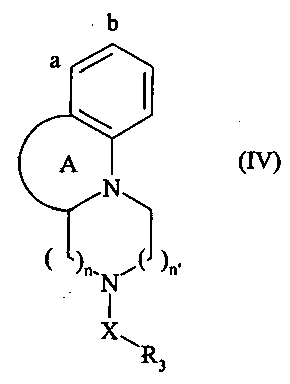 dans laquelle A, X, n, n' et R₃, sont tels que définis dans la formule (I), le groupement -Y-Cl étant rattaché en position a ou b du système tricyclique ainsi défini, composé de formule (III) sur lequel on condense en milieu basique en présence ou non d'un agent de couplage, le composé de formule (V) : 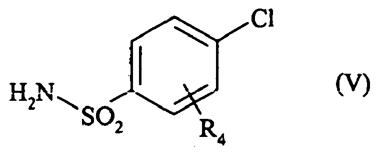 dans laquelle R₄ est tel que défini dans la formule (I),
pour obtenir le composé de formule (VI) : 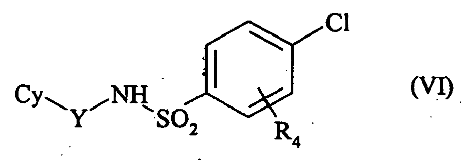 dans laquelle Cy, Y et R₄ sont tels que définis précédemment,
sur lequel on condense le composé de formule HNR₇R'₇ dans laquelle R₇ et R'₇ sont tels que définis dans la formule (I) pour conduire au composé de formule (I/a), cas particulier des composés de formule (I) : 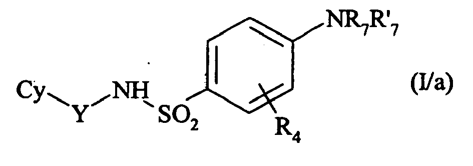 dans laquelle Cy, Y, R₄, R₇ et R'₇ sont tels que définis précédemment,
qui peut être purifié selon une technique classique de séparation, que l'on transforme, si on le souhaite, en ses sels d'addition à un acide ou à une base pharmaceutiquement acceptable et dont on sépare éventuellement les isomères selon une technique classique de séparation.

Les composés de formules (III) et (V) sont soit commerciaux, soit accessibles à l'homme du métier par des réactions chimiques classiques et décrites dans la littérature.

Une variante avantageuse concerne le procédé de préparation des composés de formule (I) caractérisé en ce que l'on utilise comme produit de départ le composé de formule (III') : 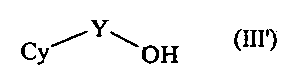 dans laquelle Y est tel que défini dans la formule (I) et Cy représente un système tricyclique fusionné de formule (IV) : 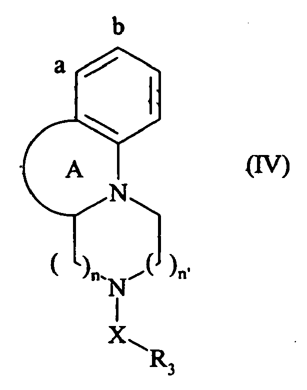 dans laquelle A, X, R₃, n et n' sont tels que définis dans la formule (I), le groupement -Y-OH étant rattaché en position a ou b du système tricyclique ainsi défini,
composé de formule (III') sur lequel on condense en milieu basique en présence d'un agent de couplage, le composé de formule (VII) : 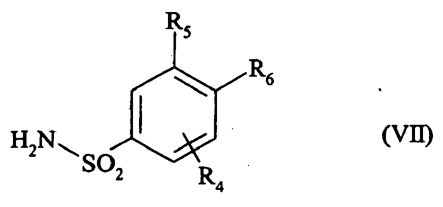 dans laquelle R₄, R₅ et R₆ sont tels que définis dans la formule (I),
pour conduire au composé de formule (I) qui peut être purifié selon une technique classique de séparation, que l'on transforme, si on le souhaite, en ses sels d'addition à un acide ou à une base pharmaceutiquement acceptable et dont on sépare éventuellement les isomères selon une technique classique de séparation.

Les composés de formule (III') et (VII) sont soit commerciaux, soit accessibles à l'homme du métier par des réactions chimiques classiques et décrites dans la littérature.

L'étude pharmacologique des dérivés de l'invention a montré qu'ils possédaient des propriétés pro-apoptotiques. La capacité à réactiver le processus apoptotique dans les cellules cancéreuses représente un intérêt thérapeutique majeur dans le traitement des cancers.

Plus particulièrement, les composés selon l'invention seront utiles dans le traitement des cancers chimio ou radiorésistants, ainsi que dans les hémopathies malignes et le cancer du poumon à petites cellules.

Parmi les traitements des cancers envisagés on peut citer, sans s'y limiter, les cancers de la vessie, du cerveau, du sein, de l'utérus, les leucémies lymphoïdes chroniques, les cancers du colon, de l'oesophage, du foie, les leucémies lymphoblastiques, les lymphomes folliculaires, les mélanomes, les hémopathies malignes, les myélomes, les cancers de l'ovaire, les cancers du poumon non à petites cellules, les cancers de la prostate et les cancers du poumon à petites cellules.

La présente invention a également pour objet les compositions pharmaceutiques contenant au moins un composé de formule (I) seul ou en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,01 mg et 1 g par 24 heures en une ou plusieurs prises.

Par ailleurs, la présente invention concerne également l'association d'un composé de formule (I) avec un agent anticancéreux choisi parmi les agents génotoxiques, les poisons mitotiques, les anti-métabolites, les inhibiteurs du protéasome, ou les inhibiteurs de kinase, ainsi que l'utilisation de ce type d'association pour la fabrication de médicaments utiles dans le traitement du cancer.

Les composés de l'invention peuvent également être utilisés en association avec une radiothérapie dans le traitement du cancer.

Les Préparations et Exemples suivants illustrent l'invention et ne la limitent en aucune façon.

### Préparation 1 : Acide (4aS,R)-3-[(4'-chloro[1,1'-biphényl]-2-yl)méthyl]-2,3,4,4a,5,6-bexabydro-1H-pyrazino[1,2-a]quinoline-8-carboxylique, chlorhydrate

### Stade A : 6-Méthoxy-2-quinolinecarbaldéhyde

A une solution de 6-méthoxy-2-méthylquinoline (42 g) dans 400 ml du mélange dioxane/H₂O (5 %) on ajoute par portions de l'oxyde de sélénium, puis l'ensemble est chauffé à reflux durant une nuit. On laisse refroidir on filtre le métal et on concentre à sec. Le solide marron foncé obtenu est purifié par chromatographie sur colonne de silice (Heptane/AcOEt 80/20) pour conduire au produit du titre sous la forme d'un solide blanc.

### Stade B : 2-{[(6-Méthoxy-2-quinolinyl)méthyl]amino}éthanol

A une suspension du composé obtenu au Stade A (28 g) dans 200 ml de EtOH on ajoute 9,5 ml de 3-aminopropanol et l'ensemble est porté à reflux sous Dean-Stark durant une nuit. Le milieu réactionnel est ensuite concentré à sec puis repris avec un volume de 200 ml de EtOH placé à 0°C, et on ajoute 14 g de NaBH₄ par portions. L'ensemble est ensuite chauffé à reflux une nuit. Le milieu réactionnel est ensuite concentré à sec, hydrolysé avec H₂O et extrait avec CH₂Cl₂. Après séchage sur MgSO₄, concentration à sec, on obtient une huile qui cristallise progressivement. Les cristaux sont ensuite triturés dans le diisopropyl ether, filtrés et séchés pour conduire au produit du titre sous la forme de cristaux beiges.

### Stade C : 2-{[(6-Méthoxy-1,2,3,4-tétrahydro-2-quinolinyl)méthyl]amino}éthanol

A une solution du composé obtenu au Stade B (33 g) dans un mélange 50/50 MeOH/KOH 1M (1,21), on ajoute par portions 169 g de nickel de Raney. L'ensemble est ensuite agité à température ambiante une nuit. Le métal est ensuite filtré et le filtrat concentré à sec. Le résidu est ensuite repris avec CH₂Cl₂, hydrolysé avec H₂O puis extrait plusieurs fois avec CH₂Cl₂. Les extraits sont ensuite regroupés, séchés sur MgSO₄, filtrés et concentrés à sec. Les cristaux obtenus sont triturés dans le diisopropyl ether, filtrés et séchés pour conduire au produit du titre sous la forme de cristaux blancs.

### Stade D : (4aS,R)- 8-Méthoxy-2,3,4,4a,5,6-hexahydro-1H-pyrazino[1,2-a]quinoline

A une suspension du composé obtenu au Stade C (10 g) dans 200 ml d'o-xylène, on ajoute P₂O₅ (18g). L'ensemble est ensuite chauffé à 150°C une nuit. On laisse refroidir, on concentre à sec puis on hydrolyse lentement à froid avec H₂O, puis on ajoute lentement à froid NaOH 5N et l'ensemble est agité à température ambiante 30 minutes. Le milieu réactionnel est ensuite extrait plusieurs fois avec CH₂Cl₂, séché sur MgSO₄ et concentré à sec. On obtient une huile brune correspondant au produit du titre qui est utilisée directement sans purification pour l'étape suivante.

### Stade E : (4aS,R)-3-Benzyl-8-méthoxy-2,3,4,4a,5,6-hexahydro-1H-pyrazino[1,2-a] quinoline

Le composé obtenu au Stade D (7 g) est solubilisé dans 100 ml de DMF, puis on ajoute successivement 8,85 g de K₂CO₃, 4,2 ml de bromure de benzyle et 100 mg de NaI et l'ensemble est chauffé à 80°C pendant 2 h. On concentre à sec et on reprend avec AcOEt. On lave la phase organique avec H₂O puis avec une solution saturée de LiCI puis avec une solution saturée de NaCl. La phase organique est séchée sur MgSO₄ et concentrée à sec. Le résidu est purifié par chromatographie sur colonne de silice (Heptane/AcOEt 95/5) pour conduire au produit du titre sous la forme d'un solide blanc crémeux.

### Stade F : (4aS,R)-3-Benzyl-2,3,4,4a,5,6-hexahydro-1H-pyrazino[1,2-a]quinolin-8-yl trifluoro méthanesulfonate

A une solution du composé obtenu au Stade E (5 g) dans 100 ml de CH₂Cl₂ placée à 0°C on ajoute une solution de BBr₃/CH₂Cl₂ 1M puis l'ensemble est agité progressivement à température ambiante. La température et l'agitation sont maintenues durant une nuit. Puis on se place à 0°C et on ajoute lentement 50 mL de MeOH et le mélange est agité à température ambiante pendant 30 minutes. Le milieu réactionnel est ensuite concentré à sec repris plusieurs fois avec du diisopropyléther. Les cristaux beiges obtenus sont alors filftrés et séchés. Ceux si sont ensuite solubilisés dans 100 ml de CH₂Cl₂, on ajoute goutte à goutte 11,6 ml de Et₃N et le donneur de triflate (8,84 g), puis l'ensemble est agité à température ambiante. On hydrolyse avec H₂O puis on extrait 2 fois avec CH₂Cl₂. Les extraits organiques sont regroupés, séchés sur MgSO₄ et concentrés à sec. Le résidu est ensuite purifié par chromatographie sur colonne de silice (Heptane/AcOEt 9/1) pour conduire au produit du titre sous la forme de cristaux blancs crémeux.

### Stade G : (4aS,R)-3-Benzyl-2,3,4,4a,5,6-hexahydro-1H-pyrazino[1,2-a]quinoline-8-carboxylate de méthyle

Le composé obtenu au Stade F (3,6 g) est solubilisé dans 100 ml d'un mélange DMSO/MeOH (3/2) puis on ajoute successivement 2,6 ml de Et₃N, 0,19 g de Pd(OAc)₂ et 0,935 g de ligand dppf, on dégaze sous argon 20 minutes puis on fait buller le monoxyde de carbone pendant 30 minutes puis on sature avec le monoxyde de carbone pendant 15 minutes. L'ensemble est ensuite fermé hermétiquement et chauffé à 65 °C pendant 3 h. On laisse refroidir et on chasse le monoxyde de carbone par l'argon. Le milieu réactionnel est ensuite hydrolysé avec H₂O puis extrait avec AcOEt. Les extraits organiques sont regroupés, séchés sur MgSO₄ et concentrés à sec. Le résidu est purifié par chromatographie sur colonne de silice pour conduire au produit du titre sous la forme d'une huile qui cristallise.

### Stade H : (4aS,R)-2,3,4,4a,5,6-Hexahydro-1H-pyrazino[1,2-a]quinoline-8-carboxylate de méthyle

A une solution du composé obtenu au Stade G (3,2 g) dans 100 ml d'un mélange THF/MeOH (50/50), on ajoute successivement 0,64 g de Pd/C 10 % puis 2,4 g de NH₄COOH et l'ensemble est chauffé à 50°C pendant 4 h. Le milieu réactionnel est ensuite refroidi puis filtré et concentré à sec. Le solide obtenu est repris dans de l'éther diisopropylique, trituré, filtré et concentré à sec pour conduire au produit du titre sous la forme d'un solide blanc.

### Stade I : (4aS,R)-3-[(4'-Chloro[1,1'-biphényl]-2-yl)méthyl]-2,3,4,4a,5,6-hexahydro-1H-pyrazino[1,2-a]quinoline-8-carboxylate de méthyle

Le composé obtenu au Stade H (7 g) est solubilisé dans 100 ml de DMF, puis on ajoute successivement 8,85 g de K₂CO₃, 4,2 ml de 4-chloro-2'-(chlorométhyl)-1,1'-biphényle et 100 mg de NaI et l'ensemble est chauffé à 80°C pendant 2 heures. On concentre à sec, on reprend avec AcOEt et on lave avec H₂O, une solution saturée de LiCl puis avec une solution saturée de NaCl. La phase organique est séchée sur MgSO₄ et concentrée à sec. Le résidu est purifié par chromatographie sur colonne de silice (heptane/AcOEt 95/5) pour conduire au produit du titre sous la forme d'un solide blanc crémeux.

### Stade J : Acide (4aS,R)-3-[(4'-chloro[1,1'-biphényl]-2-yl)méthyl]-2,3,4,4a,5,6-hexahydro-1H-pyrazino[1,2-a]quinoline-8-carboxylique, chlorhydrate

A une solution de 0,5 g du composé obtenu au Stade I dans 9 ml de dioxane on ajoute 9 ml d'HCl 6N. L'ensemble est ensuite chauffé à reflux 4 h puis concentré à sec. Le solide résultant est trituré dans l'éther diisopropylique, filtré et séché pour conduire au composé du titre sous la forme d'un solide blanc/bleuté.

### Préparation 2 : Acide (4aS,R)-3-[(4'-chloro[1,1'-biphényl]-3-yl)méthyl]-2,3,4,4a,5,6-hexahydro-1H-pyrazino[1,2-a]quinoline-8-carboxylique

On procède comme dans la Préparation 1 en remplaçant au Stade I le 4-chloro-2'-(chlorométhyl)-1,1'-biphényle par le 4-chloro-3'-(chlorométhyl)-1,1'-biphényle.

### Préparation 3 : Acide (4aS,R)-3-[(4'-chloro[1,1'-biphényl]-4-yl)méthyl]-2,3,4,4a,5,6-hexabydro-1H-pyrazino[1,2-a]quinoline-8-carboxylique, chlorhydrate

On procède comme dans la Préparation 1 en remplaçant au Stade I le 4-chloro-2'-(chlorométhyl)-1,1'-biphényle par le 4-chloro-4'-(chlorométhyl)-1,1'-biphényle.

### Préparation 4 : Acide (4aS,R)-3-([1,1'-biphényl]-2-ylméthyl)-2,3,4,4a,5,6-hexahydro-1H-pyrazino[1,2-a]quinoline-8-carboxylique, chlorhydrate

On procède comme dans la Préparation 1 en remplaçant au Stade I le 4-chloro-2'-(chlorométhyl)-1,1'-biphényle par le 2-(chlorométhyl)-1,1'-biphényle.

### Préparation 5 : Acide (4aS,R)-3-(2-benzylbenzyl)-2,3,4,4a,5,6-hexahydro-1H-pyrazino[1,2-a] quinoline-8-carboxylique, chlorhydrate

On procède comme dans la Préparation 1 en remplaçant au Stade I le 4-chloro-2'-(chlorométhyl)-1,1'-biphényle par le 1-benzyl-2-(chlorométhyl)benzène.

### Préparation 6 : Acide (4aS,R)-3-[2-(2-phényléthyl)benzyl]-2,3,4,4a,5,6-hexahydro-1H-pyrazino[1,2-a]quinoline-8-carboxylique, chlorhydrate

On procède comme dans la Préparation 1 en remplaçant au Stade I le 4-chloro-2'-(chlorométhyl)-1,1'-biphényle par le 1-(chlorométhyl)-2-(2-phényléthyl)benzène.

### Préparation 7 : Acide (4aS,R)-3-[(4'-chloro[1,1'-biphényl]-2-yl)méthyl]-2,3,4,4a,5,6-hexahydro-1H-pyrazino[1,2-a]quinoline-7-carboxylique, chlorhydrate

On procède comme dans la Préparation 1 en remplaçant au Stade A la 6-méthoxy-2-méthylquinoline par la 5-méthoxy-2-méthylquinoline.

### Préparation 8 : Acide (4aS,R)-3-([1,1'-biphényl]-2-ylméthyl)-2,3,4,4a,5,6-hexahydro-1H-pyrazino[1,2-a]quinoline-7-carboxylique, chlorhydrate

On procède comme dans la Préparation 1 en remplaçant au Stade A la 6-méthoxy-2-méthylquinoline par la 5-méthoxy-2-méthylquinoline, et au Stade I le 4-chloro-2'-(chlorométhyl)-1,1'-biphényle par le 2-(chlorométhyl)-1,1'-biphényle.

### Préparation 9 : Acide 2-([1,1'-biphényl]-2-ylméthyl)-1,2,3,4-tétrahydropyrazino[1,2-a]indole-8-carboxylique, chlorhydrate

### Stade A : 5-Méthoxy-1H-indole-2-carboxylate d'éthyle

A une suspension de 20 g d'acide 5-méthoxy-1H-indole-2carboxylique dans 100 ml d'éthanol absolu placée à 0°C on ajoute goutte à goutte via une ampoule à brome 14,5 ml de chlorure de thionyle. On laisse revenir progressivement à température ambiante après l'addition, puis on chauffe à léger reflux pendant 4 h. On concentre ensuite le milieu réactionnel puis le solide résultant est trituré dans l'éther diisopropylique, filtré et séché. Le produit du titre est obtenu sous la forme d'un solide brun foncé.

### Stade B : 1-(Cyanométhyl)-5-méthoxy-1H-indole-2-carboxylate d'éthyle

A une suspension de 5,7 g de NaH (60 %) dans 100 ml de DMF anhydre on ajoute goutte à goutte via une ampoule à brome 20,8 g du composé obtenu au Stade A en solution dans 150 ml de DMF anhydre, on laisse agiter 30 minutes à température ambiante puis on ajoute 12 ml de chloroacétonitrile et l'ensemble est agité la nuit à température ambiante. Le milieu réactionnel est ensuite concentré à sec, repris à l'AcOEt, hydrolysé avec H₂O, puis extrait 2 fois avec AcOEt. Les phases organiques sont ensuite combinées, lavées avec une solution saturée de LiCl puis avec une solution saturée en NaCl, séchées sur MgSO₄, filtrées et concentrées à sec. Le solide est ensuite purifié par chromatographie sur gel de silice ( Heptane/AcOEt 95/5 ) pour conduire au produit du titre sous la forme d'un solide jaunâtre.

### Stade C : 8-Méthoxy-1,2,3,4-tétrahydropyrazino[1,2-a]indole

A 7,35 g d'une solution du composé obtenu au Stade B dans 150 ml de THF anhydre placée à 0°C on ajoute goutte à goutte via une ampoule à brome 57 ml d'une solution commerciale d'hydrure d'aluminium 1M dans le THF. On laisse revenir progressivement à température ambiante puis on chauffe à léger reflux pendant 3h. On laisse refroidir puis on se place à 0 °C et on hydrolyse lentement avec une solution saturée de sel de Rochelle. Le milieu réactionnel est ensuite extrait avec AcOEt. Les extraits organiques sont regroupés, lavés avec une solution saturée NaCl, séchés sur MgSO₄ et concentrés à sec. On obtient 5,7 g d'une huile brune correspondant au produit du titre.

### Stade D : 2-([1,1'-Biphényl]-2-ylméthyl)-8-méthoxy-1,2,3,4-tétrahydropyrazino[1,2-a]indole

On procède comme au Stade I de la Préparation 1 à partir du composé obtenu au Stade C en remplaçant le 4-chloro-2'-(chlorométhyl)-1,1'-biphényle par le 2-(chlorométhyl)-1,1'-biphényle. Le produit du titre est obtenu sous la forme d'un solide jaunâtre.

### Stade E : 2-([1,1'-Biphényl]-2-ylméthyl)-1,2,3,4-tétrahydropyrazino[1,2-a]indol-8-yl trifluorométhanesulfonate

On procède comme au Stade F de la Préparation 1. Le produit du titre est obtenu sous la forme d'un solide jaune-orangé.

### Stade F : 2-([1,1'-Biphényl]-2-ylméthyl)-1,2,3,4-tétrahydropyrazino[1,2-a]indole-8-carboxylate de méthyle

On procède comme au Stade G de la Préparation 1. Le produit du titre est obtenu sous la forme d'un solide jaunâtre.

### Stade G : Acide 2-([1,1'-biphényl]-2-ylméthyl)-1,2,3,4-tétrahydropyrazino[1,2-a]indole-8-carboxylique, chlorhydrate

On procède comme au Stade J de la Préparation 1. Le produit du titre est obtenu sous la forme d'un solide jaunâtre.

### Préparation 10 :Acide 2-[(4'-chloro[1,1'-biphényl]-2-yl)méthyl]-1,2,3,4-tétrahydro pyrazino[1,2-a]indole-8-carboxylique, chlorhydrate

On procède comme dans la Préparation 9 en remplaçant au Stade D le 2-(chlorométhyl)-1,1'-biphényle par le 4-chloro-2'-(chlorométhyl)-1,1'-biphényle.

### Préparation 11 :Acide (10aS,R)-2-[(4'-chloro[1,1'-biphényl]-2-yl)méthyl]-1,2,3,4,10,10a-hexahydropyrazino[1,2-a]indole-8-carboxylique, chlorhydrate

### Stade A : 2-[(4'-Chloro[1,1'-biphényl]-2-yl)méthyl]-1,2,3,4-tétrahydropyrazino[1,2-a]indole-8-carboxylate de méthyle

On procède comme dans la Préparation 9 Stades A à F en remplaçant au Stade D le 2-(chlorométhyl)-1,1'-biphényle par le 4-chloro-2'-(chlorométhyl)-1,1'-biphényle.

### Stade B : (10aS,R)-2-[(4'-Chloro[1,1'-biphényl]-2-yl)méthyl]-1,2,3,4,10,10a-hexahydropyrazino [1,2-a]indole-8-carboxylate de méthyle

A une solution de 0,2 g du composé obtenu au Stade A dans 5 ml d'acide acétique, on additionne à température ambiante 0,158 g de NaBH₃CN. Le milieu réactionnel est agité pendant 48h. On hydrolyse le milieu avec une solution saturée de NaHCO₃ puis on extrait avec AcOEt. Les phases organiques sont réunies puis lavées avec une solution saturée de NaCl, puis séchées sur MgSO₄, filtrées et évaporées à sec. Le composé du titre est ensuite purifié par chromatographie sur gel de silice.

### Stade C : Acide (10aS,R)-2-[(4'-chloro[1,1'-biphényl]-2-yl)méthyl]-1,2,3,4,10,10a-hexahydropyrazino[1,2-a]indole-8-carboxylique, chlorhydrate

On procède comme au Stade J de la Préparation 1 à partir du composé obtenu au Stade B.

### Préparation 12 :Acide (4aS)-3-[(4'-chloro[1,1'-biphényl]-2-yl)méthyl]-2,3,4,4a,5,6-hexahydro-1H-pyrazino[1,2-a]quinoline-8-carboxylique, chlorhydrate

### Stade A : (4aS)-8-Méthoxy-3-[(2S)-2-méthoxy-2-phényléthanoyl]-2,3,4,4a,5,6-hexahydro-1H-pyrazino[1,2-a]quinoline

Dans un premier temps, à une solution de 4,94 g d'acide S-méthoxyphénylacétique dans 100 ml de CH₂Cl₂, on additionne 36,5 ml de chlorure de thionyle. On chauffe le milieu réactionnel à 40°C pendant une demie journée puis on le laisse refroidir à température ambiante. On évapore à sec et on obtient une huile. Dans un second temps, à une solution de 6,18 g du composé obtenu au Stade D de la Préparation 1 dans 120 ml de CH₂Cl₂ et 240 ml de NaOH 1N, on additionne l'huile obtenue précédemment dans 120 ml de CH₂Cl₂. On agite le tout vigoureusement à température ambiante pendant 1 h. On sépare les deux phases et on extrait une fois avec CH₂Cl₂. Après un lavage avec une solution saturée en NaCl et un séchage sur MgSO_{4,} on concentre à sec. Le mélange est purifiée par chromatographie flash sur gel de silice (éther de pétrole/AcOEt 80/20) et conduit au mélange des deux diastéréoisomères sous la forme d'une huile.
La séparation des diastéréoisomères est ensuite réalisée par chromatographie liquide préparative optique sur chiralpak AD avec comme solvant et éluant EtOH.

### Stade B : (4aS)-8-Méthoxy-2,3,4,4a,5,6-hexahydro-1H-pyrazino[1,2-a]quinoline

A une solution de 3,91 g du composé obtenu au Stade A dans 150 ml de THF on additionne 9,6 g de KO*t*Bu. Le milieu réactionnel est agité à température ambiante une demie journée plus une nuit. On évapore le THF puis le milieu réactionnel est hydrolysé avec H₂O et extrait avec AcOEt. Après un lavage avec une solution saturée en NaCl puis un séchage sur MgSO₄, on concentre à sec. Le mélange est purifié par chromatographie flash sur gel de silice (CH₂Cl₂/MeOH/NH₄OH(95/5/0,5)) pour conduire au produit du titre sous la forme d'une huile.

### Stade C : Acide (4aS)-3-[(4'-chloro[1,1'-biphényl]-2-yl)méthyl]-2,3,4,4a,5,6-hexahydro-1H-pyrazino[1,2-a]quinoline-8-carboxylique, chlorhydrate

On procède comme dans les Stades E à J de la Préparation 1 à partir du composé obtenu au Stade B.

### Préparation 13 :Acide (4aR)-3-[(4'-chloro[1,1'-biphényl]-2-yl)méthyl]-2,3,4,4a,5,6-hexahydro-1H-pyrazino[1,2-a]quinoline-8-carboxylique, chlorhydrate

On procède comme dans la Préparation 12 en prenant au Stade A l'autre diastéréoisomère obtenu.

### Préparation 14 :Acide (4aS)-3-([1,1'-biphényl]-2-ylméthyl)-2,3,4,4a,5,6-hexabydro-1H-pyrazino[1,2-a]quinoline-8-carboxylique, chlorhydrate

On procède comme dans la Préparation 12, et en remplaçant au Stade I de la Préparation 1 le 4-chloro-2'-(chlorométhyl)-1,1'-biphényle par le 2-(chlorométhyl)-1,1'-biphényle.

### Préparation 15 : Acide (4aR)-3-([1,1'-biphényl]-2-ylméthyl)-2,3,4,4a,5,6-hexahydro-1H-pyrazino[1,2-a]quinoline-8-carboxylique, chlorhydrate

On procède comme dans la Préparation 13, et en remplaçant au Stade I de la Préparation 1 le 4-chloro-2'-(chlorométhyl)-1,1'-biphényle par le 2-(chlorométhyl)-1,1'-biphényle.

### Préparation 16 :Acide (4aS,R)-3-([1,1'-biphényl]-2-ylsulfonyl)-2,3,4,4a,5,6-hexahydro-1H-pyrazino[1,2-a]quinoline-8-carboxylique, chlorhydrate

### Stade A : 3-([1,1'-Biphényl]-2-ylsulfonyl)-8-méthoxy-2,3,4,4a,5,6-hexahydro-1H-pyrazino[1,2-a]quinoline

On procède comme dans le Stade E de la Préparation 1 en remplaçant le bromure de benzyle par le chlorure de [1,1'-biphényl]-2-sulfonyle.

### Stade B : Acide 3-([1,1'-biphényl]-2-ylsulfonyl)-2,3,4,4a,5,6-hexahydro-1H-pyrazino[1,2-a]quinoline-8-carboxylique, chlorhydrate

On procède comme dans les Stades F, G et J de la Préparation 1 à partir du composé obtenu au Stade A.

### Préparation 17 :Acide 3-[(4'-chloro[1,1'-biphényl]-2-yl)méthyl]-1,2,3,4,4a,5,6,7-octahydropyrazino[1,2-a][1]benzazépine-9-carboxylique

### Stade A : 6-Méthoxy-3,4-dihydro-1(2H)-naphthalènone O-méthyl-oxime

A une solution de 28,64 g de 6-méthoxy-3,4-dihydro-1(2H)-naphthalènone dans 500 ml de méthanol sont additionnés 28,93 g de Na₂HPO₄.2H₂O et 27,15 g de MeONH₂.HCl. Le mélange réactionnel est ensuite agité à température ambiante pendant 2 h. Après concentration, le résidu est repris dans un mélange CH₂Cl₂/H₂O et la phase organique est lavée à l'eau, séchée sur sulfate de magnésium et concentrée pour conduire au produit attendu.

### Stade B : N-(6-Méthoxy-1,2,3,4-tétrahydro-naphthalèn-1-yl)-O-méthyl-hydroxylamine

A une solution de 10 g du composé obtenu au stade A dans 100 ml d'éthanol sont additionnés 17,2 ml du complexe BH3.pyridine. Après avoir porté le milieu réactionnel à 0°C, 200 ml d'une solution d'HCl, 2.5 N sont ajoutés goutte à goutte pendant une durée de 3 h. Après retour à température ambiante et agitation pendant 1 h, une solution saturée de NaHCO₃ est additionnée goutte à goutte à 0°C jusqu'à pH 5. La phase aqueuse est extraite au CH₂Cl₂, puis la phase organique est séchée sur sulfate de magnésium, concentrée et purifiée par une chromatographie sur colonne de silice (heptane/AcOEt) pour conduire au produit attendu.

### Stade C : 7-Méthoxy-2-vinyl-2,3,4,5-tétrahydro-1H-1-benzazépine

A une solution de 10 g du composé précédent dans 100 ml de THF sont additionnées 145 mmol de bromovinyl magnésien dans 145 ml de THF. L'addition se fait à 0°C en 40 minutes, puis le mélange réactionnel est remonté à température ambiante. Après 1 h d'agitation, le milieu est hydrolysé à l'eau goutte à goutte à 0°C, puis la phase aqueuse est extraite au CH₂Cl₂. Les phases organiques sont réunies, séchées sur sulfate de magnésium, filtrées et concentrées. Le résidu obtenu est purifié par une chromatographie sur colonne de silice (heptane/AcOEt) pour conduire au produit attendu.

### Stade D : 7-Méthoxy-2-vinyl-2,3,4,5-tétrahydro-1H-1-benzazépine-1-carboxylate de tert-butyle

A une solution de 1,52 g du composé du stade C dans 15 ml de THF sont additionnés 2,45 g de Boc₂O et 1,55 g de K₂CO₃. Le mélange réactionnel est porté à 60°C et agité pendant 16 h. Après dilution dans un mélange AcOEt/H₂O, la phase aqueuse est extraite à l'AcOEt, puis les phases organiques sont réunies, lavées à l'eau et à une solution saturée de NaCl, séchées sur sulfate de magnésium, filtrées et concentrées. Le résidu obtenu est purifié par une chromatographie sur colonne de silice (heptane/AcOEt) pour conduire au produit attendu.

### Stade E : 7-Méthoxy-2,3,4,5-tétrahydro-1H-1-benzazépine-2-carboxylate de méthyle

A une solution de 1 g du composé précédent dans 50 ml de CH₂Cl₂ sont ajoutés 12 ml d'une solution de NaOH, 2.5N dans le méthanol. L'ensemble est refroidi à -78°C et un courant d'ozone est appliqué. Après apparition de la couleur bleu caractéristique, le milieu réactionnel est hydrolysé et extrait à l'AcOEt. Les phases organique sont réunies, séchées sur sulfate de magnésium, filtrées et concentrées. Le résidu obtenu est mis en solution dans une solution d'HCl, 4N dans le dioxane. Après neutralisation, la phase aqueuse est extraite au CH₂Cl₂ et les phases organiques sont réunies, séchées sur sulfate de magnésium, filtrées et concentrées. Le résidu obtenu est purifié par une chromatographie sur colonne de silice (CH₂Cl₂/MeOH) pour conduire au produit attendu.

### Stade F : 1-({[(Benzyloxy)carbonyl]amino}acétyl)-7-méthoxy-2,3,4,5-tétrahydro-1H-1-benzazépine-2-carboxylate de méthyle

A une solution de 14,3 g d'acide [(benzyloxy)carbonyl]amino-acétique dans 300 ml de THF sont ajoutés 14,2 g de PCl₅ par portions à 0°C. L'ensemble est ensuite agité à cette même température pendant 2 h. Une solution de 10 g du composé du stade E dans 100 ml de THF et 50 ml de pyridine est additionnée goutte à goutte à 0°C pendant 2 h au milieu réactionnel. Celui-ci est ensuite porté à température ambiante et agité pendant 16 h. Le milieu hétérogène obtenu est hydrolysé goutte à goutte à 0°C puis extrait à l'AcOEt. Les phases organiques sont réunies, lavées avec une solution saturée de NaHCO₃ et une solution saturée de NaCl, séchées sur sulfate de magnésium, filtrées et concentrées. Le résidu obtenu est purifié par une chromatographie sur colonne de silice (heptane/AcOEt) pour conduire au produit attendu.

### Stade G : 9-Méthoxy-2,3,4a,5,6,7-hexahydropyrazino[1,2-a][1]benzazépine-1,4-dione

A une solution de 10 g du composé obtenu au stade F dans 400 mL d'un mélange THF/MeOH (1/3), on ajoute successivement 2 g de Pd/C 10 % et 4,1 g de NH₄COOH. L'ensemble est chauffé à 50°C pendant 10 h. Le milieu réactionnel est ensuite refroidi, puis filtré et concentré à sec. Le solide obtenu est repris dans diisopropyl éther, trituré, filtré et concentré à sec pour conduire au composé attendu.

### Stade H : 3-Benzyl-9-méthoxy-2,3,4a,5,6,7-hexahydropyrazino[1,2-a][1]benzazépine-1,4-dione

A une solution hétérogène de 1,6 g de NaH 60 % dans 50 ml de DMF sont additionnés, goutte à goutte, 7 g du composé du stade G dans 450 ml de DMF à 0°C pendant 2 h. Après retour à température ambiante, 4 ml de bromure de benzyle sont ajoutés en 30 min, puis le milieu réactionnel est agité pendant 16 h. Après concentration, le résidu est repris à 0°C dans un mélange AcOEt et une solution saturée de NaHCO₃. La phase aqueuse est extraite à l'AcOEt, puis les phases organiques sont réunies, lavées avec une solution saturée de LiCl, séchées sur sulfate de magnésium, filtrées et concentrées. Le résidu obtenu est purifié par une chromatographie sur colonne de silice (heptane/AcOEt) pour conduire au composé attendu.

### Stade I : 3-Benzyl-9-méthoxy-1,2,3,4,4a,5,6,7-octahydropyrazino[1,2-a][1] benzazépine

A une solution de 7 g du composé du stade H dans 150 ml de THF sont additionnés, par portions, 2,27 g de NaBH₄ à 0°C. Le mélange réactionnel est ensuite agité à cette même température pendant 30 minutes. Puis, 11,4 ml du complexe de BF₃ . Et₂O sont additionnés goutte à goutte à 0°C pendant 1 h. Après retour à température ambiante, le mélange réactionnel est reflué pendant 16 h, et 50 ml d'une solution d'HCl, 5N sont ajoutés goutte à goutte à 0°C. Le milieu réactionnel est ensuite chauffé à reflux pendant 1 h avant d'être hydrolysé par 50 ml de NaOH, 5N jusqu'à pH 5. La phase aqueuse est extraite à l'AcOEt, puis les phases organiques sont réunies et lavées à une solution saturée de NaHCO₃, séchées sur sulfate de magnésium, filtrées et concentrées. Le résidu obtenu est purifié par une chromatographie sur colonne de silice (heptane/AcOEt) pour conduire au composé attendu.

### Stade J : 3-Benzyl-1,2,3,4,4a,5,6,7-octahydropyrazino[1,2-a][1]benzazépin-9-yl trifluorométhane sulfonate

On procède comme au stade F de la préparation 1.

### Stade K : 3-Benzyl-1,2,3,4,4a,5,6,7-octahydropyrazino[1,2-a][1]benzazépine-9-carboxylate de méthyle

On procède comme au stade G de la préparation 1.

### Stade L : 1,2,3,4,4a,5,6,7-Octahydropyrazino[1,2-a][1]benzazépine-9-carboxylate de méthyle, chlorhydrate

A une solution de 1,5 g du composé précédent dans 35 ml d'une solution d'HCl, 1N dans le méthanol sont additionnés 300 mg du catalyseur au palladium. La solution hétérogène est ensuite hydrogénée pendant 48 h à une pression de 2 bars. Le milieu réactionnel est filtré et rincé au méthanol pour conduire au composé du titre sous la forme chlorhydrate.

### Stade M : 3-[(4'-Chloro[1,1'-biphényl]-2-yl)méthyl]-1,2,3,4,4a,5,6,7-octahydropyrazino[1,2-a][1]benzazépine-9-carboxylate de méthyle

On procède comme au stade I de la préparation 1.

### Stade N : Acide 3-[(4'-chloro[1,1'-biphényl]-2-yl)méthyl]-1,2,3,4,4a,5,6,7-octahydropyrazino[1,2-a][1]benzazépine-9-carboxylique

A une solution de 850 mg du composé du stade M dans 15 mL d'un mélange dioxane/H₂O (4/1) sont ajoutés 387 mg de LiOH. L'ensemble est ensuite agité pendant 4 h, puis concentré à sec. Après dilution dans HCl, 0.5 N, la phase aqueuse est extraite à l'AcOEt. Les phases organiques sont ensuite réunies, lavées avec une solution saturée de NaHCO₃ et avec une solution saturée de NaCl, séchées sur sulfate de magnésium, filtrées et concentrées. Le solide résultant est lyophilisé dans un mélange ACN/H₂O pour conduire au composé du titre.

### Préparation 18 : Acide (10aα)-2-[(4'-chloro[1,1'-biphényl]-2-yl)méthyl]-1,2,3,4,10,10a-hexahydropyrazino[1,2-a]indole-8-carboxylique (α=R ou S)

Le composé est obtenu par séparation sur colonne Chiralpak AD-H du mélange racémique obtenu à la Préparation 11 en utilisant le méthanol, l'acétonitile et la diéthylamine comme éluants.
*Temps de rétention : 8, 7 min*

### Préparation 19 :Acide (10aβ)-2-[(4'-chloro[1,1'-biphényl]-2-yl)méthyl]-1,2,3,4,10,10a-hexahydropyrazino[1,2-a]indole-8-carboxylique (β=S ou R)

Le composé est obtenu par séparation sur colonne Chiralpak AD-H du mélange racémique obtenu à la Préparation 11 en utilisant le méthanol, l'acétonitile et la diéthylamine comme éluants.
*Temps de rétention : 9, 6 min*

### Préparation 20 : Acide 6-(tert-butoxycarbonyl)-3-[(4'-chloro[1,1'-biphényl]-2-yl)méthyl]-2,3,4,4a,5,6-hexahydro-1H-pyrazino[1,2-a]quinoxaline-8-carboxylique

### Stade A : Acide 4-[(benzyloxy)carbonyl]-1-(2-nitrophényl)-2-pipérazine carboxylique

A une solution de 5 g d'acide 2-pipérazine carboxylique dichlorhydrate dans 20 ml d'eau sont additionnés 19,5 ml d'une solution de NaOH, 2,5N, puis une solution de 2,07 g de sulfate de cuivre dans 40 ml d'eau. La solution bleue ainsi obtenue est refroidie à 5°C, puis on y ajoute en une fois 2,5 g de Na₂CO₃ et, goutte à goutte, une solution de 3,85 ml de chloroformate de benzyle dans 20 ml de dioxane. Après retour à température ambiante, le mélange réactionnel est agité pendant 24 h. Le précipité est filtré pour donner l'acide 4-[(benzyloxy)carbonyl]-2-pipérazine carboxylique chélaté avec du cuivre. Ce dernier composé est mis en solution dans 375 ml d'eau, puis on y ajoute 4,5 g d'EDTA . Le mélange réactionnel est chauffé à 80°C pendant 3 h, puis concentré à sec. Le résidu est repris dans 75 ml de DMSO. Y sont additionnés 3,43 g de 2-fluoro-nitrobenzène et 15 ml d'Et₃N, puis le mélange réactionnel est porté à 60°C pendant 48 h. Après retour à température ambiante, la solution est amenée à pH 3 à l'aide de HCl, 5N, puis diluée dans 250 ml d'eau, et extraite à l'AcOEt. Les phases organiques sont lavées à l'eau, séchées sur sulfate de magnésium, concentrées et purifiées par une chromatographie sur colonne de silice (CH₂Cl₂/MeOH) pour conduire au produit attendu.

### Stade B: 5-Oxo-1,2,4,4a,5,6-hexahydro-3H-pyrazino[1,2-a]quinoxaline-3-carboxylate de benzyle

A une solution de 5,7 g du composé du Stade A dans 100 ml d'acide acétique sont additionnés, par portion, 8 g de poudre de fer. Le mélange est chauffé à 60°C pendant 3 h, puis après retour à température ambiante, 50 ml d'HCl, 1N sont additionnés. La solution est extraite au dichlorométhane et les phases organiques sont réunies, séchées sur sulfate de magnésium puis concentrées. Le résidu est purifié par une chromatographie sur colonne de silice (heptane/AcOEt) pour conduire au produit attendu.

### Stade C: 8-Bromo-5-oxo-1,2,4,4a,5,6-hexahydro-3H-pyrazino[1,2-a]quinoxaline-3-carboxylate de benzyle

A une solution de 4,4 g du composé du Stade B dans 40 ml de DMF sont additionnés à 0°C en 25 minutes une solution de 2,55 g de N-bromosuccinimide dans 30 ml de DMF. La solution orange est mélangée à cette même température pendant 1,5 h, puis est diluée dans un mélange H₂O/AcOEt (1/1). La phase aqueuse est extraite à l'AcOEt, puis les phases organiques sont réunies et lavées à l'eau, puis à une solution saturée de LiCl avant d'être séchées sur sulfate de magnésium, filtrées et concentrées. Le résidu obtenu est purifié par une chromatographie sur colonne de silice (heptane/AcOEt) pour conduire au produit attendu.

### Stade D: 8-Bromo-1,2,4,4a,5,6-hexahydro-3H-pyrazino[1,2-a]quinoxaline-3-carboxylate de benzyle

On procède comme au Stade I de la Préparation 17 en utilisant le composé obtenu à l'étape précédente.

### Stade E: 3-Benzyl-6-tert-butyl-8-bromo-4a,5-dihydro-1H-pyrazino[1,2-a] quinoxaline-3,6(2H,4H)-dicarboxylate

On procède comme au Stade D de la Préparation 17 en utilisant le composé obtenu à l'étape précédente.

### Stade F: 3-Benzyl-6-tert-butyl-8-méthyl 4a,5-dihydro-1H-pyrazino[1,2-a] quinoxaline-3,6,8(2H,4H)-tricarboxylate

On procède comme au Stade G de la Préparation 1 en utilisant le composé obtenu à l'étape précédente.

### Stade G: 6-tert-Butyl-8-méthyl-1,2,3,4,4a,5-hexahydro-6H-pyrazino[1,2-a] quinoxaline-6,8-dicarboxylate

A une solution de 1,1 g du composé obtenu au Stade F dans 40 mL d'un mélange THF/MeOH (1/1), on ajoute successivement 0,22 g de Pd/C,10 %, puis 144 mg de NH₄COOH. L'ensemble est chauffé à 50°C pendant 4 h. Le milieu réactionnel est ensuite refroidi, puis filtré et concentré à sec. Le solide obtenu est repris dans diisopropyl éther, trituré, filtré et concentré à sec pour conduire au composé du titre.

### Stade H: 6-tert-Butyl-8-méthyl-3-[(4'-chloro[1,1'-biphényl]-2-yl)méthyl]-1,2,3,4,4a,5-hexahydro-6H-pyrazino[1,2-a]quinoxaline-6,8-dicarboxylate

On procède comme au Stade I de la Préparation 1 en utilisant le composé obtenu à l'étape précédente.

### Stade I: Acide 6-(tert-butoxycarbonyl)-3-[(4'-chloro[1,1'-biphényl]-2-yl)méthyl]-2,3,4,4a,5,6-hexahydro-1H-pyrazino[1,2-a]quinoxaline-8-carboxylique

On procède comme au Stade N de la Préparation 17 en utilisant le composé obtenu à l'étape précédente.

### Préparation 21 : Acide 6-(tert-butoxycarbonyl)-3-[(4'-chloro[1,1'-biphényl]-2-yl) méthyl]-5-oxo-2,3,4,4a,5,6-hexahydro-1H-pyrazino[1,2-a] quinoxaline-8-carboxylique

Le composé du titre est obtenu en suivant les mêmes étapes développées à la Préparation 20 à deux différences près : le stade D est supprimé, tandis que dans le stade E, l'addition du groupement Boc se fait en présence de HNa (et non plus de K₂CO₃) dans le DMF (et non plus le THF).

### Préparation 22 : 1-Bromo-2-(bromométhyl)-4,4-diméthyl-1-cyclohexène

### Stade A: 4,4-Diméthyl-cyclohexanone

A une solution de 4,4-diméthyl-2-cyclohexén-1-one (0,0805 mol, 10,6 ml) dans 110 ml de AcOEt, on ajoute 1 g, par portion, de Pd/C, 5%, puis l'ensemble est agité durant 2 h à température ambiante sous pression atmosphérique d'hydrogène. On filtre le palladium et on concentre à sec. L'huile ainsi obtenue cristallise progressivement. On obtient ainsi le composé du titre sous forme d'un solide blanc.

### Stade B: 2-Bromo-5,5-diméthyl-1-cyclohexène-1-carbaldéhyde

A un mélange de 110 mL de CH₂Cl₂ et de 18,7 mL de DMF placé à 0°C dans un bain de glace, on ajoute, goutte à goutte, 20,1 ml de tribromure de phosphore. L'ensemble est agité à température ambiante pendant 30 minutes. On refroidit ensuite à 0°C le milieu réactionnel et on ajoute 10,5 g du composé du Stade A en solution dans 90 mL de CH₂Cl₂. Puis, l'ensemble est agité progressivement à température ambiante durant 4 heures, avant d'être versé dans un mélange glace / solution saturée de NaHCO₃. Il est ensuite agité pendant une heure et extrait avec Et₂O. Les phases organiques sont alors regroupées, lavées avec une solution saturée de NaCl, séchées sur sulfate de magnésium puis concentrées à sec .Le résidu est alors purifié sur colonne de silice (heptane AcOEt gradient 0% à 5% AcOEt). On obtient le composé du titre sous forme d'une huile incolore.

### Stade C: (2-Bromo-5,5-diméthyl-1-cyclohexén-1-yl)-méthanol

A une solution de 12,48 g du composé du Stade B placée à 0°C dans 120 mL de méthanol, on ajoute, par portion, 3,23 g de borohydrure de sodium. L'ensemble est agité progressivement à température ambiante durant 5 heures. Le milieu réactionnel est ensuite refroidi à 0°C, puis hydrolysé et extrait avec CH₂Cl₂. Les phases organiques sont alors regroupées, lavées avec une solution saturée de NaCl, séchées sur sulfate de magnésium et finalement concentrées à sec. On obtient le composé du titre sous forme d'une huile incolore qui est purifiée par chromatographie sur colonne de silice (heptane/ AcOEt).

### Stade D: 1-Bromo-2-(bromométhyl)-4,4-diméthyl-1-cyclohexène

3,96 g du composé du Stade C sont mis en solution dans 70 mL de Et₂O, le tout étant placé à 0°C. On y ajoute ensuite, goutte à goutte, 1,7 ml de tribromure de phosphore. L'ensemble est agité à cette température pendant 1h30. Puis, le milieu réactionnel est hydrolysé avant d'être extrait par Et₂O. Les phases organiques sont alors regroupées, lavées avec une solution saturée de NaHCO₃, puis une solution de NaCl saturée, séchées sur sulfate de magnésium et finalement concentrées à sec. Le composé du titre est obtenu sous forme d'une huile incolore qui est purifiée par chromatographie sur colonne de silice (heptane/ AcOEt).

### Préparation 23 : Acide (4aR)-3-{[2-(4-chlorophényl)-5,5-diméthyl-1-cyclohexén-1-yl]méthyl}-2,3,4,4a,5,6-hexahydro-1H-pyrazino[1,2-a]quinoline-8-carboxylique, chlorhydrate

### Stade A: (4aR)-2,3,4,4a,5,6-Hexahydro-1H-pyrazino[1,2-a]quinoline-8-carboxylate de méthyle

On procède comme dans les Stades A et B de la Préparation 12 en sélectionnant l'autre diastéréoisomère. On applique ensuite au composé obtenu les mêmes traitements que ceux présentés aux Stades E, F, G et H de la Préparation 1.

### Stade B: (4aR)-3-[(2-Bromo-5,5-diméthyl-1-cyclohexén-1-yl)méthyl]-2,3,4,4a,5,6-hexahydro-1H-pyrazino[1,2-a]quinoline-8-carboxylate de méthyle

A une solution de 0,76 g du composé du Stade A dans 20 mL de DMF, on ajoute successivement 0,98 ml d'Et₃N ,0,1 g de NaI et 1,06 g du composé de la préparation 22. L'ensemble est ensuite chauffé à 80°C pendant 3 heures. Après refroidissement à température ambiante, le milieu réactionnel est hydrolysé puis extrait avec AcOEt . Les phases organiques sont regroupées, lavées avec une solution saturée de LiCl, avec une une solution saturée de NaCl, puis séchées sur sulfate de magnésium et concentrées à sec. Le solide résultant est alors repris dans l'ether diisopropylique, trituré puis filtré. On obtient le composé du titre sous forme d'un solide blanc suffisamment pur pour être utilisé à l'étape suivante.

### Stade C: (4aR)-3-{[2-(4-Chlorophényl)-5,5-diméthyl-1-cyclohexén-1-yl]méthyl}-2,3,4,4a,5,6-hexahydro-1H-pyrazino[1,2-a]quinoline-8-carboxylate de méthyle

A une suspension de 1,33 g du composé du Stade B dans un mélange DME/ H₂O/ EtOH (15 mL/6 mL/4 mL), on ajoute successivement 0,14 g de PdCl₂(Ph₃)₂, puis 0,697 g d'acide 4-chlorophényl boronique et 2,4 ml d'une solution aqueuse 2M de Na₂CO₃. L'ensemble est dégazé sous argon pendant 15 minutes, puis chauffé à 80°C pendant 16 heures. Le milieu réactionnel est ensuite filtré à température ambiante. Le filtrat est alors hydrolysé et extrait avec CH₂Cl₂. Les phases organiques sont regroupées, lavées avec une solution saturée de NaCl, puis séchées sur sulfate de magnésium et concentrées à sec. L'huile verte résultante est alors purifiée par chromatographie sur gel de silice (heptane /AcOEt : 95/5) pour conduire au composé du titre sous forme d'un solide blanc.

### Stade D: Acide (4aR)-3-{[2-(4-chlorophényl)-5,5-diméthyl-1-cyclohexén-1-yl]méthyl}-2,3,4,4a,5,6-hexahydro-1H-pyrazino[1,2-a]quinoline-8-carboxylique, chlorhydrate

Une suspension de 0,910 g du composé du Stade C dans un mélange dioxane/ HCl, 6N (10 mL/ 15 mL) est porté à 70°C pendant 20 heures. On laisse ensuite revenir le milieu réactionnel à température ambiante. Le précipité obtenu est alors filtré, puis lavé avec l'ether diisopropylique et séché.On obtient le composé du titre sous forme d'un solide vert clair.

### Préparation 24: Acide (10aα)-2-{[2-(4-Chlorophényl)-5,5-diméthyl-1-cyclohexén-1-yl]méthyl}-1,2,3,4,10,10a-hexahydropyrazino[1,2-a]indole-8-carboxylique (α=R ou S)

### Stade A: 2-{[2-(4-Chlorophényl)-5,5-diméthyl-1-cyclohexén-1-yl]méthyl}-8-méthoxy-1,2,3,4-tétrahydropyrazino[1,2-a]indole

On applique au composé obtenu au Stade C de la Préparation 9 les procédés développés aux Stades B et C de la Préparation 23.

### Stade B: 2-{[2-(4-Chlorophényl)-5,5-diméthyl-1-cyclohexén-1-yl]méthyl}-1,2,3,4-tétrahydropyrazino[1,2-a]indole-8-carboxylate de méthyle

On applique au composé du Stade A les procédés des Stades E et F de la Préparation 9.

### Stade C: Acide 2-{[2-(4-chlorophényl)-5,5-diméthyl-1-cyclohexén-1-yl]méthyl}-1,2,3,4,10,10a-hexahydropyrazino[1,2-a]indole-8-carboxylique, chlorhydrate

On applique au composé du Stade précédent les procédés des Stades B et C de la préparation 11.

### Stade D: Acide (10aα)-2-{[2-(4-chlorophényl)-5,5-diméthyl-1-cyclohexén-1-yl]méthyl}-1,2,3,4,10,10a-hexahydropyrazino[1,2-a]indole-8-carboxylique (α=R ou S)

Le composé est obtenu par séparation du mélange d'énantiomères obtenu au Stade C.

### Préparation 25: Acide (4aR)-3-{[4-(4-chlorophényl)-3-pyridyl]méthyl}-2,3,4,4a, 5,6-hexahydro-1H-pyrazino[1,2-a]quinoline-8-carboxylique, trifluoroacétate

### Stade A: (4-Bromo-3-pyridyl)méthanol

A une solution de 1 g de 4-bromonicotinaldéhyde dans 50 mL de MeOH placée à 0°C, on ajoute, par portion, 0,2 g de borohydrure de sodium. L'ensemble est ensuite agité progressivement à température ambiante durant 6 heures. Le milieu réactionnel est refroidi à 0°C, puis hydrolysé avec une solution saturée de NH₄Cl et extrait avec CH₂Cl₂. Les phases organiques sont alors regroupées, lavées avec une solution saturée de NaCl, séchées sur sulfate de magnésium, puis concentrées à sec. On obtient le composé du titre sous forme d'un gel marron clair utilisé tel que à l'étape suivante.

### Stade B: [4-(4-Chlorophényl)-3-pyridyl]méthanol

A une suspension de 0,545 g du composé du Stade A dans un mélange DME/ EtOH (7,5 mL/ 3 mL), on ajoute successivement 0,335g de Pd(Ph₃)₄, 0,453 g d'acide 4-chlorophényl boronique, puis 2,9 ml d'une solution aqueuse 2M de Na₂CO₃. L'ensemble est dégazé sous argon pendant 15 minutes puis chauffé à 80°C pendant 18 heures. Le milieu réactionnel est ensuite filtré à température ambiante. Le filtrat est alors hydrolysé et extrait avec CH₂Cl₂. Les phases organiques sont regroupées, lavées avec une solution saturée de NaCl, puis séchées sur sulfate de magnésium et concentrées à sec. Le solide résultant est finalement purifié par chromatographie sur gel de silice (CH₂Cl₂ / MeOH ) pour conduire au composé du titre.

### Stade C: 3-(Chlorométhyl)-4-(4-chlorophényl)pyridine

A une solution de 0,176 g du composé du Stade B dans 5 mL de CH₂Cl₂ placée à 0°C, on ajoute, goutte à goutte, une solution de 0,590 ml de chlorure de thionyle (0,008 mole) dans 5 mL de CH₂Cl₂. L'ensemble est agité progressivement à température ambiante durant 2 heures. Le milieu réactionnel est ensuite concentré à sec. Le solide obtenu est lavé à l'heptane et séché. On obtient le composé du titre sous forme d'un solide beige clair utilisé tel que à l'étape suivante.

### Stade D: (4aR)-3-{[4-(4-Chlorophényl)-3-pyridyl]méthyl}-2,3,4,4a,5,6-hexahydro-1H-pyrazino[1,2-a]quinoline-8-carboxylate de méthyle

On procède comme au Stade B de la Préparation 23 en faisant réagir le composé du Stade A de la Préparation 23 avec le composé du Stade C précédent.

### Stade E: Acide (4aR)-3-{[4-(4-chlorophényl)-3-pyridyl]méthyl}-2,3,4,4a,5,6-hexahydro-1H-pyrazino[1,2-a]quinoline-8-carboxylique, trifluoroacétate

On applique au composé précédent le procédé du Stade N de la Préparation 17. On obtient un dérivé non cristallin qui est purifié par chromatographie en phase inverse (C-18) (gradient H₂O, CH₃CN, 0,1% de TFA). Après lyophilisation, on obtient le produit du titre sous forme d'un sel de TFA.

### Préparation 26: Acide (4aR)-3-{[2-(4-chlorophényl)-3-pyridyl]méthyl}-2,3,4,4a, 5,6-hexahydro-1H-pyrazino[1,2-a]quinoline-8-carboxylique, trifluoroacétate

On procède comme à la préparation 25 en remplaçant le 4-bromonicotinaldéhyde par le 2-bromonicotinaldéhyde.

### Préparation 27: Acide (4aR)-3-{[3-(4-chlorophényl)-2-pyridyl]méthyl}-2,3,4,4a, 5,6-hexahydro-1H-pyrazino[1,2-a]quinoline-8-carboxylique, trifluoroacétate

On procède comme à la préparation 25 en remplaçant le 4-bromonicotinaldéhyde par le 3-bromo-2-pyridinecarbaldéhyde.

### Préparation 28: Acide (4aR)-3-{[3-(4-chlorophényl)-4-pyridyl]méthyl}-2,3,4,4a, 5,6-hexahydro-1H-pyrazino[1,2-a]quinoline-8-carboxylique, trifluoroacétate

On procède comme à la préparation 25 en remplaçant le 4-bromonicotinaldéhyde par le 3-bromoisonicotinaldéhyde.

### Préparation 29: Acide (4aR)-3-[(4-[(tert-butoxycarbonyl)amino]-4'-chloro[1,1'-biphényl]-2-yl)méthyl]-2,3,4,4a,5,6-hexahydro-1H-pyrazino[1,2-a]quinoline-8-carboxylique

### Stade A: 4-Nitro-4'-chloro[1,1'-biphényl]-2-carboxylate de méthyle

Ce composé est obtenu en utilisant la méthode de couplage décrite au Stade B de la Préparation 25 en remplaçant la (4-bromo-3-pyridyl)méthanol par le 2-bromo-5-nitro-benzoate de méthyle. On obtient le produit attendu après une étape de purification sur gel de silice (éther de pétrole/AcOEt) sous forme d'un solide jaune.

### Stade B: 4-Nitro-4'-chloro[1,1'-biphén-2-yl]méthanol

A une solution de 2,38 g du composé du Stade A dans 20 mL de MeOH placée à 0°C, on ajoute, par portion, 0,617 g de borohydrure de sodium 0,617 g. L'ensemble est agité progressivement à température ambiante durant 6 heures, puis chauffé à reflux pendant 24 heures. Le milieu réactionnel est ensuite refroidi à 0°C, hydrolysé avec une solution saturée de NH₄Cl et extrait avec CH₂Cl₂. Les phases organiques sont alors regroupées et lavées avec une solution saturée de NaCl, séchées sur sulfate de magnésium puis concentrées à sec. Après purification sur gel de silice (éther de pétrole / AcOEt), on obtient le composé attendu sous forme d'un solide jaune.

### Stade C: 4-Amino-4'-chloro[1,1'-biphén-2-yl]méthanol

A une solution de 0,890 g du composé du Stade B dans un mélange THF (15 ml) / MeOH (20 ml), on ajoute, par portion, 3,8 g de chlorure stannique (SnCl₂). L'ensemble est agité progressivement à reflux durant 3 heures. Le milieu réactionnel est ensuite concentré à sec, repris avec CH₂Cl₂, refroidi à 0°C avant d'être hydrolysé avec une solution de NaOH, 5N et extrait avec CH₂Cl₂. Les phases organiques sont alors regroupées, lavées avec une solution saturée de NaCl, séchées sur sulfate de magnésium puis concentrées à sec. On obtient le composé attendu sous forme d'un solide jaune utilisé tel que à l'étape suivante.

### Stade D: 4-[(tert-Butoxycarbonyl)amino]-4'-chloro-2-(hydroxyméthyl)-1,1'-biphényl

A une solution de 0,76 g du composé du Stade C dans 25 ml d'éthanol, on ajoute 0,71 g de Boc₂O. L'ensemble est agité progressivement à 35°C durant 20 heures. Le milieu réactionnel est ensuite concentré à sec, repris avec Et₂O, puis hydrolysé et extrait avec Et₂O. Les phases organiques sont alors regroupées, lavées avec une solution saturée de NaCl, séchées sur sulfate de magnésium, puis concentrées à sec. Après purification sur gel de silice (éther de pétrole / AcOEt), on obtient le composé du titre sous forme d'un solide beige.

### Stade E: 4-[(tert-Butoxycarbonyl)amino]-4'-chloro-2-(chlorométhyl)-1,1'-biphényl

A une solution de 0,634 g du composé du Stade D dans 15 ml de THF placée à 0°C, on ajoute successivement 0,532 mL d' Et₃N, 0,22 ml de chlorure de mésyle (0,00284 mole). L'ensemble est ensuite agité progressivement à température ambiante durant 96 heures.

Puis, le milieu réactionnel est concentré à sec. Après purification sur gel de silice (éther de pétrole / AcOEt), on obtient le composé attendu sous forme d'une huile jaune qui cristallise.

### Stade F: (4aR)-3-({4-[(tert-Butoxycarbonyl)amino]-4'-chloro[1,1'-biphényl]-2-yl} méthyl)-2,3,4,4a,5,6-hexahydro-1H-pyrazino[1,2-a]quinoline-8-carboxylate de méthyle

On procède comme au Stade B de la Préparation 23 en faisant réagir le composé du Stade A de la Préparation 23 avec le composé du Stade E précédent.

### Stade G: Acide (4aR)-3-[(4-[(tert-butoxycarbonyl)amino]-4'-chloro[1,1'-biphényl]-2-yl)méthyl] -2,3,4,4a,5,6-hexahydro-1H-pyrazino[1,2-a]quinoline-8-carboxylique

On applique au composé précédent le procédé du Stade N de la Préparation 17.

### Préparation 30: Acide (4aR)-3-[(4-{[(tert-butoxycarbonyl)amino]méthyl}-4'-chloro[1,1'-biphényl]-2-yl)méthyl]-2,3,4,4a,5,6-hexahydro-1H-pyrazino[1,2-a] quinoline-8-carboxylique

### Stade A: 4-Méthoxy-4'-chloro[1,1'-biphényl]-2-carboxylate de méthyle

Ce composé est obtenu en utilisant la méthode de couplage décrite au Stade B de la Préparation 25 en remplaçant la (4-bromo-3-pyridyl)méthanol par le 2-bromo-5-méthoxy-benzoate de méthyle. On obtient le produit attendu après une étape de purification sur gel de silice (heptane/AcOEt) sous forme d'un solide.

### Stade B: 4'-Chloro-4-hydroxy-[1,1'-biphényl]-2-carboxylate de méthyle

A une solution de 1,6 g du composé du Stade A dans 20 ml de CH₂Cl₂ placée à -78°C, on ajoute lentement une solution 1 M de BBr₃ dans 42 ml de CH₂Cl₂. L'ensemble est agité à cette température pendant 1h30. On ajoute ensuite un mélange H₂O/ MeOH (40 mL/ 10 mL). L'ensemble est agité durant 45 minutes toujours à -78°C, puis extrait au CH₂Cl₂.Les phases organiques sont alors regroupées et séchées sur sulfate de magnésium avant d'être concentrées à sec. On obtient une mousse brune qui est utilisée telle que à l'étape suivante.

### Stade C: 4'-Chloro-4-trifluorométhanesulfonyl-[1,1'-biphényl]-2-carboxylate de méthyle

A une solution de 1,5 g du composé du Stade B dans 20 ml de CH₂Cl₂ placée à 0°C, on ajoute successivement 4 ml d'Et₃N (0,029 mol), 3,1 g de N-phényl-bis(trifluorométhane sulfonamide). Puis, l'ensemble est agité progressivement à température ambiante durant 20 h. Le milieu réactionnel est ensuite concentré à sec, repris avec AcOEt et lavé successivement avec une solution de HCl, 1N, une solution saturée de NaHCO₃ et une solution saturée de NaCl, puis séché sur sulfate de magnésium. On obtient le composé attendu après purification sur gel de silice (heptane/ AcOEt) une huile incolore.

### Stade D: 4'-Chloro-4-cyano-[1,1'-biphényl]-2-carboxylate de méthyle

A une solution de 1,2 g du composé du Stade C dans 50 ml de DMF, on ajoute successivement 0,44 g de Pd₂(dba)₃, 0,066 g de dppf, et 0,422 g Zn(CN)₂. Puis, l'ensemble est agité progressivement à 90°C durant 3 heures. Le milieu réactionnel est ensuite concentré, repris avec AcOEt et lavé successivement avec une solution saturée de LiCl et une solution saturée de NaCl avant d'être séché sur sulfate de magnésium. On obtient le composé attendu après purification sur gel de silice (heptane/ AcOEt) sous forme d'une huile incolore qui cristallise progressivement.

### Stade E: 4-Aminométhyl-4'-chloro-[1,1 '-biphényl]-2-carboxylate de méthyle

A une suspension de 3,52 g du composé du Stade D dans 40 mL de MeOH placée à 0°C, on ajoute 1,68 g de NiCl₂, et 1,47 g de borohydrure de sodium par portion. L'ensemble est ensuite agité progressivement à température ambiante durant 6 h. Le milieu réactionnel est ensuite filtré, et le filtrat est dilué avec AcOEt avant d'être hydrolysé. Les phases organiques sont alors regroupées, lavées avec une solution saturée de NaCl, séchées sur sulfate de magnésium puis concentrées. On obtient le composé du titre sous forme d'une mousse blanche utilisée telle que à l'étape suivante.

### Stade F: 4-{[(tert-Butoxycarbonyl)amino]méthyl}-4'-chloro[1,1'-biphényl]-2-carboxylate de méthyle

A une solution de 3,02 g du composé du Stade E dans 60 ml de CH₂Cl₂, on ajoute 2,82g de Boc₂O. Puis, l'ensemble est agité à température ambiante durant 20 heures. Le milieu réactionnel est ensuite concentré à sec. Après purification sur gel de silice (éther de pétrole/ AcOEt); le composé du titre est obtenu sous forme d'un solide blanc.

### Stade G: 4-{[(tert-Butoxycarbonyl)amino]méthyl}-4'-chloro-2-(hydroxyméthyl)-1,1'-biphényl

A une solution de 1,6 g du composé du Stade F placée à 0°C dans 60 ml de THF, on ajoute, goutte à goutte, une solution 2,4M de LAH dans le THF. L'ensemble est agité à température ambiante durant 2 h. Le milieu réactionnel est ensuite hydrolysé avec une solution saturée de sel de Rochelle à température ambiante pendant 1h30. Il est ensuite extrait avec AcOEt. Les extraits organiques sont alors regroupés, lavés avec une solution saturée de NaCl et séchés sur sulfate de magnésium, puis concentrés à sec. Après purification sur gel de silice (éther de pétrole/ AcOEt), le composé du titre est obtenu sous forme d'une huile translucide.

### Stade H: 4-{[(tert-Butoxycarbonyl)amino]méthyl}-4'-chloro-2-(chlorométhyl)-1,1'-biphényl

A une solution de 1,56 g du composé du Stade G dans 50 ml de THF placée à 0°C, on ajoute successivement 1,26 ml d'Et₃N (0,00896 mole), 0,52 mL de chlorure de mésyle (0,00672 mole). Puis, l'ensemble est agité progressivement à température ambiante durant 96 h. Le milieu réactionnel est ensuite concentré à sec. Après purification sur gel de silice (éther de pétrole/ AcOEt), le composé attendu est obtenu sous forme d'une huile qui cristallise.

### Stade I: (4aR)-3-[(4-{[(tert-Butoxycarbonyl)amino]méthyl}-4'-chloro[1,1'-biphényl]-2-yl)méthyl]-2,3,4,4a,5,6-hexahydro-1H-pyrazino[1,2-a]quinoline-8-carboxylate de méthyle

On procède comme au Stade B de la Préparation 23 en faisant réagir le composé du Stade A de la Préparation 23 avec le composé du Stade H précédent. Après une étape de purification sur gel de silice (heptane / AcOEt), on obtient le composé du titre.

### Stade J: Acide (4aR)-3-[(4-{[(tert-butoxycarbonyl)amino]méthyl}-4'-chloro[1,1'-biphényl]-2-yl)méthyl]-2,3,4,4a,5,6-hexahydro-1H-pyrazino[1,2-a]quinoline-8-carboxylique

On applique au composé précédent le procédé du Stade N de la Préparation 17. Le produit attendu est obtenu sous forme d'un solide blanc.

### Préparation 31: Acide (4aR)-3-[(3'-fluoro-4'-chloro[1,1'-biphényl]-2-yl)méthyl]-2,3,4,4a,5,6-hexahydro-1H-pyrazino[1,2-a]quinoline-8-carboxylique

### Stade A: (4aR)-3-(2-Bromobenzyl)-2,3,4,4a,5,6-hexahydro-1H-pyrazino[1,2-a]quinoline-8-carboxylate de méthyle

On procède comme au Stade B de la Préparation 23 en faisant réagir le composé du Stade A de la Préparation 23 avec le 1-bromo-2-(bromométhyl)benzène.

### Stade B: (4aR)-3-[(3'-Fluoro-4'-chloro[1,1'-biphényl]-2-yl)méthyl]-2,3,4,4a,5,6-hexahydro-1H-pyrazino[1,2-a]quinoline-8-carboxylate de méthyle

On procède comme au Stade B de la Préparation 25 en remplaçant l'acide 4-chlorophényl boronique par l'acide 3-fluoro-4-chlorophényl boronique.

### Stade C: Acide (4aR)-3-[(3'-Fluoro-4'-chloro[1,1'-biphényl]-2-yl)méthyl]-2,3,4,4a, 5,6-hexahydro-1H-pyrazino[1,2-a]quinoline-8-carboxylique

On applique au composé précédent le procédé du Stade N de la Préparation 17.

### Préparation 32: Acide (4aR)-3-[(4'-cyano[1,1'-biphényl]-2-yl)méthyl]-2,3,4,4a,5,6-hexahydro-1H-pyrazino[1,2-a]quinoline-8-carboxylique

On procède comme à la Préparation 31 en remplaçant l'acide 3-fluoro-4-chlorophényl boronique au stade B par l'acide 4-cyano boronique.

### Préparation 33: Acide (4aR)-3-[(4-trifluoro[1,1'-biphényl]-2-yl)méthyl]-2,3,4,4a,5,6-hexahydro-1H-pyrazino[1,2-a]quinoline-8-carboxylique

On procède comme à la Préparation 31 en remplaçant l'acide 3-fluoro-4-chlorophényl boronique au stade B par l'acide 4-trifluorométhyl boronique.

### Préparation 34: Acide (4aR)-3-[2-(1,3-benzodioxol-5-yl)benzyl]-2,3,4,4a,5,6-hexahydro-1H-pyrazino[1,2-a]quinoline-8-carboxylique

On procède comme à la Préparation 31 en remplaçant l'acide 3-fluoro-4-chlorophényl boronique au stade B par l'acide 1,3-benzodioxol-5-yl boronique.

### Préparation 35: Acide (4aR)-3-benzhydryl-2,3,4,4a,5,6-hexahydro-1H-pyrazino[1,2-a]quinoline-8-carboxylique

### Stade A: (4aR)-3-Benzhydryl-2,3,4,4a,5,6-hexahydro-1H-pyrazino[1,2-a]quinoline-8-carboxylate de méthyle

On procède comme au Stade B de la Préparation 23 en faisant réagir le composé du Stade A de la Préparation 23 avec le [bromo(phényl)méthyl]benzène.

### Stade B: Acide (4aR)-3-benzhydryl-2,3,4,4a,5,6-hexahydro-1H-pyrazino[1,2-a] quinoline-8-carboxylique

On applique au composé précédent le procédé du Stade N de la Préparation 17.

### Préparation 36: Acide (4aS,R)-3-{[4-(4-chlorophényl)-3-pyridyl]méthyl}-2,3,4,4a, 5,6-hexahydro-1H-pyrazino[1,2-a]quinoline-8-carboxylique, trifluoroacétate

On procède comme à la Préparation 25 en remplaçant au Stade D le composé du Stade A de la Préparation 23 par le mélange énantiomérique du Stade H de la Préparation 1.

### Préparation 37: Acide (4aS,R)-3-{[2-(4-chlorophényl)-3-pyridyl]méthyl}-2,3,4,4a, 5,6-hexahydro-1H-pyrazino[1,2-a]quinoline-8-carboxylique, trifluoroacétate

On procède comme à la Préparation 26 en remplaçant au Stade D le composé du Stade A de la Préparation 23 par le composé du Stade H de la Préparation 1.

### Préparation 38: Acide (4aS,R)-3-{[3-(4-chlorophényl)-2-pyridyl]méthyl}-2,3,4,4a, 5,6-hexahydro-1H-pyrazino[1,2-a]quinoline-8-carboxylique, trifluoroacétate

On procède comme à la Préparation 27 en remplaçant au Stade D le composé du Stade A de la Préparation 23 par le composé du Stade H de la Préparation 1.

### Préparation 39: Acide (4aS,R)-3-{[3-(4-chlorophényl)-4-pyridyl]méthyl}-2,3,4,4a, 5,6-hexahydro-1H-pyrazino[1,2-a]quinoline-8-carboxylique, trifluoroacétate

On procède comme à la Préparation 28 en remplaçant au Stade D le composé du Stade A de la Préparation 23 par le composé du Stade H de la Préparation 1.

### Préparation 40: Acide (4aS,R)-3-[(2-(4-pyridyl)-3-pyridyl)méthyl]-2,3,4,4a,5,6-hexahydro-1H-pyrazino[1,2-a]quinoline-8-carboxylique, trifluoroacétate

On procède comme à la Préparation 25 en utilisant le 2-bromo-nicotinaldéhyde au Stade A, l'acide 4-pyridyl boronique au Stade B et en prenant au Stade D comme synthon tricyclique le mélange énantiomérique du Stade H de la Préparation 1.

### Préparation 41: Acide (4aS,R)-3-[(2-(6-chloro-pyridin-3-yl)-3-pyridyl)méthyl]-2,3,4,4a,5,6-hexabydro-1H-pyrazino[1,2-a]quinoline-8-carboxylique, trifluoroacétate

On procède comme à la Préparation 25 en utilisant le 2-bromo-nicotinaldéhyde au Stade A, l'acide 6-chloro-3-pyridyl boronique au Stade B et en prenant au Stade D comme synthon tricyclique le mélange énantiomérique du Stade H de la Préparation 1.

### Préparation 42: Acide (4aS,R)-3-[(2-(6-hydroxy-pyridin-3-yl)-3-pyridyl)méthyl] - 2,3,4,4a,5,6-hexahydro-1H-pyrazino[1,2-a]quinoline-8-carboxylique, trifluoroacétate

On procède comme à la Préparation 25 en utilisant le 2-bromo-nicotinaldéhyde au Stade A, l'acide 6-hydroxy-3-pyridyl boronique au Stade B et en prenant au Stade D comme synthon tricyclique le mélange énantiomérique du Stade H de la Préparation 1.

### Préparation 43: Acide 2-{[2-(4-chlorophényl)-3-pyridyl]méthyl}-1,2,3,4-tétrahydropyrazino[1,2-a]indole-8-carboxylique, trifluoroacétate

On procède comme à la Préparation 25 en utilisant le 2-bromonicotinaldéhyde au Stade A, l'acide 4-chlorophényl boronique au Stade B et en prenant au Stade D comme synthon tricyclique le composé du Stade F de la Préparation 9.

### Préparation 44: Acide 2-{[2-(6-chloro-pyridin-3-yl)-3-pyridyl]méthyl}-1,2,3,4-tétrahydropyrazino[1,2-a]indole-8-carboxylique, trifluoroacétate

On procède comme à la Préparation 25 en utilisant le 2-bromonicotinaldéhyde au Stade A, l'acide 6-chloro-3-pyridyl boronique au Stade B et en prenant au Stade D comme synthon tricyclique le composé du Stade F de la Préparation 9.

### Préparation 45: Acide (4aS,R)-3-[(4'-tert-butyl-[1,1'-biphényl]-2-yl)méthyl]-2,3,4,4a,5,6-hexahydro-1H-pyrazino[1,2-a]quinoline-8-carboxylique

On procède comme à la Préparation 31 en remplaçant l'acide 3-fluoro-4-chlorophényl boronique au stade B par l'acide 4-tert-butyl-phényl boronique, et en remplaçant au Stade C le (4a*R*)-2,3,4,4a,5,6-hexahydro-1*H*-pyrazino[1,2-*a*]quinoline-8-carboxylate de méthyle (Stade A de la Préparation 23) par le mélange (4a*S*,*R*)-2,3,4,4a,5,6-hexahydro-1*H* pyrazino[1,2-*a*]quinoline-8-carboxylate de méthyle (Stade H de la Préparation 1).

### Préparation 46: Acide (4aS,R)-3-[2-(4-chlorobenzyl)benzyl]-2,3,4,4a,5,6-hexahydro-1H-pyrazino[1,2-a]quinoline-8-carboxylique

On procède comme dans la Préparation 1 en remplaçant au Stade I le 4-chloro-2'-(chlorométhyl)-1,1'-biphényle par le 1-chloro-2-(4-chlorobenzyl)benzène.

### Préparation 47: Acide 3-(2-phénoxybenzyl)-2,3,4,4a,5,6-hexahydro-1H-pyrazino[1,2-a]quinoline-8-carboxylique

### Stade A : 3-(2-Phénoxybenzyl)-2,3,4,4a,5,6-hexahydro-1H-pyrazino[1,2-a]quinoline-8-carboxylate de méthyle

On soumet le composé du Stade H de la Préparation 1 à une amination réductive en le faisant réagir avec le 2-phénoxybenzaldéhyde en présence de NaBH(OAc)₃. Le milieu réactionnel est ensuite traité en présence d'acide acétique, puis extrait avec CH₂Cl₂.

### Stade B : Acide 3-(2-phénoxybenzyl)-2,3,4,4a,5,6-hexahydro-1H-pyrazino[1,2-a] quinoline-8-carboxylique

On procède comme dans le Stade J de la Préparation 1.

### Exemple 1 : N-({(4aS,R)-3-[(4'-Chloro[1,1'-biphényl]-2-yl)méthyl]-2,3,4,4a,5,6-hexahydro-1H-pyrazino[1,2-a]quinolin-8-yl}carbonyl)-4-({(1R)-3-(diméthylamino)-1-[(phénylsulfanyl)méthyl]propyl}amino)-3-nitrobenzènesulfonamide, bistrifluoroacétate

### Stade A : N-({(4aS,R)-3-[(4'-Chloro[1,1'-biphényl]-2-yl)méthyl]-2,3,4,4a,5,6-hexahydro-1H-pyrazino[1,2-a]quinolin-8-yl}carbonyl)-4-({(1R)-3-(diméthylamino)-1-[(phénylsulfanyl)méthyl]propyl}amino)-3-nitrobenzènesulfonamide

A une solution de 1,26 g du composé obtenu dans la Préparation 1 dans 50 ml d'un mélange CH₂Cl₂/THF(1/1), on ajoute à température ambiante 2,05 ml de DIEA puis 1,5 g de 4-({(1*R*)-3-(diméthylamino)-1-[(phénylsulfanyl)méthyl]propyl}amino)-3-nitrobenzènesulfonamide puis 0,783 g d'EDC et 0,5 g de DMAP. Le milieu réactionnel est agité à température ambiante pendant 2 jours. On évapore à sec puis on reprend le résidu obtenu dans une solution saturée en NH₄Cl et on extrait 2 fois avec CH₂Cl_{2.} On lave la phase organique avec une solution saturée en NaCl, puis sèche sur MgSO₄, on filtre et on évapore à sec. L'huile obtenue est purifiée par chromatographie flash sur gel de silice (CH₂Cl₂/MeOH/NH₄OH 84/16/1,6) puis lyophilisé pour conduire au produit du titre sous la forme d'un solide jaune.

| *Microanalyse élémentaire :* | | | | |
|---|---|---|---|---|
| | *% C* | *%H* | *%N* | *%S* |
| *Calculé* | *62,95* | *5,64* | *10,01* | *7,64* |
| *Trouvé* | *63,20* | *5,62* | *9, 78* | *7, 22* |

### Stade B : N-({(4aS,R)-3-[(4'-Chloro[1,1'-biphényl]-2-yl)méthyl]-2,3,4,4a,5,6-hexahydro-1H-pyrazino[1,2-a]quinolin-8-yl}carbonyl)-4-({(1R)-3-(diméthylamino)-1-[(phénylsulfanyl)méthyl]propyl}amino)-3-nitrobenzènesulfonamide, bistrifluoroacétate

Le composé obtenu au Stade A (0,3 g) est solubilisé dans 10 ml de CH₂Cl₂ placés à 0°C, puis on ajoute goutte à goutte l'acide trifluoroacétique (56 µl). L'ensemble est ensuite agité à température ambiante pendant 30 minutes, puis concentré à sec. Le solide résultant est alors repris dans H₂O et l'on ajoute goutte à goutte du CH₃CN jusqu'à dissolution complète du milieu réactionnel, qui est ensuite lyophilisé à basse température pendant 24 heures. On obtient un solide cotonneux jaune correspondant au produit du titre.

### Exemple 2 : N-({(4aS,R)-3-[(4'-Chloro[1,1'-biphényl]-3-yl)méthyl]-2,3,4,4a,5,6-hexahydro-1H-pyrazino[1,2-a]quinolin-8-yl}carbonyl)-3-nitro-4-{[2-(phénylsulfanyl)éthyl]amino}benzènesulfonamide, chlorhydrate

### Stade A : N-({(4aS,R)-3-[(4'-Chloro[1,1'-biphényl]-3-yl)méthyl]-2,3,4,4a,5,6-hexahydro-1H-pyrazino[1,2-a]quinolin-8-yl}carbonyl)-3-nitro-4-{[2-(phénylsulfanyl) éthyl]amino}benzènesulfonamide

On procède comme dans le Stade A de l'Exemple 1 en remplaçant le composé de la Préparation 1 par le composé de la Préparation 2, et en remplaçant le 4-({(1*R*)-3-(diméthylamino)-1-[(phénylsulfanyl)méthyl]propyl}amino)-3-nitrobenzènesulfonamide par le 3-nitro-4-{[2-(phénylsulfanyl)éthyl]amino}benzènesulfonamide.

### Stade B : N-({(4aS,R)-3-[(4'-Chloro[1,1'-biphényl]-3-yl)méthyl]-2,3,4,4a,5,6-hexahydro-1H-pyrazino[1,2-a]quinolin-8-yl}carbonyl)-3-nitro-4-{[2-(phénylsulfanyl)éthyl]amino}benzènesulfonamide, chlorhydrate

Le composé obtenu au Stade A (0,3 g) est solubilisé dans 10 ml de CH₂Cl₂ placés à 0°C, puis on ajoute goutte à goutte une solution d'acide chlorhydrique dans Et₂O (2M) (375 µl). L'ensemble est ensuite agité à température ambiante pendant 30 minutes, puis concentré à sec. Le solide obtenu est alors repris dans H₂O et l'on ajoute goutte à goutte du CH₃CN jusqu'à dissolution complète du milieu réactionnel, qui est ensuite lyophilisé à basse température pendant 24 heures.

### Exemple 3 : N-({(4aS,R)-3-[(4'-Chloro[1,1'-biphényl]-4-yl)méthyl]-2,3,4,4a,5,6-hexahydro-1H-pyrazino[1,2-a]quinolin-8-yl}carbonyl)-3-nitro-4-{[2-(phénylsulfanyl)éthyl]amino}benzènesulfonamide, chlorhydrate

### Stade A : N-({(4aS,R)-3-[(4'-Chloro[1,1'-biphényl]-4-yl)méthyl]-2,3,4,4a,5,6-hexahydro-1H-pyrazino[1,2-a]quinolin-8-yl}carbonyl)-3-nitro-4-{[2-(phénylsulfanyl) éthyl]amino}benzènesulfonamide

On procède comme dans le Stade A de l'Exemple 1 en remplaçant le composé de la Préparation 1 par le composé de la Préparation 3, et en remplaçant le 4-({(1*R*)-3-(diméthylamino)-1-[(phénylsulfanyl)méthyl]propyl}amino)-3-nitrobenzènesulfonamide par le 3-nitro-4-{[2-(phénylsulfanyl)éthyl]amino}benzènesulfonamide.

### Stade B : N-({(4aS,R)-3-[(4'-Chloro[1,1'-biphényl]-4-yl)méthyl]-2,3,4,4a,5,6-hexahydro-1H-pyrazino[1,2-a]quinolin-8-yl}carbonyl)-3-nitro-4-{[2-(phénylsulfanyl) éthyl]amino}benzènesulfonamide, chlorhydrate

On procède comme dans le Stade B de l'Exemple 2.

### Exemple 4 : N-{[(4aS,R)-3-([1,1'-Biphényl]-2-ylméthyl)-2,3,4,4a,5,6-hexahydro-1H-pyrazino[1,2-a]quinolin-8-yl]carbonyl}-4-({(1R)-3-(diméthylamino)-1-[(phénylsulfanyl)méthyl]propyl}amino)-3-nitrobenzènesulfonamide, bis(chlorhydrate)

### Stade A : N-{[(4aS,R)-3-([1,1'-Biphényl]-2-ylméthyl)-2,3,4,4a,5,6-hexahydro-1H-pyrazino[1,2-a]quinolin-8-yl]carbonyl}-4-({(1R)-3-(diméthylamino)-1-[(phénylsulfanyl)méthyl]propyl}amino)-3-nitrobenzènesulfonamide

On procède comme dans le Stade A de l'Exemple 1 en remplaçant le composé de la Préparation 1 par le composé de la Préparation 4.

| *Microanalyse élémentaire :* | | | | |
|---|---|---|---|---|
| | *%C* | *%H* | *%N* | *%S* |
| *Calculé* | *65, 65* | *6, 01* | *10,44* | *7,97* |
| *Trouvé* | *65, 24* | *5,94* | *10, 24* | *7,89* |

### Stade B : N-{[(4aS,R)-3-([1,1'-Biphényl]-2-ylméthyl)-2,3,4,4a,5,6-hexahydro-1H-pyrazino[1,2-a]quinolin-8-yl]carbonyl}-4-({(1R)-3-(diméthylamino)-1-[(phénylsulfanyl)méthyl]propyl}amino)-3-nitrobenzènesulfonamide, bis(chlorhydrate)

On procède comme dans le Stade B de l'Exemple 2.

| *Microanalyse élémentaire :* | | | | |
|---|---|---|---|---|
| | *% C* | *%H* | *%N* | *%S* |
| *Calculé* | *59,21* | *5,69* | *9, 42* | *7,19* |
| *Trouvé* | *58,54* | *5,92* | *9, 06* | *6,50* |

### Exemple 5 : N-{[(4aS,R)-3-(2-Benzylbenzyl)-2,3,4,4a,5,6-hexahydro-1H-pyrazino[1,2-a]quinolin-8-yl]carbonyl}-3-nitro-4-{[2-(phénylsulfanyl)éthyl] amino}benzènesulfonamide, chlorhydrate

### Stade A : N-{[(4aS,R)-3-(2-Benzylbenzyl)-2,3,4,4a,5,6-hexahydro-1H-pyrazino[1,2-a]quinolin-8-yl]carbonyl}-3-nitro-4-{[2-(phénylsulfanyl)éthyl] amino}benzènesulfonamide

On procède comme dans le Stade A de l'Exemple 1 en remplaçant le composé de la Préparation 1 par le composé de la Préparation 5, et en remplaçant le 4-({(1*R*)-3-(diméthylamino)-1-[(phénylsulfanyl)méthyl]propyl}amino)-3-nitrobenzènesulfonamide par le 3-nitro-4-{[2-(phénylsulfanyl)éthyl]amino}benzènesulfonamide.

### Stade B : N-{[(4aS,R)-3-(2-Benzylbenzyl)-2,3,4,4a,5,6-hexahydro-1H-pyrazino[1,2-a]quinolin-8-yl]carbonyl}-3-nitro-4-{[2-(phénylsulfanyl)éthyl] amino}benzènesulfonamide, chlorhydrate

On procède comme dans le Stade B de l'Exemple 2.

### Exemple 6 : 3-Nitro-N-({(4aS,R)-3-[2-(2-phénytéthyl)benzyl]-2,3,4,4a,5,6-hexahydro-1H-pyrazino[1,2-a]quinolin-8-yl}carbonyl)-4-{[2-(phénylsulfanyl) éthyl] amino}benzènesulfonamide

On procède comme dans le Stade A de l'Exemple 1 en remplaçant le composé de la Préparation 1 par le composé de la Préparation 6, et en remplaçant le 4-({(1*R*)-3-(diméthylamino)-1-[(phénylsulfanyl)méthyl]propyl}amino)-3-nitrobenzènesulfonamide par le 3-nitro-4-{[2-(phénylsulfanyl)éthyl]amino}benzènesulfonamide.

| *Microanalyse élémentaire :* | | | | |
|---|---|---|---|---|
| | *%C* | *%H* | *%N* | *%S* |
| *Calculé* | *66,21* | *5,69* | *9,19* | *8, 42* |
| *Trouvé* | *65,98* | *5,93* | *8,89* | *8, 08* |

### Exemple 7 : N-({(4aS,R)-3-[(4'-Chloro[1,1'-biphényl]-2-yl)méthyl]-2,3,4,4a,5,6-hexahydro-1H-pyrazino[1,2-a]quinolin-8-yl}carbonyl)-3-nitro-4-{[2-(phénylsulfanyl) éthyl]amino}benzènesulfonamide

On procède comme dans le Stade A de l'Exemple 1 en remplaçant le 4-({(1*R*)-3-(diméthylamino)-1-[(phénylsulfanyl)méthyl]propyl}amino)-3-nitrobenzènesulfonamide par le 3-nitro-4-{[2-(phénylsulfanyl)éthyl]amino}benzènesulfonamide.

| *Microanalyse élémentaire :* | | | | |
|---|---|---|---|---|
| | *% C* | *%H* | *%N* | *%S* |
| *Calculé* | *62,53* | *4,98* | *9,11* | *8,35* |
| *Trouvé* | *62,53* | *5,12* | *8,70* | *8,12* |

### Exemple 8 : N-({(4aS,R)-3-[(4'-Chloro[1,1'-biphényl]-2-yl)méthyl]-2,3,4,4a,5,6-hexahydro-1H-pyrazino[1,2-a]quinolin-8-yl}carbonyl)-3-nitro-4-[(2-phénoxyéthyl) amino]benzènesulfonamide

On procède comme dans le Stade A de l'Exemple 1 en remplaçant le 4-({(1*R*)-3-(diméthylamino)-1-[(phénylsulfanyl)méthyl]propyl}amino)-3-nitrobenzènesulfonamide par le 3-nitro-4-[(2-phénoxyéthyl)amino]benzènesulfonamide.

| *Microanalyse élémentaire :* | | | | |
|---|---|---|---|---|
| | *%C* | *%H* | *%N* | *%S* |
| *Calculé* | *63,86* | *5,09* | *9,31* | *4,26* |
| *Trouvé* | *63,82* | *5, 23* | *8,98* | *3,82* |

### Exemple 9 : N-{[(4aS,R)-3-([1,1'-Biphényl]-2-ylméthyl)-2,3,4,4a,5,6-hexahydro-1H-pyrazino[1,2-a]quinolin-8-yl]carbonyl}-3-nitro-4-[(3-phénylpropyl) amino]benzènesulfonamide

On procède comme dans le Stade A de l'Exemple 1 en remplaçant le composé obtenu dans la Préparation 1 par le composé obtenu dans la Préparation 4, et en remplaçant le 4-({(1*R*)-3-(diméthylamino)-1-[(phénylsulfanyl)méthyl]propyl}amino)-3-nitrobenzènesulfonamide par le 3-nitro-4-[(3-phénylpropyl)amino]benzènesulfonamide.

| *Microanalyse élémentaire :* | | | | |
|---|---|---|---|---|
| | *% C* | *%H* | *%N* | *%S* |
| *Calculé* | *68,79* | *5,77* | *9,78* | *4,48* |
| *Trouvé* | *68,15* | *5,85* | *9,76* | *3, 63* |

### Exemple 10: 4-[(2-Anilinoéthyl)amino]-N-{[(4aS,R)-3-([1,1'-biphényl]-2-ylméthyl)-2,3,4,4a,5,6-hexahydro-1H-pyrazino[1,2-a]quinolin-8-yl]carbonyl}-3-nitrobenzènesulfonamide

On procède comme dans le Stade A de l'Exemple 1 en remplaçant le composé obtenu dans la Préparation 1 par le composé obtenu dans la Préparation 4, et en remplaçant le 4-({(1*R*)-3-(diméthylamino)-1-[(phénylsulfanyl)méthyl]propyl}amino)-3-nitrobenzènesulfonamide par le 4-[(2-anilinoéthyl)amino]-3-nitrobenzènesulfonamide.

| *Microanalyse élémentaire :* | | | | |
|---|---|---|---|---|
| | *%C* | *%H* | *%N* | *%S* |
| *Calculé* | *67,02* | *5,62* | *11,72* | *4,47* |
| *Trouvé* | *65,93* | *5,80* | *11,61* | *3,72* |

### Exemple 11 : N-{[(4aS,R)-3-([1,1'-Biphényl]-2-ylméthyl)-2,3,4,4a,5,6-hexahydro-1H-pyrazino[1,2-a]quinolin-8-yl]carbonyl}-4-{[3-(diméthylamino)propyl] [2-(phénylsulfanyl)éthyl]amino}-3-nitrobenzènesulfonamide, chlorhydrate

### Stade A : N-{[(4aS,R)-3-([1,1'-Biphényl]-2-ylméthyl)-2,3,4,4a,5,6-hexahydro-1H-pyrazino [1,2-a]quinolin-8-yl]carbonyl}-4-{[3-(diméthylamino)propyl][2-(phényl sulfanyl)éthyl]amino}-3-nitrobenzènesulfonamide

On procède comme dans le Stade A de l'Exemple 1 en remplaçant le composé obtenu dans la Préparation 1 par le composé obtenu dans la Préparation 4, et en remplaçant le 4-({(1*R*)-3-(diméthylamino)-1-[(phénylsulfanyl)méthyl]propyl}amino)-3-nitrobenzènesulfonamide par le 4-{[3-(diméthylamino)propyl][2-(phénylsulfanyl)éthyl]amino}-3-nitrobenzènesulfonamide.

### Stade B : N-{[(4aS,R)-3-([1,1'-Biphényl]-2-ylméthyl)-2,3,4,4a,5,6-hexahydro-1H-pyrazino [1,2-a]quinolin-8-yl]carbonyl}-4-{[3-(diméthylamino)propyl][2-(phénylsulfanyl) éthyl]amino}-3-nitrobenzènesulfonamide, chlorhydrate

On procède comme dans le Stade B de l'Exemple 2.

| *Microanalyse élémentaire :* | | | | |
|---|---|---|---|---|
| | *%C* | *%H* | *%N* | *%S* |
| *Calculé* | *64,55* | *6, 08* | *10, 04* | *7, 66* |
| *Trouvé* | *64, 67* | *5,99* | *10, 03* | *7,47* |

### Exemple 12 : N-({(4aS,R)-3-[(4'-Chloro[1,1'-biphényl]-2-yl)méthyl]-2,3,4,4a,5,6-hexahydro-1H-pyrazino[1,2-a]quinolin-8-yl}carbonyl)-4-{[3-(diméthylamino)propyl]amino}-3-nitrobenzènesulfonamide

On procède comme dans le Stade A de l'Exemple 1 en remplaçant le 4-({(1*R*)-3-(diméthylamino)-1-[(phénylsulfanyl)méthyl]propyl}amino)-3-nitrobenzènesulfonamide par le 4-{[3-(diméthylamino)propyl]amino}-3-nitrobenzènesulfonamide.

| *Microanalyse élémentaire :* | | | | |
|---|---|---|---|---|
| | *% C* | *%H* | *%N* | *%S* |
| *Calculé* | *61,96* | *5,76* | *11,72* | *4,47* |
| *Trouvé* | *62,36* | *6, 08* | *11,50* | *4,94* |

### Exemple 13 : N-({(4aS,R)-3-[(4'-Chloro[1,1'-biphényl]-2-yl)méthyl]-2,3,4,4a,5,6-hexahydro-1H-pyrazino[1,2-a]quinolin-8-yl}carbonyl)benzènesulfonamide

On procède comme dans le Stade A de l'Exemple 1 en remplaçant le 4-({(1*R*)-3-(diméthylamino)-1-[(phénylsulfanyl)méthyl]propyl}amino)-3-nitrobenzènesulfonamide par le benzènesulfonamide.

| *Microanalyse élémentaire :* | | | | |
|---|---|---|---|---|
| | *% C* | *%H* | *%N* | *%S* |
| *Calculé* | *67,18* | *5,29* | *7,34* | *5,60* |
| *Trouvé* | *66, 74* | *5,22* | *7,19* | *5,48* |

### Exemple 14 : 4-{[(2-Aminoéthyl)(2-phényléthyl)amino]méthyl}-N-({(4aS,R)-3-[(4'-chloro[1,1'-biphényl]-2-yl)méthyl]-2,3,4,4a,5,6-hexahydro-1H-pyrazino [1,2-a]quinolin-8-yl}carbonyl)benzènesulfonamide tris(trifluoroacétate)

### Stade A : 4-{[(2-Aminoéthyl)(2-phényléthyl)amino]méthyl}-N-({(4aS,R)-3-[(4'-chloro[1,1'-biphényl]-2-yl)méthyl]-2,3,4,4a,5,6-hexahydro-1H-pyrazino[1,2-a] quinolin-8-yl}carbonyl)benzènesulfonamide

On procède comme dans le Stade A de l'Exemple 1 en remplaçant le 4-({(1*R*)-3-(diméthylamino)-1-[(phénylsulfanyl)méthyl]propyl}amino)-3-nitrobenzènesulfonamide par le 4-{[(2-aminoéthyl)(2-phényléthyl)amino]méthyl}benzènesulfonamide.

### Stade B : 4-{[(2-Aminoéthyl)(2-phényléthyl)amino]méthyl}-N-({(4aS,R)-3-[(4'-chloro[1,1'-biphényl]-2-yl)méthyl]-2,3,4,4a,5,6-hexahydro-1H-pyrazino[1,2-a] quinolin-8-yl}carbonyl)benzènesulfonamide tris(trifluoroacétate)

On procède comme dans le Stade B de l'Exemple 1.

| *Microanalyse élémentaire :* | | | | |
|---|---|---|---|---|
| | *% C* | *%H* | *%N* | *%S* |
| *Calculé* | *53,97* | *4,53* | *6,42* | *2,94* |
| *Trouvé* | *53, 22* | *4,78* | *6, 20* | *2,46* |

### Exemple 15 : N-({(4aS,R)-3-[(4'-Chloro[1,1'-biphényl]-2-yl)méthyl]-2,3,4,4a,5,6-hexabydro-1H-pyrazino[1,2-a]quinolin-7-yl}carbonyl)-4-({(1R)-3-(diméthylamino)-1-[(phénylsulfanyl)méthyl]propyl}amino)-3-nitrobenzènesulfonamide

On procède comme dans le Stade A de l'Exemple 1 en remplaçant le composé obtenu dans la Préparation 1 par le composé obtenu dans la Préparation 7.

| *Microanalyse élémentaire :* | | | | |
|---|---|---|---|---|
| | *%C* | *%H* | *%N* | *%S* |
| *Calculé* | *62,95* | *5, 64* | *10, 01* | *7, 64* |
| *Trouvé* | *62, 07* | *5, 60* | *9,47* | *7,34* |

### Exemple 16 : N-{[(4aS,R)-3-([1,1'-Biphényl]-2-ylméthyl)-2,3,4,4a,5,6-hexahydro-1H-pyrazino [1,2-a]quinolin-7-yl]carbonyl}-4-({(1R)-3-(diméthylamino)-1-[(phénylsulfanyl)méthyl]propyl}amino)-3-nitrobenzènesulfonamide

On procède comme dans le Stade A de l'Exemple 1 en remplaçant le composé obtenu dans la Préparation 1 par le composé obtenu dans la Préparation 8.
*m*/*z théorique : 805,3206*
*m*/*z mesurée : 805,3207*

### Exemple 17 : N-{[2-([1,1'-Biphényl]-2-ylméthyl)-1,2,3,4-tétrahydropyrazino[1,2-a]indol-8-yl]carbonyl}-4-({(1R)-3-(diméthylamino)-1-[(phénylsulfanyl) méthyl]propyl}amino)-3-nitrobenzènesulfonamide, bis(chlorhydrate)

### Stade A : N-{[2-([1,1'-Biphényl]-2-ylméthyl)-1,2,3,4-tétrahydropyrazino[1,2-a]indol-8-yl]carbonyl}-4-({(1R)-3-(diméthylamino)-1-[(phénylsulfanyl)méthyl] propyl}amino)-3-nitrobenzènesulfonamide

On procède comme dans le Stade A de l'Exemple 1 en remplaçant le composé obtenu dans la Préparation 1 par le composé obtenu dans la Préparation 9.

### Stade B : N-{[2-([1,1'-Biphényl]-2-ylméthyl)-1,2,3,4-tétrahydropyrazino[1,2-a]indol-8-yl]carbonyl}-4-({(1R)-3-(diméthylamino)-1-[(phénylsulfanyl)méthyl] propyl}amino)-3-nitrobenzènesulfonamide, bis(chlorhydrate)

On procède comme dans le Stade B de l'Exemple 2.

### Exemple 18 : N-({2-[(4'-Chloro[1,1'-biphényl]-2-yl)méthyl]-1,2,3,4-tétrahydro pyrazino[1,2-a]indol-8-yl}carbonyl)-4-({(1R)-3-(diméthylamino)-1-[(phénylsulfanyl)méthyl]propyl}amino)-3-nitrobenzènesulfonamide, bis(chlorhydrate)

### Stade A : N-({2-[(4'-Chloro[1,1'-biphényl]-2-yl)méthyl]-1,2,3,4-tétrahydro pyrazino[1,2-a]indol-8-yl}carbonyl)-4-({(1R)-3-(diméthylamino)-1-[(phénylsulfanyl)méthyl]propyl}amino)-3-nitrobenzènesulfonamide

On procède comme dans le Stade A de l'Exemple 1 en remplaçant le composé obtenu dans la Préparation 1 par le composé obtenu dans la Préparation 10.

### Stade B : N-({2-[(4'-Chloro[1,1'-biphényl]-2-yl)méthyl]-1,2,3,4-tétrahydro pyrazino[1,2-a]indol-8-yl}carbonyl)-4-({(1R)-3-(diméthylamino)-1-[(phénylsulfanyl)méthyl]propyl}amino)-3-nitrobenzènesulfonamide, bis(chlorhydrate)

On procède comme dans le Stade B de l'Exemple 2.

### Exemple 19 : N-({(10aS,R)-2-[(4'-Chloro[1,1'-biphényl]-2-yl)méthyl]-1,2,3,4,10,10a-hexahydropyrazino[1,2-a]indol-8-yl}carbonyl)-4-({(1R)-3-(diméthylamino)-1-[(phénylsulfanyl)méthyl]propyl}amino)-3-nitrobenzènesulfonamide

On procède comme dans le Stade A de l'Exemple 1 en remplaçant le composé obtenu dans la Préparation 1 par le composé obtenu dans la Préparation 11.

| *Microanalyse élémentaire :* | | | | |
|---|---|---|---|---|
| | *% C* | *%H* | *%N* | *%S* |
| *Calculé* | *57,49* | *5,27* | *9,35* | *7,14* |
| *Trouvé* | *57,31* | *5,47* | *9,06* | *6,95* |

### Exemple 20 : N-({(4aR)-3-[(4'-Chloro[1,1'-biphényl]-2-yl)méthyl]-2,3,4,4a,5,6-hexahydro-1H-pyrazino[1,2-a]quinolin-8-yl}carbonyl)-4-({(1R)-3-(diméthylamino)-1-[(phénylsulfanyl)méthyl]propyl}amino)-3-nitrobenzènesulfonamide, bis(chlorhydrate)

### Stade A : N-({(4aR)-3-[(4'-Chloro[1,1'-biphényl]-2-yl)méthyl]-2,3,4,4a,5,6-hexahydro-1H-pyrazino[1,2-a]quinolin-8-yl}carbonyl)-4-({(1R)-3-(diméthylamino)-1-[(phénylsulfanyl)méthyl]propyl}amino)-3-nitrobenzènesulfonamide

On procède comme dans le Stade A de l'Exemple 1 en remplaçant le composé obtenu dans la Préparation 1 par le composé obtenu dans la Préparation 13.

| *Microanalyse élémentaire :* | | | | |
|---|---|---|---|---|
| | *%C* | *%H* | *%N* | *%S* |
| *Calculé* | *62,95* | *5,64* | *10,01* | *7,64* |
| *Trouvé* | *62,30* | *5,59* | *9, 66* | *7,40* |

### Stade B : N-({(4aR)-3-[(4'-Chloro[1,1'-biphényl]-2-yl)méthyl]-2,3,4,4a,5,6-hexahydro-1H-pyrazino[1,2-a]quinolin-8-yl}carbonyl)-4-({(1R)-3-(diméthylamino)-1-[(phénylsulfanyl)méthyl]propyl}amino)-3-nitrobenzènesulfonamide, bis(chlorhydrate)

On procède comme dans le Stade B de l'Exemple 2.

| *Microanalyse élémentaire :* | | | | |
|---|---|---|---|---|
| | *%C* | *%H* | *%N* | *%S* |
| *Calculé* | *57,92* | *5,41* | *9,21* | *7,03* |
| *Trouvé* | *58,44* | *5,21* | *9,19* | *6,14* |

### Exemple 21 : N-({(4aS)-3-[(4'-Chloro[1,1'-biphényl]-2-yl)méthyl]-2,3,4,4a,5,6-hexahydro-1H-pyrazino[1,2-a]quinolin-8-yl}carbonyl)-4-({(1R)-3-(diméthylamino)-1-[(phénylsulfanyl)méthyl]propyl}amino)-3-nitrobenzènesulfonamide, bis(chlorhydrate)

### Stade A : N-({(4aS)-3-[(4'-Chloro[1,1'-biphényl]-2-yl)méthyl]-2,3,4,4a,5,6-hexahydro-1H-pyrazino[1,2-a]quinolin-8-yl}carbonyl)-4-({(1R)-3-(diméthylamino)-1-[(phénylsulfanyl)méthyl]propyl}amino)-3-nitrobenzènesulfonamide

On procède comme dans le Stade A de l'Exemple 1 en remplaçant le composé obtenu dans la Préparation 1 par le composé obtenu dans la Préparation 12.

### Stade B : N-({(4aS)-3-[(4'-Chloro[1,1'-biphényl]-2-yl)méthyl]-2,3,4,4a,5,6-hexahydro-1H-pyrazino[1,2-a]quinolin-8-yl}carbonyl)-4-({(1R)-3-(diméthylamino)-1-[(phénylsulfanyl)méthyl]propyl}amino)-3-nitrobenzènesulfonamide, bis(chlorhydrate)

On procède comme dans le Stade B de l'Exemple 2.

| *Microanalyse élémentaire :* | | | | |
|---|---|---|---|---|
| | *% C* | *%H* | *%N* | *%S* |
| *Calculé* | *57,92* | *5,41* | *9,21* | *7,03* |
| *Trouvé* | *57,58* | *5,29* | *8,75* | *6,97* |

### Exemple 22 : N-{[(4aS)-3-([1,1'-Biphényl]-2-ylméthyl)-2,3,4,4a,5,6-hexahydro-1H-pyrazino[1,2-a]quinolin-8-yl]carbonyl}-4-({(1R)-3-(diméthylamino)-1-[(phénylsulfanyl)méthyl]propyl}amino)-3-nitrobenzènesulfonamide, tris (chlorhydrate)

### Stade A : N-{[(4aS)-3-([1,1'-Biphényl]-2-ylméthyl)-2,3,4,4a,5,6-hexahydro-1H-pyrazino[1,2-a]quinolin-8-yl]carbonyl}-4-({(1R)-3-(diméthylamino)-1-[(phénylsulfanyl)méthyl]propyl}amino)-3-nitrobenzènesulfonamide

On procède comme dans le Stade A de l'Exemple 1 en remplaçant le composé obtenu dans la Préparation 1 par le composé obtenu dans la Préparation 14.

### Stade B : N-{[(4aS)-3-([1,1'-Biphényl]-2-ylméthyl)-2,3,4,4a,5,6-hexahydro-1H-pyrazino[1,2-a]quinolin-8-yl]carbonyl}-4-({(1R)-3-(diméthylamino)-1-[(phénylsulfanyl)méthyl]propyl}amino)-3-nitrobenzènesulfonamide, tris (chlorhydrate)

On procède comme dans le Stade B de l'Exemple 2.

| *Microanalyse élémentaire :* | | | | |
|---|---|---|---|---|
| | *%C* | *%H* | *%N* | *%S* |
| *Calculé* | *59,21* | *5,69* | *9,42* | *7,19* |
| *Trouvé* | *58, 5* | *6,05* | *9,12* | *6,48* |

### Exemple 23 : N-{[(4aR)-3-([1,1'-Biphényl]-2-ylméthyl)-2,3,4,4a,5,6-hexahydro-1H-pyrazino[1,2-a]quinolin-8-yl]carbonyl}-4-({(1R)-3-(diméthylamino)-1-[(phénylsulfanyl)méthyl]propyl}amino)-3-nitrobenzènesulfonamide, bis(chlorhydrate)

### Stade A : N-{[(4aR)-3-([1,1'-Biphényl]-2-ylméthyl)-2,3,4,4a,5,6-hexahydro-1H-pyrazino[1,2-a]quinolin-8-yl]carbonyl}-4-({(1R)-3-(diméthylamino)-1-[(phénylsulfanyl)méthyl]propyl}amino)-3-nitrobenzènesulfonamide

On procède comme dans le Stade A de l'Exemple 1 en remplaçant le composé obtenu dans la Préparation 1 par le composé obtenu dans la Préparation 15.

### Stade B : N-{[(4aR)-3-([1,1'-Biphényl]-2-ylméthyl)-2,3,4,4a,5,6-hexahydro-1H-pyrazino[1,2-a]quinolin-8-yl]carbonyl}-4-({(1R)-3-(diméthylamino)-1-[(phénylsulfanyl)méthyl]propyl}amino)-3-nitrobenzènesulfonamide, bis (chlorhydrate)

On procède comme dans le Stade B de l'Exemple 2.

| *Microanalyse élémentaire :* | | | | |
|---|---|---|---|---|
| | *%C* | *%H* | *%N* | *%S* |
| *Calculé* | *58,73* | *5, 67* | *9,34* | *7,13* |
| *Trouvé* | *58,78* | *5,9* | *9,17* | *7, 29* |

### Exemple 24 : N-{[(4aS,R)-3-([1,1'-Biphényl]-2-ylsulfonyl)-2,3,4,4a,5,6-hexahydro-1H-pyrazino[1,2-a]quinolin-8-yl]carbonyl}-3-nitro-4-{[2-(phénylsulfanyl) éthyl]amino}benzènesulfonamide

On procède comme dans le Stade A de l'Exemple 1 en remplaçant le composé de la Préparation 1 par le composé de 1a Préparation 16, et en remplaçant le 4-({(1*R*)-3-(diméthylamino)-1-[(phénylsulfanyl)méthyl]propyl}amino)-3-nitrobenzènesulfonamide par le 3-nitro-4-{[2-(phénylsulfanyl)éthyl]amino}benzènesulfonamide.

| *Microanalyse élémentaire :* | | | | |
|---|---|---|---|---|
| | *%C* | *%H* | *%N* | *%S* |
| *Calculé* | *59, 75* | *4,76* | *8,93* | *12,27* |
| *Trouvé* | *60,02* | *4,98* | *8,41* | *11,81* |

### Exemple 25 : N-({(4aS,R)-3-[(4'-Chloro[1,1'-biphényl]-2-yl)méthyl]-2,3,4,4a,5,6-hexahydro-1H-pyrazino[1,2-a]quinolin-8-yl}carbonyl)-4-{(1R)-3-(diméthylamino)-1-[(phénylsulfanyl)méthyl]propyl}-3,4-dihydro-2H-1,2,4-benzothiadiazine-7-sulfonamide 1,1-dioxide

On procède comme dans le Stade A de l'Exemple 1 en remplaçant le 4-({(1*R*)-3-(diméthylamino)-1-[(phénylsulfanyl)méthyl]propyl}amino)-3-nitrobenzènesulfonamide par le 4-{(1*R*)-3-(diméthylamino)-1-[(phénylsulfanyl)méthyl]propyl}-3,4-dihydro-2*H*-1,2,4-benzothiadiazine-7-sulfonamide 1,1-dioxide.

### Exemple 26 : N-({(4aS,R)-3-[(4'-Chloro[1,1'-biphényl]-2-yl)méthyl]-2,3,4,4a,5,6-hexahydro-1H-pyrazino[1,2-a]quinolin-8-yl}carbonyl)-4-{(1R)-3-(diméthylamino)-1-[(phénylsulfanyl)méthyl]propyl}-4H-1,2,4-benzothiadiazine-7-sulfonamide 1,1-dioxide

On procède comme dans le Stade A de l'Exemple 1 en remplaçant le 4-({(1*R*)-3-(diméthylamino)-1-[(phénylsulfanyl)méthyl]propyl}amino)-3-nitrobenzènesulfonamide par le 4-{(1*R*)-3-(diméthylamino)-1-[(phénylsulfanyl)méthyl]propyl}-4*H*-1,2,4-benzothiadiazine-7-sulfonamide 1,1-dioxide.

### Exemple 27 : N-({3-[(4'-Chloro[1,1'-biphényl]-2-yl)méthyl]-1,2,3,4,4a,5,6,7-octahydropyrazino[1,2-a][1]benzazépin-9-yl}carbonyl)-4-({(1R)-3-(diméthylamino)-1-[(phénylsulfanyl)méthyl]propyl}amino)-3-nitrobenzènesulfonamide

On procède comme au Stade A de l'Exemple 1 en remplaçant le composé de la Préparation 1 par le composé de la Préparation 17.

### Exemple 28 : N-({(4aS)-3-[(4-Chloro[1,1'-biphényl]-2-yl)méthyl]-1,2,4,4a,5,6,7-octahydropyrazino[1,2-a][1]benzazépin-9-yl}carbonyl)-4-({(1R)-3-(diméthylamino)-1-[(phénylsulfanyl)méthyl]propyl}amino)-3-nitrobenzènesulfonamide

Le mélange d'énantiomères décrit à la Préparation 17 est séparé sur colonne. Le composé du titre est obtenu en soumettant l'énantiomère choisi au procédé du Stade A de l'Exemple 1.

### Exemple 29 : N-({(4aR)-3-[(4'-Chloro[1,1'-biphényl]-2-yl)méthyl]-1,2,3,4,4a,5,6,7-octahydropyrazino[1,2-a][1]benzazépin-9-yl}carbonyl)-4-({(1R)-3-(diméthylamino)-1-[(phénylsulfanyl)méthyl]propyl}amino)-3-nitrobenzènesulfonamide

On procède comme à l'Exemple 28 en prenant l'autre énantiomère.

### Exemple 30: N-({3-[(4'-Chloro[1,1'-biphényl]-2-yl)méthyl]-1,2,3,4,4a,5,6,7-octahydropyrazino[1,2-a][1]benzazépin-9-yl}carbonyl)-4-({(1R)-3-(4-morpholinyl)-1-[(phénylsulfanyl)méthyl]propyl}amino)-3-nitrobenzènesulfonamide

On procède comme au Stade A de l'Exemple 1 en remplaçant le composé de la Préparation 1 par le composé de la Préparation 17, et en remplaçant le 4-({(1*R*)-3-(diméthylamino)-1-[(phénylsulfanyl)méthyl]propyl}amino)-3-nitrobenzènesulfonamide par le 4-({(1*R*)-3-(4-morpholinyl)-1-[(phénylsulfanyl)méthyl]propyl}amino)-3-nitrobenzènesulfonamide.

### Exemple 31 : N-({3-[(4'-Chloro[1,1'-biphényl]-2-yl)méthyl]-1,2,3,4,4a,5,6,7-octahydropyrazino[1,2-a][1]benzazépin-9-yl}carbonyl)-4-({1R)-3-(4-morpholinyl)-1-[(phénylsulfanyl)méthyl]propyl}amino)-3-[(trifluorométhyl)sulfonyl]benzènesulfonamide

On procède comme au Stade A de l'Exemple 1 en remplaçant le composé de la Préparation 1 par le composé de la Préparation 17, et en remplaçant le 4-({(1*R*)-3-(diméthylamino)-1-[(phénylsulfanyl)méthyl]propyl}amino)-3-nitrobenzènesulfonamide par le 4-({(1*R*)-3-(4-morpholinyl)-1-[(phénylsulfanyl)méthyl]propyl}amino)-3-[(trifluorométhyl)sulfonyl] benzènesulfonamide.

### Exemple 32 : N-({(10aα)-2-[(4'-Chloro[1,1'-biphényl]-2-yl)méthyl]-1,2,3,4,10,10a-hexahydropyrazino[1,2-a]indol-8-yl}carbonyl)-4-({(1R)-3-(diméthylamino)-1-[(phénylsulfanyl)méthyl]propyl}amino)-3-nitrobenzènesulfonamide, bischlorhydrate (α=R ou S)

Le composé du titre est obtenu selon le procédé du Stade A de l'Exemple 1 en remplaçant le composé de la Préparation 1 par celui de la Préparation 18.

| *Microanalyse élémentaire :* | | | | |
|---|---|---|---|---|
| | *% C* | *%H* | *%N* | *%S* |
| *Calculé* | *57,49* | *5,27* | *9,35* | *7,14* |
| *Trouvé* | *57,72* | *4,57* | *9,40* | *7,46* |

### Exemple 33 : N-({(10aβ)-2-[(4'-Chloro[1,1'-biphényl]-2-yl)méthyl]-1,2,3,4,10,10a-hexahydropyrazino[1,2-a]indol-8-yl}carbonyl)-4-({(1R)-3-(diméthylamino)-1-[(phénylsulfanyl)méthyl]propyl}amino)-3-nitrobenzènesulfonamide, bischlorhydrate (β=S ou R)

Le composé du titre est obtenu selon le procédé du Stade A de l'Exemple 1 en remplaçant le composé de la Préparation 1 par celui de la Préparation 19.

| *Microanalyse élémentaire :* | | | | |
|---|---|---|---|---|
| | %*C* | *%H* | *%N* | *%S* |
| *Calculé* | *57,49* | *5,27* | *9,35* | *7,14* |
| *Trouvé* | *57,31* | *4, 29* | *9,48* | *7,87* |

### Exemple 34 : N-({(10aα)-2-[(4-Chloro[1,1'-biphényl]-2-yl)méthyl]-1,2,3,4,10,10a-hexahydropyrazino[1,2-a]indol-8-yl}carbonyl)-4-({(1R)-3-(4-morpholinyl)-1-[(phénylsulfanyl)méthyl]propyl}amino)-3-nitrobenzènesulfonamide, bischlorhydrate (α=R ou S)

On procède comme au Stade A de l'Exemple 1 en remplaçant le composé de la Préparation 1 par le composé de la Préparation 18, et en remplaçant le 4-({(1*R*)-3-(diméthylamino)-1-[(phénylsulfanyl)méthyl]propyl}amino)-3-nitrobenzènesulfonamide par le 4-({(1*R*)-3-(4-morpholinyl)-1-[(phénylsulfanyl)méthyl]propyl} amino)-3-nitrobenzènesulfonamide.

| *Microanalyse élémentaire :* | | | | |
|---|---|---|---|---|
| | *%C* | *%H* | *%N* | *%S* |
| *Calculé* | *57,47* | *5,25* | *8,94* | *6,82* |
| *Trouvé* | *57,56* | *5,14* | *9,16* | *6,31* |

### Exemple 35 : N-({(10aα)-2-[(4'-Chloro[1,1'-biphényl]-2-yl)méthyl]-1,2,3,4,10,10a-hexahydropyrazino[1,2-a]indol-8-yl}carbonyl)-4-({(1R)-3-(4-morpholinyl)-1-[(phénylsulfanyl)méthyl]propyl}amino)-3-[(trifluorométhyl)sulfonyl]benzènesulfonamide, bischlorhydrate (α=R ou S)

On procède comme au Stade A de l'Exemple 1 en remplaçant le composé de la Préparation 1 par le composé de la Préparation 18, et en remplaçant le 4-({(1*R*)-3-(diméthylamino)-1-[(phénylsulfanyl)méthyl]propyl}amino)-3-nitrobenzènesulfonamide par le 4-({(1*R*)-3-(4-morpholinyl)-1-[(phénylsulfanyl)méthyl]propyl}amino)-3-[(trifluorométhyl)sulfonyl] benzènesulfonamide.

| *Microanalyse élémentaire :* | | | | |
|---|---|---|---|---|
| | *%C* | *%H* | *%N* | %S |
| *Calculé* | *53,77* | *4,81* | *6,82* | *9,36* |
| *Trouvé* | *53,63* | *4,82* | *7, 28* | *8,79* |

### Exemple 36 : N-({3-[(4'-Chloro[1,1'-biphényl]-3-yl)méthyl]-5-oxo-2,3,4,4a,5,6-hexahydro-1H-pyrazino[1,2-a]quinoxalin-8-yl}carbonyl)-4-({(1R)-3-(diméthylamino)-1-[(phénylsulfanyl)méthyl]propyl}amino)-3-nitrobenzènesulfonamide

On procède comme au Stade A de l'Exemple 1 en remplaçant le composé de la Préparation 1 par le composé de la Préparation 21. S'ensuit une étape de déprotection où le résidu précédemment isolé est mis en solution dans une solution d'HCl, 4N dans le dioxane. Après neutralisation, la phase aqueuse est extraite au CH₂Cl₂ et les phases organiques sont réunies, séchées sur sulfate de magnésium, filtrées et concentrées. Le résidu obtenu est purifié par une chromatographie sur colonne de silice (CH₂Cl₂/MeOH) pour conduire au produit attendu.

### Exemple 37 : N-({3-[(4'-Chloro[1,1'-biphényl]-3-yl)méthyl]-2,3,4,4a,5,6-hexabydro-1H-pyrazino[1,2-a]quinoxalin-8-yl}carbonyl)-4-({(1R)-3-(diméthylamino)-1-[(phénylsulfanyl)méthyl]propyl}amino)-3-nitrobenzènesulfonamide

On procède comme au Stade A de l'Exemple 1 en remplaçant le composé de la Préparation 1 par le composé de la Préparation 20. S'ensuit une étape de déprotection où le résidu précédemment isolé est mis en solution dans une solution d'HCl, 4N dans le dioxane. Après neutralisation, la phase aqueuse est extraite au CH₂Cl₂ et les phases organiques sont réunies, séchées sur sulfate de magnésium, filtrées et concentrées. Le résidu obtenu est purifié par une chromatographie sur colonne de silice (CH₂Cl₂/MeOH) pour conduire au produit attendu.

### Exemple 38 : N-({3-[(4'-Chloro[1,1'-biphényl]-3-yl)méthyl]-6-méthyl-2,3,4,4a,5,6-hexahydro-1H-pyrazino[1,2-a]quinoxalin-8-yl}carbonyl)-4-({(1R)-3-(diméthylamino)-1-[(phénylsulfanyl)méthyl]propyl}amino)-3-nitrobenzènesulfonamide

On déprotège l'azote en position 6 de la partie tricyclique du composé du Stade H de la Préparation 20 à l'aide d'une solution d'HCI, 4N dans le dioxane. Après neutralisation, extraction et purification, le résidu obtenu est soumis à une réaction d'alkylation en présence d'iodure de méthyle et de K₂CO₃. Après traitement avec LiOH, on obtient l'acide 3-[(4'-chloro[1,1'-biphényl]-2-yl)méthyl]-6-méthyl-2,3,4,4a,5,6-hexahydro-1H-pyrazino[1,2-a]quinoxaline-8-carboxylique. On applique à ce dernier composé le procédé du Stade A de l'Exemple 1 et on obtient ainsi le composé du titre.

### Exemple 39 : N-[((4aR)-3-{[2-(4-Chlorophényl)-5,5-diméthyl-1-cyclohexén-1-yl] méthyl}-2,3,4,4a,5,6-hexahydro-1H-pyrazino[1,2-a]quinolin-8-yl)carbonyl]-4-({(1R)-2-(diméthylamino)-1-[(phénylsulfanyl)méthyl] éthyl}amino)-3-nitrobenzènesulfonamide, bischlorhydrate

On procède comme au Stade A de l'Exemple 1 en remplaçant le composé de la Préparation 1 par le composé de la Préparation 23.

| *Microanalyse élémentaire :* | | | | |
|---|---|---|---|---|
| | *%C* | *%H* | *%N* | *%S* |
| *Calculé* | *58,50* | *6,08* | *8,90* | *6,79* |
| *Trouvé* | *58, 06* | *5,94* | *8,84* | *6,85* |

### Exemple 40 : N-[((4aR)-3-{[2-(4-Chlorophényl)-5,5-diméthyl-1-cyclohexén-1-yl] méthyl}-2,3,4,4a,5,6-hexahydro-1H-pyrazino[1,2-a]quinolin-8-yl)carbonyl]-4-({(1R)-3-(4-morpholinyl)-1-[(phénylsulfanyl)méthyl] propyl}amino)-3-[(trifluorométhyl)sulfonyl]benzènesulfonamide, bischlorhydrate

On procède comme au Stade A de l'Exemple 1 en remplaçant le composé de la Préparation 1 par le composé de la Préparation 23, et en remplaçant le 4-({(1*R*)-3-(diméthylamino)-1-[(phénylsulfanyl)méthyl]propyl}amino)-3-nitrobenzènesulfonamide par le 4-({(1*R*)-3-(4-morpholinyl)-1-[(phénylsulfanyl)méthyl]propyl}amino)-3-[(trifluororméthyl)sulfonyl] benzènesulfonamide.

| *Microanalyse élémentaire :* | | | | |
|---|---|---|---|---|
| | *%C* | *%H* | *%N* | *%S* |
| *Calculé* | *54,82* | *5,54* | *6,52* | *8,96* |
| *Trouvé* | *54,56* | *5,13* | *6, 67* | *8,45* |

### Exemple 41 : N-[((10aα)-2-{[2-(4-Chlorophényl)-5,5-diméthyl-1-cyclohexén-1-yl]méthyl}-1,2,3,4,10,10a-hexahydropyrazino[1,2-a]indol-8-yl) carbonyl]-4-({(1R)-3-(4-morpholinyl)-1-[(phénylsulfanyl)méthyl] propyl}amino)-3-[(trifluorométhyl)sulfonyl]benzènesulfonamide, bischlorhydrate (α=R ou S)

On procède comme au Stade A de l'Exemple 1 en remplaçant le composé de la Préparation 1 par le composé de la Préparation 24, et en remplaçant le 4-({(1*R*)-3-(diméthylamino)-1-[(phénylsulfanyl)méthyl]propyl}amino)-3-nitrobenzènesulfonamide par le 4-({(1*R*)-3-(4-morpholinyl)-1-[(phénylsulfanyl)méthyl]propyl}amino)-3-[(trifluorométhyl)sulfonyl] benzènesulfonamide.

| *Microanalyse élémentaire :* | | | | |
|---|---|---|---|---|
| | *% C* | *%H* | *%N* | *%S* |
| *Calculé* | *54,41* | *5,42* | *6, 61* | *9, 08* |
| *Trouvé* | *53, 66* | *5,51* | *6,50* | *8,71* |

### Exemple 42 : N-[((4aR)-3-{[4-(4-Chlorophényl)-3-pyridyl]méthyl}-2,3,4,4a,5,6-hexahydro-1H-pyrazino[1,2-a]quinolin-8-yl)carbonyl]-4-({(1R)-2-(diméthylamino)-1-[(phénylsulfanyl)méthyl]éthyl}amino)-3-nitrobenzènesulfonamide, trichlorhydrate

On procède comme au Stade A de l'Exemple 1 en remplaçant le composé de la Préparation 1 par le composé de la Préparation 25.

| *Microanalyse élémentaire :* | | | | |
|---|---|---|---|---|
| | *% C* | *%H* | *%N* | *%S* |
| *Calculé* | *54,37* | *5,20* | *10,32* | *6,75* |
| *Trouvé* | *54,70* | *5,12* | *10, 35* | *6,71* |

### Exemple 43 : N-[((4aR)-3-{[2-(4-Chlorophényl)-3-pyridyl]méthyl}-2,3,4,4a,5,6-hexahydro-1H-pyrazino[1,2-a]quinolin-8-yl)carbonyl]-4-({(1R)-3-(diméthylamino)-1-[(phénylsulfanyl)méthyl]propyl}amino)-3-nitrobenzènesulfonamide

On procède comme au Stade A de l'Exemple 1 en remplaçant le composé de la Préparation 1 par le composé de la Préparation 26.

### Exemple 44 : N-[((4aR)-3-{[3-(4-Chlorophényl)-2-pyridyl]méthyl}-2,3,4,4a,5,6-hexahydro-1H-pyrazino[1,2-a]quinolin-8-yl)carbonyl]-4-({(1R)-3-(diméthylamino)-1-[(phénylsulfanyl)méthyl]propyl}amino)-3-nitrobenzènesulfonamide

On procède comme au Stade A de l'Exemple 1 en remplaçant le composé de la Préparation 1 par le composé de la Préparation 27.

### Exemple 45 : N-[((4aR)-3-{[3-(4-Chlorophényl)-4-pyridyl]méthyl}-2,3,4,4a,5,6-hexahydro-1H-pyrazino[1,2-a]quinolin-8-yl)carbonyl]-4-({(1R)-3-(diméthylamino)-1-[(phénylsulfanyl)méthyl]propyl}amino)-3-nitrobenzènesulfonamide

On procède comme au Stade A de l'Exemple 1 en remplaçant le composé de la Préparation 1 par le composé de la Préparation 28.

### Exemple 46 : N-({(4aR)-3-[(4-Amino-4'-chloro[1,1'-biphényl]-2-yl)méthyl]-2,3,4,4a,5,6-hexahydro-1H-pyrazino[1,2-a]quinolin-8-yl}carbonyl)-4-({(1R)-3-(diméthylamino)-1-[(phénylsulfanyl)méthyl]propyl}amino)-3-nitrobenzènesulfonamide, trichlorhydrate

On procède comme au Stade A de l'Exemple 1 en remplaçant le composé de la Préparation 1 par le composé de la Préparation 29. S'ensuit une étape de déprotection où le résidu précédemment isolé est mis en solution dans une solution d'HCl, 4N dans le dioxane. Après neutralisation, la phase aqueuse est extraite au CH₂Cl₂ et les phases organiques sont réunies, séchées sur sulfate de magnésium, filtrées et concentrées. Le résidu obtenu est purifié par une chromatographie sur colonne de silice (CH₂Cl₂/MeOH) pour conduire au produit attendu.

| *Microanalyse élémentaire :* | | | | |
|---|---|---|---|---|
| | *%C* | *%H* | *%N* | *%S* |
| *Calculé* | *54,83* | *5,33* | *10,17* | *6, 65* |
| *Trouvé* | *54,97* | *5, 25* | *10, 07* | *6, 62* |

### Exemple 47 : N-[((4aR)-3-{[4-(Aminométhyl)-4'-chloro[1,1'-biphényl]-2-yl]méthyl}-2,3,4,4a,5,6-hexahydro-1H-pyrazino[1,2-a]quinolin-8-yl)carbonyl]-4-({(1R)-3-(diméthylamino)-1-[(phénylsulfanyl)méthyl]propyl}amino)-3-nitrobenzènesulfonamide, trichlorhydrate

On procède comme au Stade A de l'Exemple 1 en remplaçant le composé de la Préparation 1 par le composé de la Préparation 30. S'ensuit une étape de déprotection où le résidu précédemment isolé est mis en solution dans une solution d'HCl, 4N dans le dioxane. Après neutralisation, la phase aqueuse est extraite au CH₂Cl₂ et les phases organiques sont réunies, séchées sur sulfate de magnésium, filtrées et concentrées. Le résidu obtenu est purifié par une chromatographie sur colonne de silice (CH₂Cl₂/MeOH) pour conduire au produit attendu.

| *Microanalyse élémentaire :* | | | | |
|---|---|---|---|---|
| | *%C* | *%H* | *%N* | *%S* |
| *Calculé* | *55,27* | *5,46* | *10,03* | *6,56* |
| *Trouvé* | *55,98* | *5,55* | *9,82* | *6,31* |

### Exemple 48 : N-[((4aR)-3-{[3'-Fluoro-4'-chloro[1,1'-biphényl]-2-yl]méthyl}-2,3,4,4a,5,6-hexahydro-1H-pyrazino[1,2-a]quinolin-8-yl)carbonyl]-4-({(1R)-3-(diméthylamino)-1-[(phénylsulfanyl)méthyl]propyl}amino)-3-nitrobenzènesulfonamide, bischlorhydrate

On procède comme au Stade A de l'Exemple 1 en remplaçant le composé de la Préparation 1 par le composé de la Préparation 31.

| *Microanalyse élémentaire :* | | | | |
|---|---|---|---|---|
| | *%C* | *%H* | *%N* | *%S* |
| *Calculé* | *56,80* | *5, 20* | *9, 03* | *6,89* |
| *Trouvé* | *56, 06* | *5, 20* | *9, 08* | *6,55* |

### Exemple 49 : N-[((4aR)-3-{[4'-Cyano[1,1'-biphényl]-2-yl]méthyl}-2,3,4,4a,5,6-hexahydro-1H-pyrazino[1,2-a]quinolin-8-yl)carbonyl]-4-({(1R)-3-(diméthylamino)-1-[(phénylsulfanyl)méthyl]propyl}amino)-3-nitrobenzènesulfonamide, bischlorhydrate

On procède comme au Stade A de l'Exemple 1 en remplaçant le composé de la Préparation 1 par le composé de la Préparation 32.

| *Microanalyse élémentaire :* | | | | |
|---|---|---|---|---|
| | *% C* | *%H* | *%N* | *%S* |
| *Calculé* | *59,86* | *5,47* | *10,86* | *7,10* |
| *Trouvé* | *59,86* | *5,19* | *10,86* | *6,72* |

### Exemple 50 : N-[((4aR)-3-{[4'-(Trifluorométhyl)[1,1'-biphényl]-2-yl]méthyl}-2,3,4,4a,5,6-hexahydro-1H-pyrazino[1,2-a]quinolin-8-yl)carbonyl]-4-({(1R)-3-(diméthylamino)-1-[(phénylsulfanyl)méthyl]propyl}amino)-3-nitrobenzènesulfonamide, bischlorhydrate

On procède comme au Stade A de l'Exemple 1 en remplaçant le composé de la Préparation 1 par le composé de la Préparation 33.

| *Microanalyse élémentaire :* | | | | |
|---|---|---|---|---|
| | *%C* | *%H* | *%N* | *%S* |
| *Calculé* | *57,14* | *5, 22* | *8,88* | *6,78* |
| *Trouvé* | *57,72* | *5,10* | *8,83* | *6,34* |

### Exemple 51 : N-[((4aS,R)-3-{[4'-(Trifluorométhyl)[1,1'-biphényl]-2-yl]méthyl}-2,3,4,4a,5,6-hexahydro-1H-pyrazino[1,2-a]quinolin-8-yl)carbonyl]-4-({(1R)-3-(diméthylamino)-1-[(phénylsulfanyl)méthyl]propyl}amino)-3-nitrobenzènesulfonamide, trifluoroacétate

On procède comme pour l'Exemple 50 en remplaçant le (4a*R*)-2,3,4,4a,5,6-hexahydro-1*H-*pyrazino[1,2-*a*]quinoline-8-carboxylate de méthyle (Stade A de la Préparation 23) par le mélange (4a*S,R*)-2,3,4,4a,5,6-hexahydro-1*H*-pyrazino[1,2-*a*]quinoline-8-carboxylate de méthyle (Stade H de la Préparation 1).

### Exemple 52 : N-({(4aR)-3-[2-(1,3-Benzodioxol-5-yl)benzyl]-2,3,4,4a,5,6-hexahydro-1H-pyrazino[1,2-a]quinolin-8-yl}carbonyl)-4-({(1R)-3-(diméthylamino)-1-[(phénylsulfanyl)méthyl]propyl}amino)-3-nitrobenzènesulfonamide, bischlorhydrate

On procède comme au Stade A de l'Exemple 1 en remplaçant le composé de la Préparation 1 par le composé de la Préparation 34.

| *Microanalyse élémentaire :* | | | | |
|---|---|---|---|---|
| | % *C* | *% H* | *% N* | *%S* |
| *Calculé* | *58, 62* | *5,47* | *9,12* | *6,96* |
| *Trouvé* | *58,76* | *5, 25* | *9,19* | *6,83* |

### Exemple 53 : N-({(4aS,R)-3-[2-(1,3-Benzodioxol-5-yl)benzyl]-2,3,4,4a,5,6-hexahydro-1H-pyrazino[1,2-a]quinolin-8-yl}carbonyl-4-{[2-(phénylsulfanyl)éthyl] amino})-3-nitrobenzènesulfonamide, trifluoroacétate

On procède comme pour l'Exemple 52 en remplaçant le (4a*R*)-2,3,4,4a,5,6-hexahydro-1*H-*pyrazino[1,2-*a*]quinoline-8-carboxylate de méthyle (Stade A de la Préparation 23) par le mélange (4a*S*,*R*)-2,3,4,4a,5,6-hexahydro-1*H*-pyrazino[1,2-*a*]quinoline-8-carboxylate de méthyle (Stade H de la Préparation 1), et en utilisant le 4-{[2-(phénylsulfanyl)éthyl]amino}3-nitrobenzènesulfonamide à la place du 4-({(1*R*)-3-(diméthylamino)-1-[(phénylsulfanyl)méthyl]propyl} amino)-3-nitrobenzènesulfonamide.

| *Microanalyse élémentaire :* | | | | |
|---|---|---|---|---|
| | *% C* | *%H* | *%N* | *%S* |
| *Calculé* | *55,64* | *4,38* | *7,4* | *6,78* |
| *Trouvé* | *55,42* | *4,37* | *7,35* | *6,87* |

### Exemple 54 : N-{[(4aR)-3-Benzhydryl-2,3,4,4a,5,6-hexahydro-1H-pyrazino[1,2-a]quinolin-8-yl]carbonyl}-4-({(1R)-3-(diméthylamino)-1-[(phénylsulfanyl)méthyl]propyl}amino)-3-nitrobenzènesulfonamide

On procède comme au Stade A de l'Exemple 1 en remplaçant le composé de la Préparation 1 par le composé de la Préparation 35.

| *Microanalyse élémentaire :* | | | | |
|---|---|---|---|---|
| | *% C* | *%H* | *%N* | *%S* |
| *Calculé* | *65,65* | *6,01* | *10,44* | *7,97* |
| *Trouvé* | *64, 63* | *5,91* | *10,11* | *7,81* |

### Exemple 55 : N-[((4aR)-3-{2-Bromobenzyl}-2,3,4a,4,6-hexahydro-1H-pyrazino[1,2-a]quinolin-8-yl)carbonyl]-4-({(1R)-3-(diméthylamino)-1-[(phényl sulfanyl)méthyl]propyl}amino)-3-nitrobenzènesulfonamide, bischlorhydrate

On applique au composé obtenu au Stade A de la Préparation 31 le procédé du Stade N de la Préparation 17. On soumet ensuite le produit ainsi obtenu au procédé du Stade A de l'Exemple 1.

| *Microanalyse élémentaire :* | | | | |
|---|---|---|---|---|
| | *%C* | *%H* | *%N* | *%S* |
| *Calculé* | *51,82* | *5,15* | *9,54* | *7,28* |
| *Trouvé* | *51,33* | *5,14* | *9,66* | *7,08* |

### Exemple 56 : N-[((4aS,R)-3-{2-Bromobenzyl}-2,3,4,4a,5,6-hexahydro-1H-pyrazino [1,2-a]quinolin-8-yl)carbonyl]-4-([2-(phénylsulfanyl)éthyl]amino)-3-nitrobenzènesulfonamide, chlorhydrate

On procède comme pour l'Exemple 55 en remplaçant le (4a*R*)-2,3,4,4a,5,6-hexahydro-1*H-*pyrazino[1,2-*a*]quinoline-8-carboxylate de méthyle (Stade A de la Préparation 23) par le mélange (4a*S*,*R*)-2,3,4,4a,5,6-hexahydro-1*H*-pyrazino[1,2-*a*]quinoline-8-carboxylate de méthyle (Stade H de la Préparation 1), et en utilisant le 4-{[2-(phénylsulfanyl)éthyl]amino}3-nitrobenzènesulfonamide à la place du 4-({(1*R*)-3-(diméthylamino)-1-[(phénylsulfanyl)méthyl]propyl}amino)-3-nitrobenzènesulfonamide.

| *Microanalyse élémentaire :* | | | | |
|---|---|---|---|---|
| | % C | *% H* | *% N* | *% S* |
| *Calculé* | *52,82* | *4,56* | *9,06* | *8,29* |
| *Trouvé* | *52,92* | *4,48* | *8,84* | *8,47* |

### Exemple 57 : N-({(4aR)-3-[(4'-Chloro[1,1'-biphényl]-2-yl)méthyl]-2,3,4,4a,5,6-hexahydro-1H-pyrazino[1,2-a]quinolin-8-yl}carbonyl)-4-({(1R)-3-(4-morpholinyl)-1-[(phénylsulfanyl)méthyl]propyl}amino)-3-nitrobenzènesulfonamide, bischlorhydrate

On procède comme au Stade A de l'Exemple 1 en remplaçant le composé de la Préparation 1 par le composé de la Préparation 13, et en remplaçant le 4-({(1*R*)-3-(diméthylamino)-1-[(phénylsulfanyl)méthyl]propyl}amino)-3-nitrobenzènesulfonamide par le 4-({(1*R*)-3-(4-morpholinyl)-1-[(phénylsulfanyl)méthyl]propyl}amino)-3-nitrobenzènesulfonamide.

| *Microanalyse élémentaire :* | | | | |
|---|---|---|---|---|
| | *% C* | *%H* | *%N* | *%S* |
| *Calculé* | *57,89* | *5,39* | *8,81* | *6,72* |
| *Trouvé* | *57,11* | *5, 06* | *8,48* | *7,46* |

### Exemple 58 : N-({(4aS,R)-3-[(4'-Chloro[1,1'-biphényl]-2-yl)méthyl]-2,3,4,4a,5,6-hexahydro-1H-pyrazino[1,2-a]quinolin-8-yl}carbonyl)-4-({(1R)-3-(4-morpholinyl)-1-[(phénylsulfanyl)méthyl]propyl}amino)-3-nitrobenzènesulfonamide, bischlorhydrate

On procède comme pour l'Exemple 57 en remplaçant le (4a*R*)-2,3,4,4a,5,6-hexahydro-1*H-*pyrazino[1,2-*a*]quinoline-8-carboxylate de méthyle (Stade A de la Préparation 23) par le mélange (4a*S,R*)-2,3,4,4a,5,6-hexahydro-1*H*-pyrazino[1,2-*a*]quinoline-8-carboxylate de méthyle (Stade H de la Préparation 1).

| *Microanalyse élémentaire :* | | | | |
|---|---|---|---|---|
| | *%C* | *%H* | *%N* | *%S* |
| *Calculé* | *57,89* | *5,39* | *8,81* | *6,72* |
| *Trouvé* | 57,38 | 5,22 | *8,80* | *6,57* |

### Exemple 59:N-({(4aR)-3-[(4'-Chloro[1,1'-biphényl]-2-yl)méthyl]-2,3,4,4a,5,6-hexahydro-1H-pyrazino[1,2-a]quinolin-8-yl}carbonyl)-4-({(1R)-3-(4-morpholinyl)-1-[(phénylsulfanyl)méthyl]propyl}amino)-3-[(trifluorométhyl)sulfonyl]benzènesulfonamide, bischlorhydrate

On procède comme au Stade A de l'Exemple 1 en remplaçant le composé de la Préparation 1 par le composé de la Préparation 13, et en remplaçant le 4-({(1*R*)-3-(diméthylamino)-1-[(phénylsulfanyl)méthyl]propyl}amino)-3-nitrobenzènesulfonamide par le 4-({(1*R*)-3-(4-morpholinyl)-1-[(phénylsulfanyl)méthyl]propyl}amino)-3-[(trifluorométhyl)sulfonyl] benzènesulfonamide.

| *Microanalyse élémentaire :* | | | | |
|---|---|---|---|---|
| | *%C* | *%H* | *%N* | *%S* |
| *Calculé* | *54,20* | *4,94* | *6,72* | *9,24* |
| *Trouvé* | *54,31* | *4,58* | *6, 75* | *8,90* |

### Exemple 60:N-({(4aS,R)-3-[(4'-Chloro[1,1'-biphényl]-2-yl)méthyl]-2,3,4,4a,5,6-hexahydro-1H-pyrazino[1,2-α]quinolin-8-yl}carbonyl)-4-({(1S)-3-(4-morpholinyl)-1-[(phénylsulfanyl)méthyllpropyl}amino)-3-nitrobenzènesulfonamide, bischlorhydrate

On procède comme pour l'Exemple 59 en remplaçant le (4a*R*)-2,3,4,4a,5,6-hexahydro-1H-pyrazino[1,2-α]quinoline-8-carboxylate de méthyle (Stade A de la Préparation 23) par le mélange (4a*S*,*R*)-2,3,4,4a,5,6-hexahydro-1H-pyrazino[1,2-*a*]quinoline-8-carboxylate de méthyle (Stade H de la Préparation 1), et en remplaçant le 4-({(1*R*)-3-(4-morpholinyl)-1-[(phénylsulfanyl)méthyl]propyl}amino)-3-[(trifluorométhyl)sulfonyl]benzènesulfonamide par le 4-({(1*S*)-3-(4-morpholinyl)-1-[(phénylsulfanyl)méthyl]propyl}amino)-3-nitrobenzènesulfonamide.

| *Microanalyse élémentaire :* | | | | |
|---|---|---|---|---|
| | *%C* | *%H* | *%N* | *%S* |
| *Calculé* | *57,89* | *5,39* | *8,81* | *6,72* |
| *Trouvé* | *57,69* | *5,32* | *9,05* | *6,54* |

### Exemple 61:N-({(4aR)-3-[(4'-Chloro[1,1'-biphényl]-2-yl)méthyl]-2,3,4,4a,5,6-hexahydro-1H-pyrazino[1,2-a]quinolin-8-yl}carbonyl)-4-({(1R)-3-(4-méthyl-1-pipérazinyl)-1-[(phénylsulfanyl)méthyl]propyl}aminor)-3-nitrobenzènesulfonamide, bischlorhydrate

On procède comme au Stade A de l'Exemple 1 en remplaçant le composé de la Préparation 1 par le composé de la Préparation 13, et en remplaçant le 4-({(1*R*)-3-(diméthylamino)-1-[(phénylsulfanyl)méthyl]propyl}amino)-3-nitrobenzènesulfonamide par le 4-({(1*R*)-3-(4-méthyl-1-pipérazinyl)-1-[(phénylsulfanyl)méthyl]propyl}amino)-3-nitrobenzènesulfonamide.

### Exemple 62:N-({(4aR)-3-[(4'-Chloro[1,1'-biphényl]-2-yl)méthyl]-2,3,4,4a,5,6-hexahydro-1H-pyrazino[1,2-a]quinolin-8-yl}carbonyl)-4-({(1R)-3-(1-pipéridyl)-1-[(phénylsulfanyl)méthyl]propyl}amino)-3-nitrobenzènesulfonamide, bischlorhydrate

On procède comme au Stade A de l'Exemple 1 en remplaçant le composé de la Préparation 1 par le composé de la Préparation 13, et en remplaçant le 4-({(1*R*)-3-(diméthylamino)-1-[(phénylsulfanyl)méthyl]propyl}amino)-3-nitrobenzènesulfonamide par le 4-({(1*R*)-3-(1-pipéridyl)-1-[(phénylsulfanyl)méthyl]propyl}amino)-3-nitrobenzènesulfonamide.

| *Microanalyse élémentaire :* | | | | |
|---|---|---|---|---|
| | *% C* | *%H* | *%N* | *%S* |
| *Calculé* | *59, 27* | *5, 61* | *8,82* | *6,73* |
| *Trouvé* | *59, 23* | *5,21* | *8,75* | *6,78* |

### Exemple 63:N-({(4aS,R)-3-[(4'-Chloro[1,1'-biphényl]-2-yl)méthyl]-2,3,4,4a,5,6-hexabydro-1H-pyrazino[1,2-a]quinolin-8-yl}carbonyl)-4-({(1R)-3-(1-pipéridyl)-1-[(phénylsulfanyl)méthyl]propyl}amino)-3-nitrobenzènesulfonamide, bischlorhydrate

On procède comme pour l'Exemple 62 en remplaçant le (4a*R*)-2,3,4,4a,5,6-hexahydro-1H-pyrazino[1,2-α]quinoline-8-carboxylate de méthyle (Stade A de la Préparation 23) par le mélange (4a*S,R*)-2,3,4,4a,5,6-hexahydro-1*H*-pyrazino[1,2-*a*]quinoline-8-carboxylate de méthyle (Stade H de la Préparation 1).

| *Microanalyse élémentaire :* | | | | |
|---|---|---|---|---|
| | *% C* | *%H* | *%N* | *%S* |
| *Calculé* | *59,27* | *5,61* | *8,82* | *6,73* |
| *Trouvé* | *58, 65* | *5,48* | *8, 56* | *6,37* |

### Exemple 64:N-({(4aS,R)-3-[(4'-Chloro[1,1'-biphényl]-2-yl)méthyl]-2,3,4,4a,5,6-hexahydro-1H-pyrazino[1,2-a]quinolin-8-yl}earbonyl)-4-({(1S)-3-(1-pipéridyl)-1-[(phénylsulfanyl)méthyl]propyl}amino)-3-nitrobenzènesulfonamide, bischlorhydrate

On procède comme pour l'Exemple 63 en remplaçant le 4-({(1*R*)-3-(1-pipéridyl)-1-[(phénylsulfanyl)méthyl]propyl}amino)-3-nitrobenzènesulfonamide par le 4-({(1*S*)-3-(1-pipéridyl)-1-[(phénylsulfanyl)méthyl]propyl}amino)-3-nitrobenzènesulfonamide.

| *Microanalyse élémentaire :* | | | | |
|---|---|---|---|---|
| | *%C* | *%H* | *%N* | *%S* |
| *Calculé* | *59, 27* | *5, 61* | *8,82* | *6,73* |
| *Trouvé* | *59, 00* | *5,39* | *8,58* | *6,70* |

### Exemple 65: N-({(4aR)-3-[(4'-Chloro[1,1'-biphényl]-2-yl)méthyl]-2,3,4,4a,5,6-hexahydro-1H-pyrazino[1,2-a]quinolin-8-yl}carbonyl)-4-({(1R)-3-(1-pyrrolidinyl)-1-[(phénylsulfanyl)méthyl]propyl}amino)-3-nitrobenzènesulfonamide, bischlorhydrate

On procède comme au Stade A de l'Exemple 1 en remplaçant le composé de la Préparation 1 par le composé de la Préparation 13, et en remplaçant le 4-({(1*R*)-3-(diméthylamino)-1-[(phénylsulfanyl)méthyl]propyl}amino)-3-nitrobenzènesulfonamide par le 4-({(1*R*)-3-(1-pyrrolidinyl)-1-[(phénylsulfanyl)méthyl]propyl}amino)-3-nitrobenzènesulfonamide.

| *Microanalyse élémentaire :* | | | | |
|---|---|---|---|---|
| | *%C* | *%H* | *%N* | *%S* |
| *Calculé* | *58,88* | *5,48* | *8,96* | *6,83* |
| *Trouvé* | *58,53* | *5,04* | *8,81* | *6,60* |

### Exemple 66:N-({(4aR)-3-[(4'-Chloro[1,1'-biphényl]-2-yl)méthyl]-2,3,4,4a,5,6-hexahydro-1H-pyrazino[1,2-a]quinolin-8-yl}carbonyl)-4-({(1R)-3-(3,6-dihydro-1(2H)-pyridyl)-1-[(phénylsulfanyl)méthyl]propyl}amino)-3-nitrobenzènesulfonamide, bischlorhydrate

On procède comme au Stade A de l'Exemple 1 en remplaçant le composé de la Préparation 1 par le composé de la Préparation 13, et en remplaçant le 4-({(1*R*)-3-(diméthylamino)-1-[(phénylsulfanyl)méthyl]propyl}amino)-3-nitrobenzènesulfonamide par le 4-({(1*R*)-3-(3,6-dihydro-1(2*H*)-pyridyl)-1-[(phénylsulfanyl)méthyl]propyl}amino)-3-nitrobenzènesulfonamide.

### Exemple 67:N-({(4aR)-3-[(4'-Chloro[1,1'-biphényl]-2-yl)méthyl]-2,3,4,4a,5,6-hexahydro-1H-pyrazino[1,2-a]quinolin-8-yl}carbonyl)-4-({(1R)-3-(1-azépanyl)-1-[(phénylsulfanyl)méthyl]propyl}amino)-3-nitrobenzènesulfonamide, bischlorhydrate

On procède comme au Stade A de l'Exemple 1 en remplaçant le composé de la Préparation 1 par le composé de la Préparation 13, et en remplaçant le 4-({(1*R*)-3-(diméthylamino)-1-[(phénylsulfanyl)méthyl]propyl}amino)-3-nitrobenzènesulfonamide par le 4-({(1*R*)-3-(1-azépanyl)-1-[(phénylsulfanyl)méthyl]propyl} amino)-3-nitrobenzènesulfonamide.

| *Microanalyse élémentaire :* | | | | |
|---|---|---|---|---|
| | *% C* | *%H* | *%N* | *%S* |
| *Calculé* | *59, 65* | *5,74* | *8,70* | *6, 64* |
| *Trouvé* | *60, 01* | *5, 60* | *8,40* | *6,39* |

### Exemple 68:N-({(4aR)-3-[(4'-Chloro[1,1'-biphényl]-2-yl)méthyl]-2,3,4,4a,5,6-hexahydro-1H-pyrazino[1,2-a]quinolin-8-yl}carbonyl)-4-({(1R)-3-((1R,5S)-3-azabicyclo[3.1.0]hex-3-yl)-1-[(phénylsulfanyl)méthyl] propyl}amino)-3-nitrobenzènesulfonamide, bischlorhydrate

On procède comme au Stade A de l'Exemple 1 en remplaçant le composé de la Préparation 1 par le composé de la Préparation 13, et en remplaçant le 4-({(1*R*)-3-(diméthylamino)-1-[(phénylsulfanyl)méthyl]propyl}amino)-3-nitrobenzènesulfonamide par le 4-({(1*R*)-3-((1*R*,5*S*)-3-azabicyclo[3.1.0]hex-3-yl)-1-[(phénylsulfanyl)méthyl]propyl} amino)-3-nitrobenzènesulfonamide.

| *Microanalyse élémentaire :* | | | | |
|---|---|---|---|---|
| | *% C* | *%H* | *%N* | *%S* |
| *Calculé* | *59,39* | *5,41* | *8,84* | *6,75* |
| *Trouvé* | *59,51* | *5,17* | *8,90* | *6,43* |

**Les exemples 69 à 78 suivants sont obtenus par couplage du composé tricyclique décrit à la Préparation 1 avec le dérivé de benzènesulfonamide approprié selon le procédé décrit au Stade A de l'Exemple 1:**

### Exemple 69:N-({(4aS,R)-3-[(4'-Chloro[1,1'-biphényl]-2-yl)méthyl]-2,3,4,4a,5,6-hexahydro-1H-pyrazino[1,2-a]quinolin-8-yl}carbonyl)-4-[(4-phénylbutyl)amino]-3-nitrobenzènesulfonamide, bistrifluoroacétate

| *Microanalyse élémentaire :* | | | | |
|---|---|---|---|---|
| | *%C* | *%H* | *%N* | *%S* |
| *Calculé* | *58, 24* | *4, 73* | *7, 58* | *3,47* |
| *Trouvé* | *58,61* | *4,87* | *7,54* | *2,97* |

### Exemple 70:N-({(4aS,R)-3-[(4'-Chloro[1,1'-biphényl]-2-yl)méthyl]-2,3,4,4a,5,6-hexahydro-1H-pyrazino[1,2-a]quinolin-8-yl}carbonyl)-4-{[2-(1-méthyl-1H-benzimidazol-2-yl)éthyl]amino}-3-nitrobenzènesulfonamide, tristrifluoroacétate

| *Microanalyse élémentaire :* | | | | |
|---|---|---|---|---|
| | *% C* | *%H* | *%N* | *%S* |
| *Calculé* | *50,91* | *3,83* | *8, 66* | *2,83* |
| *Trouvé* | *51,19* | *4, 03* | *8,76* | *2,34* |

### Exemple 71:N-({(4aS,R)-3-[(4'-Ghloro[1,1'-biphényl]-2-yl)méthyl]-2,3,4,4a,5,-hexahydro-1H-pyrazino[1,2-a]quinolin-8-yl}earbonyl)-4-{[2-(1H-benzimidazol-2-yl)éthyl]amino}-3-nitrobenzènesulfonamide, tristrifluoroacétate

| *Microanalyse élémentaire :* | | | | |
|---|---|---|---|---|
| | *%C* | *%H* | *%N* | *%S* |
| *Calculé* | *52,06* | *3,85* | *9,24* | *3,02* |
| *Trouvé* | *52,1* | *3, 9* | *9,1* | *2,38* |

### Exemple 72:N-({(4aS,R)-3-[(4'-Chloro[1,1'-biphényl]-2-yl)méthyl]-2,3,4,4a,5,6-hexahydro-1H-pyrazino[1,2-a]quinolin-8-yl}carbonyl)-4-({2-[(4-méthoxyphényl)sulfanyl]éthyl}amino)-3-nitrobenzènesulfonamide, trifluoroacétate

| *Microanalyse élémentaire :* | | | | |
|---|---|---|---|---|
| | *% C* | *%H* | *%N* | *%S* |
| *Calculé* | *54,52* | *4,31* | *7,22* | *6,62* |
| *Trouvé* | *53,91* | *4,51* | *7,15* | *6,32* |

### Exemple 73:N-({(4aS,R)-3-[(4'-Chloro[1,1'-biphényl]-2-yl)méthyl]-2,3,4,4a,5,6-hexabydro-1H-pyrazino[1,2-a]quinolin-8-yl}carbonyl)-4-{(1H-benzimidazol-2-yl)méthylamino}-3-nitrobenzènesulfonamide, bistrifluoroacétate

| *Microanalyse élémentaire :* | | | | |
|---|---|---|---|---|
| | *% C* | *%H* | *%N* | *%S* |
| *Calculé* | *52,29* | *3,77* | *9,57* | *3,13* |
| *Trouvé* | *52,66* | *3,89* | *10,13* | *2,6* |

### Exemple 74:N-({(4aS,R)-3-[(4'-Chloro[1,1'-biphényl]-2-yl)méthyl]-2,3,4,4a,5,6-hexabydro-1H-pyrazino[1,2-a]quinolin-8-yl}carbonyl)-4-({2-[(2-méthoxyphényl)sulfanyl]éthyl}amino)-3-nitrobenzènesulfonamide, chlorhydrate

| *Microanalyse élémentaire :* | | | | |
|---|---|---|---|---|
| | *% C* | *%H* | *%N* | *%S* |
| *Calculé* | *58,99* | *4,95* | *8,39* | *7, 68* |
| *Trouvé* | *59,14* | *4,92* | *8,45* | *7,45* |

### Exemple 75:N-({(4aS,R)-3-[(4'-Chloro[1,1'-biphényl]-2-yl)méthyl]-2,3,4,4a,5,6-hexahydro-1H-pyrazino[1,2-a]quinolin-8-yl}carbonyl)-4-({2-[(2-pyridyl)sulfanyl]éthyl}amino)-3-nitrobenzènesulfonamide, chlorhydrate

| *Microanalyse élémentaire :* | | | | |
|---|---|---|---|---|
| | *%C* | *%H* | *%N* | *%S* |
| *Calculé* | *58,13* | *4,75* | *10,43* | *7,96* |
| *Trouvé* | *57, 62* | *4, 23* | *9,96* | *7,55* |

### Exemple 76:N-({(4aS,R)-3-[(4'-Chloro[1,1'-biphényl]-2-yl)méthyl]-2,3,4,4a,5,6-hexahydro-1H-pyrazino[1,2-a]quinolin-8-yl}carbonyl)-4-{[2-(2-nitroanilino)éthyl]amino}-3-nitrobenzènesulfonamide, chlorhydrate

| *Microanalyse élémentaire :* | | | | |
|---|---|---|---|---|
| | %C | *%H* | *%N* | *%S* |
| *Calculé* | *57,69* | *4,72* | *11,77* | *3,85* |
| *Trouvé* | 58,14 | *4,54* | *11,81* | *4,01* |

### Exemple 77:N-({(4aS,R)-3-[(4'-Chloro[1,1'-biphényl]-2-yl)méthyl]-2,3,4,4a,5,6-hexahydro-1H-pyrazino[1,2-a]quinolin-8-yl}carbonyl)-4-[(2-{[2-(hydroxyméthyl)phényl]sulfanyl}éthyl)amino]-3-nitrobenzènesulfonamide, chlorhydrate

| *Microanalyse élémentaire :* | | | | |
|---|---|---|---|---|
| | *%C* | *%H* | *%N* | *%S* |
| *Calculé* | *58,99* | *4,95* | *8,39* | *7,68* |
| *Trouvé* | *59, 00* | *4,64* | *8,22* | *7,27* |

### Exemple 78:N-({(4aS,R)-3-[(4'-Chloro[1,1'-biphényl]-2-yl)méthyl]-2,3,4,4a,5,6-hexahydro-1H-pyrazino[1,2-a]quinolin-8-yl}carbonyl)-4-({(1S,R)-3-(diméthylamino)-1-[(phénylsulfanyl)méthyl]propyl}amino)-3-nitrobenzènesulfonamide

| *Microanalyse élémentaire :* | | | | |
|---|---|---|---|---|
| | *%C* | *%H* | *%N* | *%S* |
| *Calculé* | *62,95* | *5,64* | *10,01* | *7,64* |
| *Trouvé* | *63, 3 7* | *5, 50* | *10,00* | *7, 76* |

**Les exemples 79 à 90 suivants sont obtenus par couplage du composé tricyclique décrit à la Préparation 10 avec le dérivé de benzènesulfonamide approprié selon le procédé décrit au Stade A de l'Exemple 1:**

### Exemple 79:N-({2-[(4'-Chloro[1,1'-biphényl]-2-yl)méthyl]-1,2,3,4-tétrahydropyrazino[1,2-a]indol-8-yl}carbonyl)-4-({3-(4-méthyl-1-pipérazinyl)-1-[(phénylsulfanyl)méthyl]propyl}amino)-3-nitrobenzènesulfonamide, tristrifluoroacétate

| *Microanalyse élémentaire :* | | | | |
|---|---|---|---|---|
| | *%C* | *%H* | *%N* | *%S* |
| *Calculé* | *50,35* | *4,12* | *7,81* | *5,11* |
| *Trouvé* | *50, 26* | *4, 22* | *7,91* | *4,72* |

### Exemple 80: N-({2-[(4'-Chloro[1,1'-biphényl]-2-yl)méthyl]-1,2,3,4-tétrahydropyrazino[1,2-a]indol-8-yl}carbonyl)-4-{[3-(diméthylamino)-1-(2-phényléthyl)propyl]amino}-3-nitrobenzènesulfonamide, bischlorhydrate

| *Microanalyse élémentaire :* | | | | |
|---|---|---|---|---|
| | *%C* | *%H* | *%N* | *%S* |
| *Calculé* | *60,17* | *5,39* | *9,57* | *3, 65* |
| *Trouvé* | *59,55* | *5,6* | *9, 64* | *2,58* |

### Exemple 81:N-({2-[(4'-Chloro[1,1'-biphényl]2-yl)méthyl]-1,2,3,4-tetrahydro pyrazino[1,2-a]indol-8-yl}carbonyl)-4-({3-((3aR,6aS)-hexahydrocyclo-penta[c]pyrrol-2(1H)-yl)-1-[(phénylsulfanyl)méthyl]propyl}amino)-3-nitrobenzènesulfonamide, bischlorhydrate

| *Microanalyse élémentaire :* | | | | |
|---|---|---|---|---|
| | *% C* | *%H* | *%N* | *%S* |
| *Calculé* | *59,9* | *5,34* | *8,73* | *6, 66* |
| *Trouvé* | *59, 66* | *5,47* | *8,46* | *6, 3* |

### Exemple 82:N-({2-[(4'-Chloro[1,1'-biphényl]-2-yl)méthyl]-1,2,3,4-tetrahydro pyrazino[1,2-a]indol-8-yl}carbanyl)-4-{[1-[(phénylsulfanyl)méthyl]-3-(1-pipéridyl)propyl]amino}-3-nitrobenzènesulfonamide, bischlorhydrate

| *Microanalyse élémentaire :* | | | | |
|---|---|---|---|---|
| | *%C* | *%H* | *%N* | *%S* |
| *Calculé* | *59, 00* | *5, 27* | *8,97* | *6,85* |
| *Trouvé* | *58,95* | *5,4* | *8, 68* | *6,59* |

### Exemple 83:N-({2-[(4'-Chloro[1,1'-biphényl]-2-yl)méthyl]-1,2,3,4-tétrahydropyrazino [1,2-a]indol-8-yl}carbonyl)-4-{[1-[(phénylsulfanyl)méthyl]-3-(1-pyrrolidinyl)propyl]amino}-3-nitrobenzènesulfonamide, bischlorhydrate

| *Microanalyse élémentaire :* | | | | |
|---|---|---|---|---|
| | *%C* | *%H* | *%N* | *%S* |
| *Calculé* | *59,06* | *5,15* | *9,18* | *7,01* |
| *Trouvé* | *59,31* | *5,21* | *9,08* | *7,43* |

### Exemple 84:N-({2-[(4'-Chloro[1,1'-biphényl]-2-yl)méthyl]-1,2,3,4-tétrahydropyrazino[1,2-a] indol-8-yl}carbonyl)-4-{[3-(diméthylamino)-1-(phénoxyméthyl)propyl]amino}-3-nitrobenzènesulfonamide, bischlorhydrate

| *Microanalyse élémentaire :* | | | | |
|---|---|---|---|---|
| | *%C* | *%H* | *%N* | *%S* |
| *Calculé* | *58, 67* | *5,15* | *9,55* | *3, 64* |
| *Trouvé* | *58, 65* | *4,99* | *9,44* | *3,31* |

### Exemple 85:N-({2-[(4'-Chloro[1,1'-biphényl]-2-yl)méthyl]-1,2,3,4-tétrahydropyrazino[1,2-a]indol-8-yl}carbonyl)-4-({3-(4-morpholinyl)-1-[(phénylsulfanyl)méthyl]propyl}amino)-3-nitrobenzènesulfonamide, bischlorhydrate

| *Microanalyse élémentaire :* | | | | |
|---|---|---|---|---|
| | *%C* | *%H* | *%N* | *%S* |
| *Calculé* | *57,6* | *5,05* | *8,96* | *6,83* |
| *Trouvé* | *58,45* | *5, 03* | *8,78* | *6, 61* |

### Exemple 86:N-({2-[(4'-Chloro[1,1'-biphényl]-2-yl)méthyl]-1,2,3,4-tétrahydropyrazino [1,2-a]indol-8-yl}carbonyl)-4-{[1-(anilinométhyl)-3-(diméthylamino) propyl]amino}-3-nitrobenzènesutfonamide, bistrifluoroacétate

On peut noter que l'azote du groupement anilinométhyl du dérivé de benzènesulfonamide est protégé par une fonction Boc au moment du couplage sur le tricycle. L'étape de déprotection qui conduit au produit du titre est réalisée en présence d'HCl, 6N et de dioxane.

| *Microanalyse élémentaire :* | | | | |
|---|---|---|---|---|
| | *% C* | *%H* | *%N* | *%S* |
| *Calculé* | *51,86* | *4,19* | *8,71* | *2,85* |
| *Trouvé* | *52,17* | *4,5* | *9,55* | *2, 29* |

### Exemple 87:N({2-[(4'-Chloro[1,1'-biphényl]-2-yl)méthyl]-1,2,3,4-tétrahydropyrazino[1,2-a]indol-8-yl}carbonyl)-4-({3-(diméthylamino)-1-[2-(2-furyl)éthyl]propyl}amino)-3-nitrobenzènesulfonamide, chlorhydrate

| *Microanalyse élémentaire :* | | | | |
|---|---|---|---|---|
| | *%C* | *%H* | *%N* | *%S* |
| *Calculé* | *58,10* | *5,22* | *9,68* | *3, 69* |
| *Trouvé* | *57,85* | *4,90* | *9,66* | *3, 70* |

### Exemple 88:N-({2-[(4'-Chloro[1,1'-biphényl]-2-yl)méthyl]-1,2,3,4-tétrahydropyrazino[1,2-a]indol-8-yl}carbonyl)-4-{[1-[(phénylsulfanyl)méthyl]-3-(4-thiomorpholinyl)propyl]amino}-3-nitrobenzènesulfonamide, bischlorhydrate

| *Microanalyse élémentaire :* | | | | |
|---|---|---|---|---|
| | *%C* | *%H* | *%N* | *%S* |
| *Calculé* | *56,63* | *4,96* | *8,81* | *10,08* |
| *Trouvé* | *56,44* | *4,71* | *8,82* | *10,10* |

### Exemple 89:N-({2-[(4'-Chloro[1,1'-biphényl]-2-yl)méthyl]-1,2,3,4-tétrahydropyrazino[1,2-a]indol-8-yl}carbonyl)-4-({3-[méthoxy(méthyl) amino]-1-[(phénylsulfanyl)méthyl]propyl}amino)-3-nitrobenzènesulfonamide, chlorhydrate

### Exemple 90:N-({12-[(4'-Chloro[1,1'-biphényl]-2-yl)méthyl]-1,2,3,4-tétrahydropyrazino[1,2-a]indol-8-yl}carbonyl)-4-({3-(4,4-difluoro-1-pipéridyl)-1-[(phénylsulfanyl)méthyl]propyl}amino)-3-nitrobenzènesulfonamide, bischlorhydrate

| *Microanalyse élémentaire :* | | | | |
|---|---|---|---|---|
| | *% C* | *%H* | *%N* | *%S* |
| *Calculé* | *56,82* | *4,87* | *8, 64* | *6, 60* |
| *Trouvé* | *56,88* | *4, 65* | *8, 64* | *6,49* |

### Exemple 91: N-[((4aS,R)-3-{[4-(4-Chlorophényl)-3-pyridyl]méthyl}-2,3,4,4a,5,6-hexahydro-1H-pyrazino[1,2-a]quinolin-8-yl)carbonyl]-4-{[2-(phénylsulfanyl)éthyl]amino}-3-nitrobenzènesulfonamide, chlorhydrate

On procède comme au Stade A de l'Exemple 1 en remplaçant le composé de la Préparation 1 par le composé de la Préparation 36, et en remplaçant le 4-({(1*R*)-3-(diméthylamino)-1-[(phénylsulfanyl)méthyl]propyl}amino)-3-nitrobenzènesulfonamide par le 4-({2-(phénylsulfanyl)éthyl}amino)-3-nitrobenzènesulfonamide.

| *Microanalyse élémentaire :* | | | | |
|---|---|---|---|---|
| | *%C* | *%H* | *%N* | *%S* |
| *Calculé* | *58,13* | *4, 75* | *10,43* | *7,96* |
| *Trouvé* | *57,15* | *4,36* | *10,20* | *7,46* |

### Exemple 92:N-[((4aS,R)-3-{[2-(4-Chlorophényl)-3-pyridyl]méthyl}-2,3,4,4a,5,6-hexahydro-1H-pyrazino[1,2-a]quinolin-8-yl)carbonyl]-4-{[2-(phénylsulfanyl)éthyl]amino}-3-nitrobenzènesulfonamide, bistrifluoroacétate

On procède comme au Stade A de l'Exemple 1 en remplaçant le composé de la Préparation 1 par le composé de la Préparation 37, et en remplaçant le 4-({(1*R*)-3-(diméthylamino)-1-[(phénylsulfanyl)méthyl]propyl}amino)-3-nitrobenzènesulfonamide par le 4-({2-(phénylsulfanyl)éthyl} amino)-3-nitrobenzènesulfonamide.

| *Microanalyse élémentaire :* | | | | |
|---|---|---|---|---|
| | *% C* | *%H* | *%N* | *%S* |
| *Calculé* | *51,78* | *3,94* | *8,43* | *6,43* |
| *Trouvé* | *52,38* | *4,12* | *8,47* | *6,68* |

### Exemple 93:N-[((4aS,R)-3-{[2-(4-Chlorophényl)-3-pyridyl]méthyl}-2,3,4,4a,5,6-hexahydro-1H-pyrazino[1,2-a]quinolin-8-yl)carbonyl]-4-({(1R)-3-(diméthylamino)-1-[(phénylsulfanyl)méthyl]propyl}amino)-3-nitrobenzènesulfonamide, trichlorhydrate

On procède comme au Stade A de l'Exemple 1 en remplaçant le composé de la Préparation 1 par le composé de la Préparation 37.

| *Microanalyse élémentaire :* | | | | |
|---|---|---|---|---|
| | *%C* | *%H* | *%N* | *%S* |
| *Calculé* | *54,37* | *5,20* | *10,32* | *6,75* |
| *Trouvé* | *53, 74* | *5,23* | *10,15* | *5,97* |

### Exemple 94:N-[((4aS,R)-3-{[3-(4-Chlorophényl)-2-pyridyl]méthyl}-2,3,4,4a,5,6-hexahydro-1H-pyrazino[1,2-a]quinolin-8-yl)carbonyl]-3-nitro-4-{[2-(phénylsulfanyl)éthyl]amino}benzènesulfonamide, chlorhydrate

On procède comme au Stade A de l'Exemple 1 en remplaçant le composé de la Préparation 1 par le composé de la Préparation 38, et en remplaçant le 4-({(1*R*)-3-(diméthylamino)-1-[(phénylsulfanyl)méthyl]propyl}amino)-3-nitrobenzènesulfonamide par le 4-({2-(phénylsulfanyl)éthyl}amino)-3-nitrobenzènesulfonamide.

| *Microanalyse élémentaire :* | | | | |
|---|---|---|---|---|
| | *%C* | *%H* | *%N* | *%S* |
| *Calculé* | *58,13* | *4,75* | *10,43* | *7,96* |
| *Trouvé* | *57,50* | *4,46* | *10, 20* | *7,94* |

### Exemple 95:N-[((4aS,R)-3-{[3-(4-Chlorophényl)-4-pyridyl]méthyl}-2,3,4,4a,5,6-hexahydro-1H-pyrazino[1,2-a]quinolin-8-yl)carbonyl]-4-{[2-(phénylsulfanyl)éthyl]amino}-3-nitrobenzènesulfonamide, bischlorhydrate

On procède comme au Stade A de l'Exemple 1 en remplaçant le composé de la Préparation 1 par le composé de la Préparation 39, et en remplaçant le 4-({(1*R*)-3-(diméthylamino)-1-[(phénylsulfanyl)méthyl]propyl}amino)-3-nitrobenzènesulfonamide par le 4-({2-(phénylsulfanyl)éthyl} amino)-3-nitrobenzènesulfonamide.

| *Microanalyse élémentaire :* | | | | |
|---|---|---|---|---|
| | *% C* | *%H* | *%N* | *%S* |
| *Calculé* | *55, 62* | *4, 67* | *9,98* | *7,71* |
| *Trouvé* | *56,49* | *4,50* | *10,06* | *7,32* |

### Exemple 96:N-[((4aS,R)-3-[(2-(4-Pyridyl)-3-pyridyl)méthyl]-2,3,4,4a,5,6-hexahydro-1H-pyrazino[1,2-a]quinolin-8-yl)carbonyl]-4-({(1R)-3-(diméthylamino)-1-[(phénylsulfanyl)méthyl]propyl}amino)-3-nitrobenzènesulfonamide, trischlorhydrate

On procède comme au Stade A de l'Exemple 1 en remplaçant le composé de la Préparation 1 par le composé de la Préparation 40.

| *Microanalyse élémentaire :* | | | | |
|---|---|---|---|---|
| | *%C* | *%H* | *%N* | *%S* |
| *Calculé* | *53,98* | *5,34* | *11,99* | *6,86* |
| *Trouvé* | *53,34* | *5,48* | *12,68* | *5,78* |

### Exemple 97:N-[((4aS,R)-3-[(2-(6-Chloropyridin-3-yl)-3-pyridyl)méthyl]-2,3,4,4a,5,6-hexahydro-1H-pyrazino[1,2-a]quinoline-8-carbonyl]-4-({(1R)-3-(diméthylamino)-1-[(phénylsulfanyl)méthyl]propyl}amino)-3-nitrobenzènesulfonamide, trischlorhydrate

On procède comme au Stade A de l'Exemple 1 en remplaçant le composé de la Préparation 1 par le composé de la Préparation 41.

| *Microanalyse élémentaire :* | | | | |
|---|---|---|---|---|
| | *%C* | *%H* | *%N* | *%S* |
| *Calculé* | *53,05* | *5,09* | *11,78* | *6, 74* |
| *Trouvé* | *52,69* | *5,25* | *11,59* | *5,99* |

### Exemple 98:N-[((4aS,R)-3-[(2-(6-Hydroxypyridin-3-yl)-3-pyridyl)méthyl]-2,3,4,4a,5,6-hexahydro-1H-pyrazino[1,2-a]quinoline-8-carbonyl]-4-({(1R)-3-(diméthylamino)-1-[(phénylsulfanyl)méthyl]propyl}amino)-3-nitrobenzènesulfonamide, bischlorhydrate

On procède comme au Stade A de l'Exemple 1 en remplaçant le composé de la Préparation 1 par le composé de la Préparation 42.

### Exemple 99:N-[((4aS,R)-2-{[2-(4-Chlorophényl)-3-pyridyl]méthyl}-1,2,3,4-tétrahydropyrazino[1,2-a]indole-8-carbonyl]-4-({(1R)-3-(diméthylamino)-1-[(phénylsulfanyl)méthyl]propyl}amino)-3-nitrobenzènesulfonamide, bischlorhydrate

On procède comme au Stade A de l'Exemple 1 en remplaçant le composé de la Préparation 1 par le composé de la Préparation 43.

| *Microanalyse élémentaire :* | | | | |
|---|---|---|---|---|
| | *%C* | *%H* | *%N* | *%S* |
| *Calculé* | *55,32* | *4,91* | *10,75* | *7, 03* |
| *Trouvé* | *54,79* | *4,85* | *10,47* | *6,71* |

### Exemple 100: N-[((4aS,R)-2-{[2-(6-Chloropyridin-3-yl)-3-pyridyl]méthyl}-1,2,3,4-tétrahydrapyrazino[1,2-a]indole-8-carbonyl]-4-({(1R)-3-(diméthylamino)-1-[(phénylsulfanyl)méthyl]propyl}amino)-3-nitrobenzènesulfonamide, bischlorhydrate

On procède comme au Stade A de l'Exemple 1 en remplaçant le composé de la Préparation 1 par le composé de la Préparation 44.

### Exemple 101: N-[((4aS,R)-3-{[4'-Cyano[1,1'-biphényl]-2-yl]méthyl}-2,3,4,4a,5,6-hexahydro-1H-pyrazino[1,2-a]quinolin-8-yl)carbonyl]-4-{[2-(phénylsulfanyl)éthyl]amino}-3-nitrobenzènesulfonamide, trifluoroacétate

On procède comme pour l'Exemple 49 en remplaçant le (4a*R*)-2,3,4,4a,5,6-hexahydro-1*H-*pyrazino[1,2-*a*]quinoline-8-carboxylate de méthyle (Stade A de la Préparation 23) par le mélange (4a*S*,*R*)-2,3,4,4a,5,6-hexahydro-1*H*-pyrazino[1,2-*a*]quinoline-8-carboxylate de méthyle (Stade H de la Préparation 1) et en remplaçant le 4-({(1*R*)-3-(diméthylamino)-1-[(phénylsulfanyl)méthyl]propyl}amino)-3-nitrobenzène par le 4-{[2-(phénylsulfanyl) éthyl]amino}-3-nitrobenzènesulfonamide.

### Exemple 102 : N-[((4aS,R)-3-{[4-(Trifluorométhyl)[1,1'-biphényl]-2-yl]méthyl}-2,3,4,4a,5,6-hexahydro-1H-pyrazino[1,2-α]quinolin-8-yl)carbonyl]-4-{[2-(phényl sulfanyl)éthyl]amino}-3-nitrobenzènesulfonamide, trifluoroacétate

On procède comme pour l'Exemple 50 en remplaçant le (4a*R*)-2,3,4,4a,5,6-hexahydro-1*H-*pyrazino[1,2-*a*]quinoline-8-carboxylate de méthyle (Stade A de la Préparation 23) par le mélange (4a*S*,*R*)-2,3,4,4a,5,6-hexahydro-1*H*-pyrazino[1,2-*a*]quinoline-8-carboxylate de méthyle (Stade H de la Préparation 1) et en remplaçant le 4-({(1*R*)-3-(diméthylamino)-1-[(phénylsulfanyl)méthyl]propyl}amino)-3-nitrobenzène par le 4-{[2-(phénylsulfanyl) éthyl]amino}-3-nitrobenzènesulfonamide.

| *Microanalyse élémentaire :* | | | | |
|---|---|---|---|---|
| | *%C* | *%H* | *%N* | *%S* |
| *Calculé* | *54,91* | *4,15* | *7,33* | *6,71* |
| *Trouvé* | *55,12* | *4,13* | *7, 02* | *6, 63* |

### Exemple 103 : N-[((4aS,R)-3-{[3'-(Trifluorométhyl)[1,1'-biphényl]-2-yl]méthyl}-2,3,4,4a,5,6-hexahydro-1H-pyrazino[1,2-a]quinolin-8-yl)carbonyl]-4-{[2-(phényl sulfanyl)éthyl]amino}-3-nitrobenzènesulfonamide, trifluoroacétate

On procède comme pour l'Exemple 102 en remplaçant au cours de la synthèse l'acide 4-trifluorométhyl boronique par l'acide 3- trifluorométhylphényl boronique.

### Exempte 104 : N-[((4aS,R)-3-{[4'-(tert-Butyl)-[1,1'-biphényl]-2-yl]méthyl}-2,3,4,4a,5,6-hexahydro-1H-pyrazino[1,2-a]quinolin-8-yl)carbonyl]-4-{[2-(phényl sulfanyl)éthyl]amino}-3-nitrobenzènesulfonamide, trifluoroacétate

On applique au composé de la Préparation 45 le procédé du Stade A de l'Exemple 1 en remplaçant le 4-({(1*R*)-3-(diméthylamino)-1-[(phénylsulfanyl)méthyl]propyl}amino)-3-nitrobenzène par le 4-{[2-(phénylsulfanyl)éthyl]amino}-3-nitrobenzènesulfonamide.

| *Microanalyse élémentaire :* | | | | |
|---|---|---|---|---|
| | *%C* | *%H* | *%N* | *%S* |
| *Calculé* | *61,12* | *5,35* | *7,75* | *7,09* |
| *Trouvé* | *61,17* | *5,17* | *7, 85* | *6,84* |

### Exemple 105 : N-[((4aS,R)-3-{[3',5'-Diméthyl-[1,1'-biphényl]-2-yl]méthyl}-2,3,4,4a,5,6-hexahydro-1H-pyrazino[1,2-a]quinolin-8-yl)carbonyl]-4-{[2-(phénylsulfanyl)éthyl] amino}-3-nitrobenzènesulfonamide, trifluoroacétate

On procède comme à l'Exemple 104 en remplaçant dans la Préparation 45 l'acide *4-tert-*butylphényl boronique par l'acide 3,5-diméthylphényl boronique.

| *Microanalyse élémentaire :* | | | | |
|---|---|---|---|---|
| | *%C* | *%H* | *%N* | *%S* |
| *Calculé* | *57,41* | *4,89* | *7,49* | *6,86* |
| *Trouvé* | *57,46* | *4,87* | *7,32* | *6,77* |

### Exemple 106 : N-[((4aS,R)-3-{[2',4'-Diméthoxy-[1,1'-biphényl]-2-yl]méthyl}-2,3,4,4a,5,6-hexabydro-1H-pyrazino[1,2-a]quinolin-8-yl)carbonyl]-4-{[2-(phényl sulfanyl)éthyl]amino}-3-nitrobenzènesulfonamide, trifluoroacétate

On procède comme à l'Exemple 104 en remplaçant dans la Préparation 45 l'acide *4-tert-*butylphényl boronique par l'acide 2,4-diméthoxyphényl boronique.

| *Microanalyse élémentaire :* | | | | |
|---|---|---|---|---|
| | *%C* | *%H* | *%N* | *%S* |
| *Calculé* | *55,43* | *4,72* | *7,23* | *6,62* |
| *Trouvé* | *55, 5* | *4,74* | *7,13* | *6,21* |

### Exemple 107 : N-[((4aS,R)-3-{[3',4'-Diméthoxy-[1,1'-biphényl]-2-yl]méthyl}-2,3,4,4a,5,6-hexahydro-1H-pyrazino[1,2-a]quinolin-8-yl)carbonyl]-4-{[2-(phényl sulfanyl)éthyl]amino}-3-nitrobenzènesulfonamide, trifluoroacétate

On procède comme à l'Exemple 104 en remplaçant dans la Préparation 45 l'acide *4-tert-*butylphényl boronique par l'acide 3,4-diméthoxyphényl boronique.

| *Microanalyse élémentaire :* | | | | |
|---|---|---|---|---|
| | *%C* | *%H* | *%N* | *%S* |
| *Calculé* | *58,20* | *4,88* | *7,71* | *7,06* |
| *Trouvé* | *57, 25* | *4,89* | *7, 68* | *7, 66* |

### Exemple 108 : N-[((4aS,R)-3-{[2',3'-Diméthoxy-[1,1'-biphényl]-2-yl]méthyl}-2,3,4,4a,5,6-hexahydro-1H-pyrazino[1,2-a]quinolin-8-yl)carbonyl]-4-{[2-(phényl sulfanyl)éthyl]amino}-3-nitrobenzènesulfonamide, trifluoroacétate

On procède comme à l'Exemple 104 en remplaçant dans la Préparation 45 l'acide *4-tert-*butylphényl boronique par l'acide 2,3-diméthoxyphényl boronique.

### Exemple 109 : N-[((4aS,R)-3-{[4'-Fluoro-[1,1'-biphényl]-2-yl]méthyl}-2,3,4,4a,5,6-hexahydro-1H-pyrazino[1,2-a]quinolin-8-yl)carbonyl]-4-{[2-(phényl sulfanyl)éthyl]amino}-3-nitrobenzènesulfonamide, chlorhydrate

On procède comme à l'Exemple 104 en remplaçant dans la Préparation 45 l'acide *4-tert-*butylphényl boronique par l'acide 4-fluorophényl boronique.

| *Microanalyse élémentaire :* | | | | |
|---|---|---|---|---|
| | *% C* | *%H* | *%N* | *%S* |
| *Calculé* | *60,94* | *4,99* | *8,88* | *8,13* |
| *Trouvé* | *61,42* | *5,18* | *8,5* | *7,48* |

### Exemple 110 : N-[((4aS,R)-3-{[3'-Fluoro-4'-chloro-[1,1'-biphényl]-2-yl]méthyl}-2,3,4,4a,5,6-hexabydro-1H-pyrazino[1,2-a]quinolin-8-yl)carbonyl]-4-{[2-(phényl sulfanyl)éthyl]amino}-3-nitrobenzènesulfonamide, trifluoroacétate

On procède comme à l'Exemple 104 en remplaçant dans la Préparation 45 l'acide 4-*tert-*butylphényl boronique par l'acide 3-fluoro-4-chlorophényl boronique.

### Exemple 111 : N-[((4aS,R)-3-{[3',4'-Dichloro-[1,1'-biphényl]-2-yl]méthyl}-2,3,4,4a,5,6-hexahydro-1H-pyrazino[1,2-a]quinolin-8-yl)carbonyl]-4-{[2-(phénylsulfanyl)éthyl] amino}-3-nitrobenzènesulfonamide, trifluoroacétate

On procède comme à l'Exemple 104 en remplaçant dans la Préparation 45 l'acide *4-tert-*butylphényl boronique par l'acide 3,4-dichlorophényl boronique.

### Exemple 112 : N-[((4aS,R)-3-{[4'-Méthyl-[1,1'-biphényl]-2-yl]méthyl}-2,3,4,4a,5,b-hexahydro-1H-pyrazino[1,2-a]quinolin-8-yl)carbonyl]-4-{[2-(phényl sulfanyl)éthyl]amino}-3-nitrobenzènesulfonamide, trifluoroacétate

On procède comme à l'Exemple 104 en remplaçant dans la Préparation 45 l'acide 4-*tert-*butylphényl boronique par l'acide 4-méthylphényl boronique.

| *Microanalyse élémentaire :* | | | | |
|---|---|---|---|---|
| | *% C* | *%H* | *%N* | *%S* |
| *Calculé* | *59,92* | *4,91* | *8,13* | *7,44* |
| *Trouvé* | *58,77* | *4,92* | *8, 07* | *6,78* |

### Exemple 113 : N-[((4aS,R)-3-{[3'-Chloro-[1,1'-biphényl]-2-yl]méthyl}-2,3,4,4a,5,6-hexahydro-1H-pyrazino[1,2-a]quinolin-8-yl)carbonyl]-4-{[2-(phényl sulfanyl)éthyl]amino}-3-nitrobenzènesulfonamide, trifluoroacétate

On procède comme à l'Exemple 104 en remplaçant dans la Préparation 45 l'acide *4-tert-*butylphényl boronique par l'acide 3-chlorophényl boronique.

| *Microanalyse élémentaire :* | | | | |
|---|---|---|---|---|
| | *%C* | *%H* | *%N* | *%S* |
| *Calculé* | *57,17* | *4,45* | *7,94* | *7,27* |
| *Trouvé* | *56,37* | *4,50* | *7,81* | *6,67* |

### Exemple 114 : N-[((4aS,R)-3-{[3'-Fluoro-[1,1'-biphényl]-2-yl]méthyl}-2,3,4,4a,5,6-hexahydro-1H-pyrazino[1,2-a]quinolin-8-yl)carbonyl]-4-{[2-(phényl sulfanyl)éthyl]amino}-3-nitrobenzènesulfonamide, bistrifluoroacétate

On procède comme à l'Exemple 104 en remplaçant dans la Préparation 45 l'acide *4-tert-*butylphényl boronique par l'acide 3-fluorophényl boronique.

### Exemple 115 : N-[((4aS,R)-3-{[3',4'-Difluoro-[1,1'-biphényl]-2-yl]méthyl}-2,3,4,4a,5,6-hexahydro-1H-pyrazino[1,2-a]quinolin-8-yl)carbonyl]-4-{[2-(phényl sulfanyl)éthyl]amino}-3-nitrobenzènesulfonamide, trifluoroacétate

On procède comme à l'Exemple 104 en remplaçant dans la Préparation 45 l'acide *4-tert-*butylphényl boronique par l'acide 3,4-difluorophényl boronique.

| *Microanalyse élémentaire :* | | | | |
|---|---|---|---|---|
| | *%C* | *%H* | *%N* | *%S* |
| *Calculé* | *53* | *4,1* | *7,12* | *6,52* |
| *Trouvé* | *53, 21* | *4, 29* | *6,97* | *6, 29* |

### Exemple 116 : N-[(4aS,R)-3-{[3'-Chloro-4'-fluoro-[1,1'-biphényl]-2-yl]méthyl}-2,3,4,4a,5,6-hexahydro-1H-pyrazino[1,2-a]quinolin-8-yl)carbonyl]4-{[2-(phényl sulfanyl)éthyl]amino}-3-nitrobenzènesulfonamide, trifluoroacétate

On procède comme à l'Exemple 104 en remplaçant dans la Préparation 45 l'acide *4-tert-*butylphényl boronique par l'acide 3-chloro-4-fluorophényl boronique.

| *Microanalyse élémentaire :* | | | | |
|---|---|---|---|---|
| | *%C* | *%H* | *%N* | *%S* |
| *Calculé* | *53,37* | *4,00* | *7,19* | *6,58* |
| *Trouvé* | *53,7* | *4,09* | *7,1* | *6,16* |

### Exemple 117 : N-((4aS,R)-{3-[2-(2,2-Difluoro-1,3-benzodioxol-4-yl)benzyl]-2,3,4,4a,5,6-hexahydro-1H-pyrazino[1,2-a]quinolin-8-yl}carbonyl)-3-nitro-4-{[2-(phénylsulfanyl) éthyl]amino}benzènesulfonamide, trifluoroacétate

On procède comme à l'Exemple 104 en remplaçant dans la Préparation 45 l'acide *4-tert-*butylphényl boronique par l'acide 2,2-difluoro-1,3-benzodioxol-4-yl boronique.

| *Microanalyse élémentaire :* | | | | |
|---|---|---|---|---|
| | *%C* | *%H* | *%N* | *%S* |
| *Calculé* | *55, 66* | *4,13* | *7,55* | *6,91* |
| *Trouvé* | *54,95* | *3,92* | *7,51* | *7,44* |

### Exemple 118 : N-((4aS,R)-{3-[2-(2,3-Dihydro-1,4-benzodioxin-6-yl)benzyl]-2,3,4,4a,5,6-hexahydro-1H-pyrazino[1,2-a]quinolin-8-yl}carbonyl)-3-nitro-4-{[2-(phénylsulfanyl)éthyl]amino}benzènesulfonamide, trifluoroacétate

On procède comme à l'Exemple 104 en remplaçant dans la Préparation 45 l'acide *4-tert-*butylphényl boronique par l'acide 2,3-dihydro-1,4-benzodioxin-6-yl boronique.

### Exemple 119 : N-((4aS,R)-{3-[2-(1-Naphtyl)benzyl]-2,3,4,4a,5,6-hexahydro-1H-pyrazino[1,2-a]quinolin-8-yl}carbonyl)-3-nitro-4-{[2-(phénylsulfanyl)éthyl] amino}benzènesulfonamide, trifluoroacétate

On procède comme à l'Exemple 104 en remplaçant dans la Préparation 45 l'acide 4*-tert-*butylphényl boronique par l'acide 1-naphtyl boronique.

| *Microanalyse élémentaire :* | | | | |
|---|---|---|---|---|
| | *% C* | *%H* | *%N* | *%S* |
| *Calculé* | *59, 07* | *4, 62* | *8,41* | *6,78* |
| *Trouvé* | *58,92* | *4,71* | *7, 2* | *6,34* |

### Exemple 120 : N-((4aS,R)-{3-[2-(2-Naphtyl)benzyl]-2,3,4,4a,5,6-hexahydro-1H-pyrazino[1,2-a]quinolin-8-yl}carbonyl)-3-nitro-4-{[2-(phénylsulfanyl)éthyl]amino} benzènesulfonamide, trifluoroacétate

On procède comme à l'Exemple 104 en remplaçant dans la Préparation 45 l'acide *4-tert-*butylphényl boronique par l'acide 2-naphtyl boronique.

| *Microanalyse élémentaire :* | | | | |
|---|---|---|---|---|
| | *% C* | *%H* | *%N* | *%S* |
| *Calculé* | *59,42* | *4, 65* | *7,48* | *6,85* |
| *Trouvé* | *59,57* | *4,76* | *7,23* | *6,83* |

### Exemple 121 : N¹-((4aS,R)-{3-[(4'-Chloro[1,1'-biphényl]-2-yl)méthyl]-2,3,4,4a,5,6-hexahydro-1H-pyrazino[1,2-a]quinolin-8-yl}carbonyl)-4-[(2-phénoxyéthyl)amino]-1,3-benzènedisulfonamide, trifluoroacétate

On procède comme au Stade A de l'Exemple 1 en remplaçant le 4-({(1*R*)-3-(diméthylamino)-1-[(phénylsulfanyl)méthyl]propyl}amino)-3-nitrobenzènesulfonamide par le 4-[(2-phénoxyéthyl)amino]-1,3-benzènedisulfonamide.

| *Microanalyse élémentaire :* | | | | |
|---|---|---|---|---|
| | *%C* | *%H* | *%N* | *%S* |
| *Calculé* | *53,56* | *4,33* | *7,23* | *6,62* |
| *Trouvé* | *53,95* | *4,38* | *7,21* | *6,28* |

### Exemple 122 : N-((4aS,R)-{3-[(4'-Chloro[1,1'-biphényl]-2-yl)méthyl]-2,3,4,4a,5,6-hexahydro-1H-pyrazino[1,2-a]quinolin-8-yl}carbonyl)-3-nitro-4-{[3-(2-oxo-1-azépanyl)propyl]amino}benzènesulfonamide, bistrifluoroacétate

On procède comme au Stade A de l'Exemple 1 en remplaçant le 4-({(1*R*)-3-(diméthylamino)-1-[(phénylsulfanyl)méthyl]propyl}amino)-3-nitrobenzènesulfonamide par le 3-nitro-4-{[3-(2-oxo-1-azépanyl)propyl]amino}benzènesulfonamide.

### Exemple 123 : N-{(4aS,R)-[3-([1,1'-Biphényl]-2-ylméthyl)-2,3,4,4a,5,6-hexabydro-1H-pyrazino[1,2-a]quinolin-8-yl]carbonyl}-6-chloro-2-[2-(phénylsulfanyl)éthyl]-3,4-dihydro-2H-1,2,4-benzothiadiazine-7-sulfonamide 1,1-dioxide, trifluoroacétate

On procède comme au Stade A de l'Exemple 4 en remplaçant le 4-({(1*R*)-3-(diméthylamino)-1-[(phénylsulfanyl)méthyl]propyl}amino)-3-nitrobenzènesulfonamide par le 6-chloro-2-[2-(phénylsulfanyl)éthyl]-3,4-dihydro-2*H*-1,2,4-benzothiadiazine-7-sulfonamide 1,1-dioxide.

| *Microanalyse élémentaire :* | | | | |
|---|---|---|---|---|
| | *% C* | *%H* | *%N* | *%S* |
| *Calculé* | *53,82* | *4, 26* | *7,15* | *9,82* |
| *Trouvé* | *53,52* | *4, 20* | *7,10* | *9,86* |

### Exemple 124 : N-((4aS,R)-{3-[(4'-Chloro[1,1'-biphényl]-2-yl)méthyl]-2,3,4,4a,5,6-hexahydro-1H-pyrazino[1,2-a]quinolin-8-yl}carbonyl)-4-(2-phénoxyéthyl)-4H-1,2,4-benzothiadiazine-7-sulfonamide 1,1-dioxide, trifluoroacétate

On procède comme au Stade A de l'Exemple 1 en remplaçant le 4-({(1*R*)-3-(diméthylamino)-1-[(phénylsulfanyl)méthyl]propyl}amino)-3-nitrobenzènesulfonamide par le 4-(2-phénoxyéthyl)-4*H*-1,2,4-benzothiadiazine-7-sulfonamide 1,1-dioxide.

| *Microanalyse élémentaire :* | | | | |
|---|---|---|---|---|
| | *% C* | *%H* | *%N* | *%S* |
| *Calculé* | *55,86* | *4,23* | *7,50* | *6,87* |
| *Trouvé* | *55,01* | *4,30* | *7, 23* | *6,76* |

### Exemple 125 : N-{(4aS,R)-[3-(2-Benzylbenzyl)-2,3,4,4a,5,6-hexahydro-1H-pyrazino [1,2-a]quinolin-8-yl]carbonyl}-4-({(1R)-3-(diméthylamino)-1-[(phénylsulfanyl)méthyl] propyl}amino)-3-nitrobenzènesulfonamide, chlorhydrate

On procède comme au Stade A de l'Exemple 5 en remplaçant le 3-nitro-4-{[2-(phénylsulfanyl)éthyl]amino}benzènesulfonamide par le 4-({(1*R*)-3-(diméthylamino)-1-[(phénylsulfanyl)méthyl]propyl}amino)-3-nitrobenzènesulfonamide.

| *Microanalyse élémentaire :* | | | | |
|---|---|---|---|---|
| | *% C* | *%H* | *%N* | *%S* |
| *Calculé* | *60,59* | *5,88* | *9,42* | *7,19* |
| *Trouvé* | *60,50* | *5,88* | *9,43* | *6,59* |

### Exemple 126 : N-((4aS,R)-{3-[2-(4-Chlorobenzyl)benzyl]-2,3,4,4a,5,6-hexahydro-1H-pyrazino[1,2-a]quinolin-8-yl}carbonyl)-3-nitro-4-{[2-(phénylsulfanyl)éthyl]amino} benzènesulfonamide, chlorhydrate

On procède comme au Stade A de l'Exemple 5 en remplaçant le composé de la Préparation 5 par le composé de la Préparation 46.

| *Microanalyse élémentaire :* | | | | |
|---|---|---|---|---|
| | *% C* | *%H* | *%N* | *%S* |
| *Calculé* | *60,14* | *5,05* | *8,55* | *7,83* |
| *Trouvé* | *59,47* | *4,85* | *8,39* | *7,55* |

### Exemple 127 : N-((4aS,R)-{3-[2-phénoxybenzyl]-2,3,4,4a,5,6-hexahydro-1H-pyrazino[1,2-a]quinolin-8-yl}carbonyl)-3-nitro-4-{[2-(phénylsulfanyl)éthyl]amino} benzènesulfonamide, chlorhydrate

On procède comme au Stade A de l'Exemple 1 en remplaçant le composé de la Préparation 1 par le composé de la Préparation 47, et en remplaçant le 4-({(1*R*)-3-(diméthylamino)-1-[(phénylsulfanyl)méthyl]propyl}amino)-3-nitrobenzènesulfonamide par le 3-nitro-4-{[2-(phénylsulfanyl)éthyl]amino}benzènesulfonamide.

| *Microanalyse élémentaire :* | | | | |
|---|---|---|---|---|
| | *%C* | *%H* | *%N* | *%S* |
| *Calculé* | *61,10* | *5,13* | *8,91* | *8,16* |
| *Trouvé* | *61,46* | *4,96* | *8, 92* | *7,83* |

### Exemple 128 : N-((4aS,R)-{3-[2-phénoxybenzyl]-2,3,4,4a,5,6-hexahydro-1H-pyrazino[1,2-a]quinolin-8-yl}carbonyl)-3-nitro-4-({(1R)-3-(diméthylamino)-1-[(phénylsulfanyl)méthyl]propyl}amino)benzènesulfonamide, bischlorhydrate

On procède comme au Stade A de l'Exemple 1 en remplaçant le composé de la Préparation 1 par le composé de la Préparation 47.

| *Microanalyse élémentaire :* | | | | |
|---|---|---|---|---|
| | *% C* | *%H* | *%N* | *%S* |
| *Calculé* | *59,12* | *5,64* | *9,40* | *7,17* |
| *Trouvé* | *59,30* | *5,34* | *9,34* | *7, 23* |

### Exemple 129 : N-({(4aR)-3-[(4'-Chloro[1,1'-biphényl]-2-yl)méthyl]-2,3,4,4a,5,6-hexahydro-1H-pyrazino[1,2-a]quinolin-8-yl}carbonyl)-4-{[(3R)-3-amino-4-(phénylsulfanyl)butyl]amino}-3-nitrobenzènesulfanamide, tristrifluoroacétate

On procède comme à l'Exemple 20 en remplaçant le 4-({(1*R*)-3-(diméthylamino)-1-[(phénylsulfanyl)méthyl]propyl}amino)-3-nitrobenzènesulfonamide par le 4-{[(3*R*)-3-amino-4-(phénylsulfanyl)butyl]amino}-3-nitrobenzènesulfonamide.

| *Microanalyse élémentaire :* | | | | |
|---|---|---|---|---|
| | *%C* | *%H* | *%N* | *%S* |
| *Calculé* | *49,98* | *4,02* | *7,29* | *5,56* |
| *Trouvé* | *48,89* | *3,99* | *6,92* | *5, 24* |

### Exemple 130 : N-({(4aR)-3-[(4'-Chloro[1,1'-biphényl]-2-yl)méthyl]-2,3,4,4a,5,6-hexahydro-1H-pyrazino[1,2-a]quinolin-8-yl}carbonyl)-4-({(1R)-3-(diméthylamino)-1-[(phénylsulfanyl)méthyl]propyl}amino)-3-nitrobenzènesulfonamide, sodium

Ce composé est obtenu à partir d'une solution du sel de bis(chlorhydrate) décrit à l'Exemple 20 en le soumettant à trois équivalents de NaOH.

### ETUDE PHARMACOLOGIQUE

### EXEMPLE A : Induction de l'activité caspase in vitro.

Cette étude a porté sur trois lignées cellulaires tumorales humaines :
- 1 carcinome du poumon à petites cellules, H146,
- 1 leucémie myéloïde aiguë, MV4;11,
- 1 leucémie, RS4;11.
Ces lignées cellulaires sont cultivées dans un incubateur à 37°C en présence de 5% de CO₂. Les cellules H146 et RS4;11 sont cultivées dans du milieu RPMI 1640 complet contenant 10% de sérum de veau foetal, 2 mM de glutamine, 50 unités/ml de pénicilline, 50 µg/ml de streptomycine et 10 mM de tampon Hepes, pH = 7,4.
Les cellules MV4;11 sont cultivées dans un milieu similaire supplémenté en GM-CSF à 5 ng/ml.
Les cellules sont réparties dans des plaques 6 puits et exposées aux composés à tester pendant 6 heures. Elles sont ensuite collectées et lysées et l'activité caspase est mesurée dans les lysats cellulaires.
Cette mesure enzymatique est réalisée en dosant l'apparition d'un produit de clivage fluorigénique (Pharmacia).

Les résultats montrent que les composés de l'invention sont de puissants inducteurs d'apoptose, évaluée en mesurant l'activité caspase 3, dans les trois lignées tumorales testées.
A titre d'exemple, le composé de l'Exemple 20 montre une activité à 3µM de 22000 UI dans les cellules H146, une activité à 0,1µM de 20000 UI dans les cellules MV4 ;11, et une activité à 1µM de 23000 UI dans les cellules RS4 ;11.

### EXEMPLE B : Cytotoxicité in vitro.

Les études de cytotoxicité ont été réalisées sur les trois lignées tumorales présentées dans l'Exemple A.
Les cellules sont réparties dans des microplaques et exposées aux composés à tester pendant 48 heures. La viabilité cellulaire est ensuite quantifiée par un essai colorimétrique, le Microculture Tetrazolium Assay (Cancer Res., 1987, 47, 939-942).

Les résultats sont exprimés en IC₅₀ (concentration en composé qui inhibe à 50 % la viabilité cellulaire), et montrent que les composés de l'invention sont cytotoxiques.
A titre d'Exemple, le composé de l'Exemple 20 présente une IC₅₀ de 3,05.10⁻⁷M sur H146, 2,95.10⁻⁸M sur MV4 ;11 et de 1,65.10⁻⁸M sur RS4 ;11.

### EXEMPLE C : Induction de l'activité caspase in vivo.

La capacité des composés de l'invention à activer la caspase 3 est évaluée dans un modèle de xénogreffe de cellules de carcinome pulmonaire à petites cellules H146.
5.10⁶ cellules H146 sont greffées par voie sous-cutanée dans des souris immunodéprimées (souche NOD SCID). 25 à 30 jours après la greffe, les composés à tester sont injectés par voie intra-péritonéale dans un mélange tween80/eau. Seize heures après le traitement, les masses tumorales sont récupérées, lysées et l'activité caspase 3 est mesurée dans les lysats tumoraux.

Les résultats obtenus montrent que les composés de l'invention sont capables d'induire l'apoptose dans les cellules tumorales H146 *in vivo.*
A titre d'Exemple, une activation supérieure à 700% par rapport au contrôle est obtenue pour le composé de l'Exemple 20 et le composé de l'Exemple 23.

### EXEMPLE D : Activité anti-tumorale in vivo.

L'activité anti-tumorale des composés de l'invention est évaluée dans un modèle de xénogreffe de cellules de carcinome pulmonaire à petites cellules H146.
5.10⁶ cellules H146 sont greffées par voie sous-cutanée dans des souris immunodéprimées (souche NOD SCID). 25 à 30 jours après la greffe, lorsque la masse tumorale a atteint environ 150 mm³, les composés à tester sont injectés par voie intra-péritonéale (dans un mélange tween80/eau) tous les jours pendant 21 jours. La masse tumorale est mesurée 2 fois par semaine depuis le début du traitement.

Les résultats obtenus montrent que les composés de l'invention sont capables d'induire une régression tumorale durant la période de traitement.
A titre d'Exemple, le composé de l'Exemple 20 administré à la dose de 100 mg/kg induit une régression tumorale quasi complète durant la période de traitement, avec un effet persistant au moins 40 jours après la fin du traitement.

### EXEMPLE E : Toxicité plaquettaire.

Du sang de souris BDF1 est prélevé sur tube citraté, dilué dans du PBS et incubé en présence de différentes concentrations des produits à tester. Après 4 heures d'incubation à 37°C, 20µl de billes fluorescentes (1036 billes / µl) sont ajoutés dans chaque échantillon. L'analyse morphologique par cytométrie en flux permet d'identifier les plaquettes, et le comptage de 200 billes fluorescentes permet de quantifier le nombre absolu de plaquettes par µl de sang analysé. En parallèle, un marquage à l'annexin-V-FITC suivi d'une analyse par cytométrie permettent de déterminer le pourcentage de plaquettes en apoptose.

Les composés de l'invention présentent une toxicité plaquettaire acceptable pour un développement dans l'indication cancer.

### EXEMPLE F : Composition pharmaceutique : Comprimés

| | |
|---|---|
| 1000 comprimés dosés à 5 mg de *N*-({(4a*R*)-3-[(4'-chloro[1,1'-biphényl]-2-yl)méthyl]-2,3,4,4a,5,6-hexahydro-1*H*-pyrazino[1,2-*a*]quinolin-8-yl}carbonyl)-4-({(1*R*)-3-(diméthylamino)-1-[(phénylsulfanyl)méthyl]propyl}amino)-3-nitrobenzènesulfonamide, bis(chlorhydratc) (Exemple 20) | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I) : 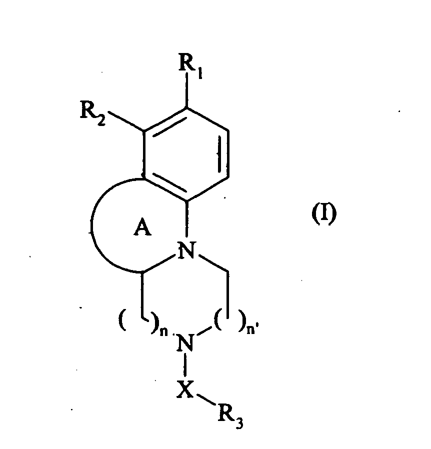 dans laquelle :
◆ A représente un cycle aromatique ou non contenant 5, 6 ou 7 chaînons, et pouvant contenir 1 ou 2 hétéroatomes choisis parmi oxygène, soufre et azote, ce dernier pouvant être substitué par un groupement alkyle (C₁-C₆) linéaire ou ramifié, étant entendu que le cycle A ainsi défini ne peut contenir 2 atomes de soufre ni 2 atomes d'oxygène et que l'un des chaînons peut représenter un groupement C=O,
◆ n et n', identiques ou différents, représentent 0, 1 ou 2, avec 0 < n +n' < 4,
◆ R₃ représente un groupement aryle ou hétéroaryle,
◆ X représente une chaîne alkylène linéaire ou ramifiée contenant de 1 à 6 atomes de carbone, dont un ou deux des atomes de carbone peut(vent) être remplacé(s) par un atome d'oxygène, un groupement cycloalkylène, un groupement arylène, un groupement hétéroarylène ou un groupement SO₂,
◆ un des groupements R₁ ou R₂ représente un atome d'hydrogène et l'autre représente un groupement de formule (II) : 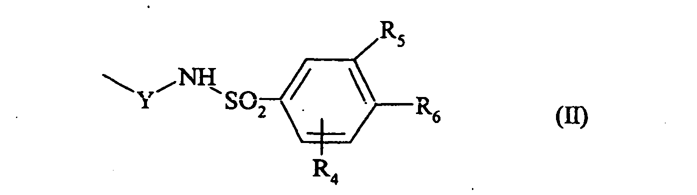
dans laquelle :
- Y représente un groupement C=O ou CH₂,
- R₅ représente un atome d'hydrogène et dans ce cas R₆ représente un atome d'hydrogène ou un groupement -NR₇R'₇ ou -CH₂-NR₇R'₇ dans lesquels R₇ et R'₇, identiques ou différents, représentent chacun indépendamment un atome d'hydrogène ou un groupement alkyle (C₁-C₆) linéaire ou ramifié substitué par un ou plusieurs groupements aryle, hétéroaryle, aryloxy, hétéroaryloxy, arylthio, hétéroarylthio, hétérocycloalkyle, ou -NR₁₀R'₁₀ dans lequel :
* R₁₀ et R'₁₀, identiques ou différents, sont choisis parmi hydrogène, alkyle (C₁-C₆) linéaire ou ramifié, alkoxy (C₁-C₆) linéaire ou ramifié, aryle et hétéroaryle,
* ou bien R₁₀ et R'₁₀ forment un groupement cyclique ou bicyclique, saturé ou non, éventuellement substitué par un hétéroatome choisi parmi oxygène, azote ou soufre, étant entendu que un ou plusieurs des chaînons peut (peuvent) représenter un groupement C=O ou peut (peuvent) être substitué(s) comme le prévoit la définition de l'hétérocycle ci-dessous,
ou R₅ et R₆ forment avec les deux atomes de carbone qui les portent, un cycle aromatique ou non contenant 5 ou 6 chaînons dont un atome d'azote en para du groupement SO₂, et pouvant contenir en plus de l'atome d'azote un autre atome d'azote et/ou un groupement SO₂, le cycle ainsi défini étant substitué par un groupement R₇ tel que défini précédemment,
- R₄ représente un atome d'halogène ou un groupement NO₂, R₈, SO₂-R₉, alkyle (C₁-C₆) linéaire ou ramifié, ou alkoxy (C₁-C₆) linéaire ou ramifié, où R₈ peut prendre toutes les valeurs de R₇ tel que défini précédemment,
- R₉ représente un groupement amino ou un groupement alkyle (C₁-C₆) linéaire ou ramifié éventuellement substitué par un ou plusieurs atomes d'halogène,
étant entendu que :
- par "aryle", on entend un groupement phényle, naphtyle ou biphényle,
- par "hétéroaryle", on entend tout groupement mono ou bi-cyclique, possédant au moins une partie aromatique, et contenant 5 à 10 chaînons et pouvant contenir 1 à 3 hétéroatomes choisis parmi oxygène, soufre ou azote comme les groupements furane, thiophène, pyrrole, imidazoline, pyridine, quinoléine, isoquinoléine, chromane, indole, benzothiophène, benzofurane, 1,3-benzodioxole et 2,3-dihydro-1,4-benzodioxine,
- par "hétérocycloalkyle", on entend tout groupement non aromatique mono ou bi-cyclique contenant 4 à 10 chaînons et pouvant contenir 1 à 3 hétéroatomes choisis parmi oxygène, soufre ou azote,
- par "cycloalkyle", on entend tout groupement non aromatique mono ou bi-cyclique contenant 4 à 10 chaînons,
les groupements aryle, hétéroaryle, hétérocycloalkyle et cycloalkyle ainsi définis pouvant être substitués par 1 à 3 groupements choisis parmi alkyle (C₁-C₆) linéaire ou ramifié éventuellement substitué par un groupement hydroxy ou amino, alkoxy (C₁-C₆) linéaire ou ramifié, hydroxy, carboxy, formyle, nitro, cyano, amino, polyhalogénoalkyle (C₁-C₆) linéaire ou ramifié, alkyloxycarbonyle, ou atomes d'halogène,
- par "arylène", "hétéroarylène"et "cycloalkylène", on entend un groupement aryle, hétéroaryle et cycloalkyle respectivement tels que définis précédemment, insérés à la place d'un atome de carbone de la chaîne alkylène,
leurs énantiomères et diastéréoisomères, ainsi que leurs sels d'addition à un acide ou à une base pharmaceutiquement acceptable.

2. Composés de formule (I) selon la revendication 1 pour lesquels Y représente un groupement C=O, leurs énantiomères et diastéréoisomères ainsi que leurs sels d'addition à un acide ou à une base pharmaceutiquement acceptable.

3. Composés de formule (I) selon la revendication 1 pour lesquels n et n' représentent 1, leurs énantiomères et diastéréoisomères ainsi que leurs sels d'addition à un acide ou à une base pharmaceutiquement acceptable.

4. Composés de formule (I) selon la revendication 1 pour lesquels R₄ représente un groupement NO₂ ou SO₂-CF₃, leurs énantiomères et diastéréoisomères ainsi que leurs sels d'addition à un acide ou à une base pharmaceutiquement acceptable.

5. Composés de formule (I) selon la revendication 1 pour lesquels X-R₃ représente un groupement ([1,1'-biphényl]-2-yl)méthyl éventuellement substitué par un ou plusieurs groupements choisis parmi halogène, cyano, amino, aminométhyl, trifluorométhyl, leurs énantiomères et diastéréoisomères ainsi que leurs sels d'addition à un acide ou à une base pharmaceutiquement acceptable.

6. Composés de formule (I) selon la revendication 1 pour lesquels R₅ représente un atome d'hydrogène, leurs énantiomères et diastéréoisomères ainsi que leurs sels d'addition à un acide ou à une base pharmaceutiquement acceptable.

7. Composés de formule (I) selon la revendication 1 pour lesquels R₇ représente le groupement 1-(*N,N*-diméthylamino)-4-(phénylsulfanyl)-butan-3-yle, leurs énantiomères et diastéréoisomères ainsi que leurs sels d'addition à un acide ou à une base pharmaceutiquement acceptable.

8. Composés de formule (I) selon la revendication 1 pour lesquels R₇ représente un groupement 1-(NR₁₀R'₁₀)-4-(phénylsulfanyl)-butan-3-yle, R₁₀ et R'₁₀ étant tels qu'ils forment un groupement cyclique ou bicyclique, saturé ou non, éventuellement substitué par un hétéroatome choisi parmi oxygène, azote ou soufre, leurs énantiomères et diastéréoisomères ainsi que leurs sels d'addition à un acide ou à une base pharmaceutiquement acceptable.

9. Composés de formule (I) selon la revendication 1 pour lesquels R'₇ représente l'atome d'hydrogène, leurs énantiomères et diastéréoisomères ainsi que leurs sels d'addition à un acide ou à une base pharmaceutiquement acceptable.

10. Composés de formule (I) qui sont :
• le *N*-({(4a*R*)-3-[(4'-chloro[1,1'-biphényl]-2-yl)méthyl]-2,3,4,4a,5,6-hexahydro-1*H-*pyrazino[1,2-*a*]quinolin-8-yl}carbonyl)-4-({(1*R*)-3-(diméthylamino)-1-[(phénlsulfanyl) méthyl]propyl}amino)-3-nitrobenzènesulfonamide,
• le *N*-({(10aα)-2-[(4'-Chloro[1,1'-biphényl]-2-yl)méthyl]-1,2,3,4,10,10a-hexahydro pyrazino[1,2-*a*]indol-8-yl}carbonyl)-4-({(1*R*)-3-(diméthylamino)-1-[(phénylsulfanyl)méthyl]propyl}amino)-3-nitrobenzènesulfonamide,
• le *N*-({(10aβ)-2-[(4'-Chloro[1,1'-biphényl]-2-yl)méthyl]-1,2,3,4,10,10a-hexahydropyrazino[1,2-*a*]indol-8-yl}carbonyl)-4-({(1*R*)-3-(diméthylamino)-1-[(phénylsulfanyl)méthyl]propyl}amino)-3-nitrobenzènesulfonamide,
• le *N*-({(10aα)-2-[(4'-chloro[1,1'-biphényl]-2-yl)méthyl]-1,2,3,4,10,10a-hexahydropyrazino[1,2-*a*]indol-8-yl}carbonyl)-4-({(1*R*)-3-(4-morpholinyl)-1-[(phénylsulfanyl)méthyl]propyl}amino)-3-nitrobenzènesulfonamide,
• le *N*-({(10aα)-2-[(4'-chloro[1,1'-biphényl]-2-yl)méthyl]-1,2,3,4,10,10a-hexahydropyrazino[1,2-*a*]indol-8-yl}carbonyl)-4-({(1*R*)-3-(4-morpholinyl)-1-[(phénylsulfanyl)méthyl]propyl}amino)-3-[(trifluorométhyl)sulfonyl]benzène sulfonamide,
• le *N*-[((4a*R*)-3-{[2-(4-chlorophényl)-5,5-diméthyl-1-cyclohexén-1-yl]méthyl}-2,3,4,4a,5,6-hexahydro-1*H*-pyrazino[1,2-*a*]quinolin-8-yl)carbonyl]-4-({(1*R*)-3-(4-morpholinyl)-1-[(phénylsulfanyl)méthyl]propyl}amino)-3-[(trifluorométhyl) sulfonyl]benzènesulfonamide,
• le *N*-[((10aβ)-2-{[2-(4-chlorophényl)-5,5-diméthyl-1-cyclohexén-1-yl]méthyl}-1,2,3,4,10,10a-hexahydropyrazino[1,2-*a*]indol-8-yl)carbonyl]-4-({(1*R*)-3-(4-morpholinyl)-1-[(phénylsulfanyl)méthyl]propyl}amino)-3-[(trifluorométhyl) sulfonyl]benzènesulfonamide,
• le *N*-[((4a*R*)-3-{[4-(4-chlorophényl)-3-pyridyl]méthyl}-2,3,4,4a,5,6-hexahydro-1*H-*pyrazino[1,2-*a*]quinolin-8-yl)carbonyl]-4-({(1*R*)-2-(diméthylamino)-1-[(phénylsulfanyl)méthyl]éthyl}amino)-3-nitrobenzènesulfonamide,
• le N-({(4a*R*)-3-[(4-amino-4'-chloro[1,1'-biphényl]-2-yl)méthyl]-2,3,4,4a,5,6-hexahydro-1*H*-pyrazino[1,2-*a*]quinolin-8-yl}carbonyl)-4-({(1*R*)-3-(diméthylamino)-1-[(phénylsulfanyl)méthyl]propyl}amino)-3-nitrobenzènesulfonamide,
• le *N*-[((4a*R*)-3-{[4-(aminométhyl)-4'-chloro[1,1'-biphényl]-2-yl]méthyl}-2,3,4,4a,5,6-hexahydro-1H-pyrazino[1,2-*a*]quinolin-8-yl)carbonyl]-4-({(1*R*)-3-(diméthylamino)-1-[(phénylsulfanyl)méthyl]propyl}amino)-3-nitrobenzènesulfonamide, trichlorhydrate,
• le *N*-[((4a*R*)-3-{[3'-fluoro-4'-chloro[1,1'-biphényl]-2-yl]méthyl}-2,3,4,4a,5,6-hexahydro-1*H*-pyrazino[1,2-*a*]quinolin-8-yl)carbonyl]-4-({(1*R*)-3-(diméthylamino)-1-[(phénylsulfanyl)méthyl]propyl} amino)-3-nitrobenzènesulfonamide,
• le *N*-[((4a*R*)-3-{[4'-(trifluorométhyl)[1,1'-biphényl]-2-yl]méthyl}-2,3,4,4a,5,6-hexahydrc-1*H*-pyrazino[1,2-*a*]quinolin-8-yl)carbonyl]-4-({(1*R*)-3-(diméthylamino)-1-[(phénylsulfanyl)méthyl]propyl}amino)-3-nitrobenzènesulfonamide,
• le *N*-[((4a*R*)-3-{[4'-cyano[1,1'-biphényl]-2-yl]méthyl}-2,3,4,4a,5,6-hexahydro-1*H-*pyrazino[1,2-*a*]quinolin-8-yl)carbonyl]-4-({(1*R*)-3-(diméthylamino)-1-[(phénylsulfanyl)méthyl]propyl}amino)-3-nitrobenzènesulfonamide,
• le *N*-({(4a*R*)-3-[2-(1,3-benzodioxol-5-yl)benzyl]-2,3,4,4a,5,6-hexahydro-1*H-*pyrazino[1,2-*a*]quinolin-8-yl}carbonyl)-4-({(1*R*)-3-(diméthylamino)-1-[(phénylsulfanyl)méthyl]propyl}amino)-3-nitrobenzènesulfonamide,
• le *N*-({(4a*R*)-3-[(4'-chloro[1,1'-biphényl]-2-yl)méthyl]-2,3,4,4a,5,6-hexahydro-1*H-*pyrazino[1,2-*a*]quinolin-8-yl}carbonyl)-4-({(1*R*)-3-(4-morpholinyl)-1-[(phénylsulfanyl)méthyl]propyl}amino)-3-nitrobenzènesulfonamide,
• le *N*-({(4a*R*)-3-[(4'-chloro[1,1'-biphényl]-2-yl)méthyl]-2,3,4,4a,5,6-hexahydro-1*H-*pyrazino[1,2-*a*]quinolin-8-yl}carbonyl)-4-({(1*R*)-3-(4-morpholinyl)-1-[(phénylsulfanyl)méthyl]propyl}amino)-3-[(trifluorométhyl)sulfonyl] benzènesulfonamide,
• le *N*-({(4a*R*)-3-[(4'-chloro[1,1'-biphényl]-2-yl)méthyl]-2,3,4,4a,5,6-hexahydro-1*H-*pyrazino[1,2-*a*]quinolin-8-yl}carbonyl)-4-({(1*R*)-3-(4-méthyl-1-pipérazinyl)-1-[(phénylsulfanyl)méthyl]propyl}amino)-3-nitrobenzènesulfonamide,
• le *N*-({(4a*R*)-3-[(4'-chloro[1,1'-biphényl]-2-yl)méthyl]-2,3,4,4a,5,6-hexahydro-1*H-*pyrazino[1,2-*a*]quinolin-8-yl}carbonyl)-4-({(1*R*)-3-(1-pipéridyl)-1-[(phénylsulfanyl)méthyl]propyl}amino)-3-nitrobenzènesulfonamide,
• le *N*-({(4a*R*)-3-[(4'-chloro[1,1'-biphényl]-2-yl)méthyl]-2,3,4,4a,5,6-hexahydro-1*H-*pyrazino[1,2-*a*]quinolin-8-yl}carbonyl)-4-({(1*R*)-3-(1-pyrtolidinyl)-1-[(phénylsulfanyl)méthyl]propyl}amino)-3-nitrobenzènesulfonamide,
• le *N*-({(4a*R*)-3-[(4'-chloro[1,1'-biphényl]-2-yl)méthyl]-2,3,4,4a,5,6-hexahydro-1*H-*pyrazino[1,2-*a*]quinolin-8-yl}carbonyl)-4-({(1*R*)-3-(3,6-dihydro-1(2*H*)-pyridyl)-1-[(phénylsulfanyl)méthyl]propyl} amino)-3-nitrobenzènesulfonamide,
• le *N*-({(4a*R*)-3-[(4'-chloro[1,1'-biphényl]-2-yl)méthyl]-2,3,4,4a,5,6-hexahydro-1*H-*pyrazino[1,2-*a*]quinolin-8-yl}carbonyl)-4-({(1*R*)-3-(1-azépanyl)-1-[(phénylsulfanyl)méthyl]propyl} amino)-3-nitrobenzènesulfonamide,
• le *N*-({(4a*R*)-3-[(4'-chloro[1,1'-biphényl]-2-yl)méthyl]-2,3,4,4a,5,6-hexahydro-1*H-*pyrazino[1,2-*a*]quinolin-8-yl}carbonyl)-4-({(1*R*)-3-((1*R*,5*S*)-3-azabicyclo[3.1.0]hex-3-yl)-1-[(phénylsulfanyl)méthyl]propyl}amino)-3-nitrobenzènesulfonamide,
ainsi que leurs sels d'addition à un acide ou à une base pharmaceutiquement acceptable.

11. Composé de formule (I) selon la revendication 1 qui est le *N*-({(4a*R*)-3-[(4'-chloro[1,1'-biphényl]-2-yl)méthyl]-2,3,4,4a,5,6-hexahydro-1*H*-pyrazino[1,2-*a*]quinolin-8-yl}carbonyl)-4-({(1*R*)-3-(diméthylamino)-1-[(phénylsulfanyl)méthyl]propyl}amino)-3-nitrobenzènesulfonamide, ainsi que ses sels d'addition à un acide ou à une base pharmaceutiquement acceptable.

12. Composé de formule (I) selon la revendication 1 qui est le *N*-({(4a*R*)-3-[(4'-chloro[1,1'-biphényl]-2-yl)méthyl]-2,3,4,4a,5,6-hexahydro-1*H*-pyrazino[1,2-*a*]quinolin-8-yl}carbonyl)-4-({(1*R*)-3-(diméthylamino)-1-[(phénylsulfanyl)méthyl]propyl}amino)-3-nitrobenzènesulfonamide, bis(chlorhydrate).

13. Composé de formule (I) selon la revendication 1 qui est le *N*-({(4a*R*)-3-[(4'-chloro[1,1'-biphényl]-2-yl)méthyl]-2,3,4,4a,5,6-hexahydro-1*H*-pyrazino[1,2-*a*]quinolin-8-yl}carbonyl)-4-({(1*R*)-3-(diméthylamino)-1-[(phénylsulfanyl)méthyl]propyl}amino)-3-nitrobenzènesulfonamide, sodium.

14. Procédé de préparation des composés de formule (I) **caractérisé en ce que** l'on utilise comme produit de départ le composé de formule (III) : 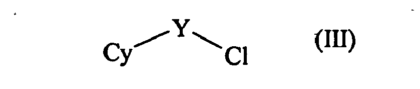 dans laquelle Y est tel que défini dans la formule (I) et Cy représente un système tricyclique fusionné de formule (IV) : 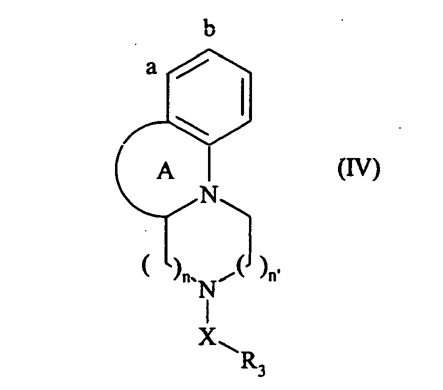 dans laquelle A, X, n, n' et R₃, sont tels que définis dans la formule (I), le groupement -Y-Cl étant rattaché en position a ou b du système tricyclique ainsi défini,
composé de formule (III) sur lequel on condense en milieu basique en présence ou non d'un agent de couplage, le composé de formule (V) : 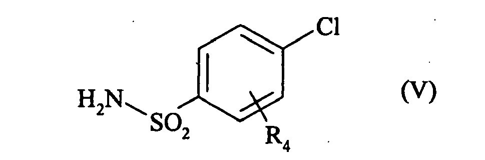 dans laquelle R₄ est tel que défini dans la formule (I),
pour obtenir le composé de formule (VI) : 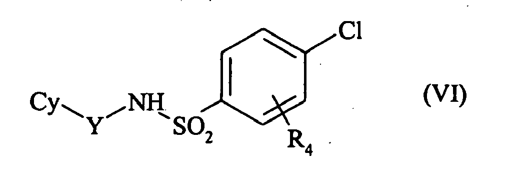 dans laquelle Cy, Y et R₄ sont tels que définis précédemment,
sur lequel on condense le composé de formule HNR₇R'₇ dans laquelle R₇ et R'₇ sont tels que définis dans la formule (I) pour conduire au composé de formule (I/a), cas particulier des composés de formule (I) : 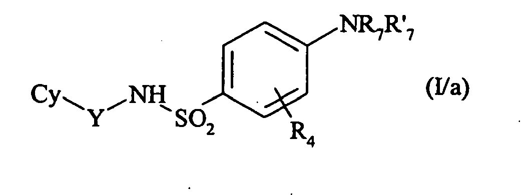 dans laquelle Cy, Y, R₄, R₇ et R'₇ sont tels que définis précédemment,
qui peut être purifié selon une technique classique de séparation, que l'on transforme, si on le souhaite, en ses sels d'addition à un acide ou à une base pharmaceutiquement acceptable et dont on sépare éventuellement les isomères selon une technique classique de séparation.

15. Procédé de préparation des composés de formule (I) **caractérisé en ce que** l'on utilise comme produit de départ le composé de formule (III') : 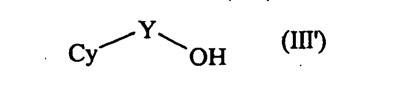 dans laquelle Y est tel que défini dans la formule (I) et Cy représente un système tricyclique fusionné de formule (IV) : 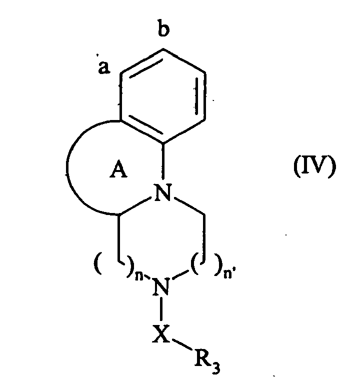 dans laquelle A, X, R₃, n et n' sont tels que définis dans la formule (I), le groupement -Y-OH étant rattaché en position a ou b du système tricyclique ainsi défini,
composé de formule (III') sur lequel on condense en milieu basique en présence d'un agent de couplage, le composé de formule (VII) : 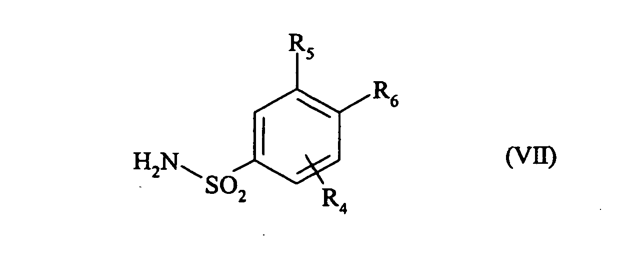 dans laquelle R₄, R₅ et R₆ sont tels que définis dans la formule (I),
pour conduire au composé de formule (I) qui peut être purifié selon une technique classique de séparation, que l'on transforme, si on le souhaite, en ses sels d'addition à un acide ou à une base pharmaceutiquement acceptable et dont on sépare éventuellement les isomères selon une technique classique de séparation.

16. Compositions pharmaceutiques contenant les composés de formule (I) selon l'une quelconque des revendications 1 à 13 ou un de leurs sels d'addition avec un acide ou une base pharmaceutiquement acceptable en combinaison avec un ou plusieurs excipients pharmaceutiquement acceptables.

17. Compositions pharmaceutiques selon la revendication 16 utiles pour la fabrication de médicaments en tant qu'agents pro-apoptotiques.

18. Compositions pharmaceutiques selon la revendication 16 utiles pour la fabrication de médicaments utiles dans le traitement des cancers.

19. Compositions pharmaceutiques selon la revendication 16 utiles pour la fabrication de médicaments utiles dans le traitement des cancers de la vessie, du cerveau, du sein, de l'utérus, des leucémies lymphoïdes chroniques, des cancers du colon, de l'oesophage, du foie, des leucémies lymphoblastiques, des lymphomes folliculaires, des mélanomes, des hémopathies malignes, des myélomes, des cancers de l'ovaire, des cancers du poumon non à petites cellules, des cancers de la prostate et des cancers du poumon à petites cellules.

20. Association d'un composé de formule (I) selon l'une quelconque des revendications 1 à 13 avec un agent anticancéreux choisi parmi les agents génotoxiques, les poisons mitotiques, les anti-métabolites, les inhibiteurs du protéasome, ou les inhibiteurs de kinases.

21. Utilisation d'une association selon la revendication 20 pour la fabrication de médicaments utiles dans le traitement des cancers.

22. Composé de formule (I) selon l'une quelconque des revendications 1 à 13 en association avec une radiothérapie pour son utilisation dans le traitement des cancers.

## Claims

1. Compounds of formula (I) : 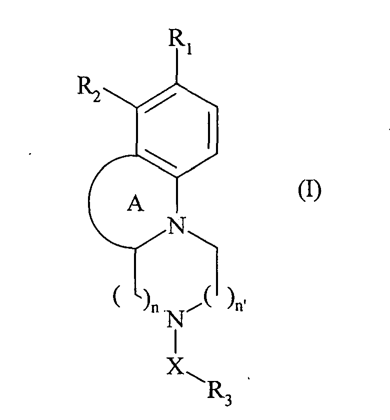 wherein :
◆ A represents a 5, 6 or 7-membered aromatic or non-aromatic ring which may contain 1 or 2 hetero atoms selected from oxygen, sulphur and nitrogen, it being possible for the latter to be substituted by a linear or branched (C₁-C₆)alkyl group, it being understood that the ring A so defined cannot contain 2 sulphur atoms or 2 oxygen atoms and that one of the ring members may be a C=O group,
◆ n and n', which may be identical or different, represent 0, 1 or 2, where 0 < n +n' < 4,
◆ R₃ represents an aryl or heteroaryl group,
◆ X represents a linear or branched alkylene chain containing from 1 to 6 carbon atoms, one or two of which carbon atoms may be replaced by an oxygen atom or by a cycloalkylene, arylene, heteroarylene or SO₂ group,
◆ one of the groups R₁ and R₂ represents a hydrogen atom and the other represents a group of formula (II) : 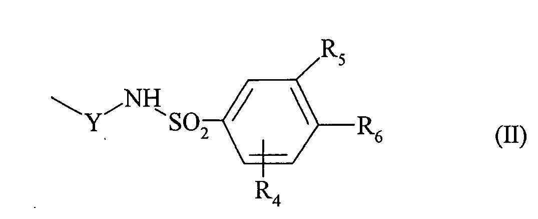
wherein :
- Y represents a C=O or CH₂ group,
- R₅ represents a hydrogen atom in which case R₆ represents a hydrogen atom or a NR₇R'₇ or -CH₂-NR₇R'₇ group wherein each of R₇ and R'₇, which may be identical or different, independently of the other represents a hydrogen atom or a linear or branched (C₁-C₆)alkyl group substituted by one or more aryl, heteroaryl, aryloxy, heteroaryloxy, arylthio, heteroarylthio, heterocycloalkyl or NR₁₀R'₁₀ groups wherein :
* R₁₀ and R'₁₀, which may be identical or different, are selected from hydrogen, linear or branched (C₁-C₆)alkyl, linear or branched (C₁-C₆)alkoxy, aryl and heteroaryl, or
*R₁₀ and R'₁₀ form a saturated or unsaturated cyclic or bicyclic group which may be substituted by a hetero atom selected from oxygen, nitrogen and sulphur, it being understood that one or more of the ring members may represent a C=O group or may be substituted as indicated in the definition of a heterocycle given below,
or R₅ and R₆ form with the two carbon atoms carrying them an aromatic or non-aromatic ring containing 5 or 6 ring members, one nitrogen atom of which being in the position *para* to the SO₂ group, and which may contain in addition to the nitrogen atom a further nitrogen atom and/or a SO₂ group, the ring so defined being substituted by an R₇ group as defined above,
- R₄ represents a halogen atom or an NO₂, R₈, SO₂-R₉, linear or branched (C₁-C₆)alkyl or linear or branched (C₁-C₆)alkoxy group, wherein R₈ may have any of the values of R₇ as defined above,
- R₉ represents an amino group or a linear or branched (C₁-C₆)alkyl group optionally substituted by one or more halogen atoms,
it being understood that :
- "aryl" is understood to mean a phenyl, naphthyl or biphenyl group,
- "heteroaryl" is understood to mean any mono- or bi-cyclic group having at least one aromatic moiety and containing from 5 to 10 ring members and which may contain from 1 to 3 hetero atoms selected from oxygen, sulphur and nitrogen, such as the groups furan, thiophene, pyrrole, imidazoline, pyridine, quinoline, isoquinoline, chroman, indole, benzothiophene, benzofuran, 1,3-benzodioxole and 2,3-dihydro-1,4-benzodioxine,
- "heterocycloalkyl" is understood to mean any mono- or bi-cyclic non-aromatic group containing from 4 to 10 ring members and which may contain from 1 to 3 hetero atoms selected from oxygen, sulphur and nitrogen,
- "cycloalkyl" is understood to mean any mono- or bi-cyclic non-aromatic group containing from 4 to 10 ring members,
it being possible for the aryl, heteroaryl, heterocycloalkyl and cycloalkyl groups so defined to be substituted by from 1 to 3 groups selected from linear or branched (C₁-C₆)alkyl optionally substituted by a hydroxy or amino group, linear or branched (C₁-C₆)alkoxy, hydroxy, carboxy, formyl, nitro, cyano, amino, linear or branched polyhalo-(C₁-C₆)alkyl, alkoxycarbonyl and halogen atoms,
- "arylene", "heteroarylene" and "cycloalkylene" are understood to mean, respectively, an aryl, heteroaryl or cycloalkyl group as defined above, replacing a carbon atom of the alkylene chain,
their enantiomers and diastereoisomers, and addition salts thereof with a pharmaceutically acceptable acid or base.

2. Compounds of formula (I) according to claim 1 wherein Y represents a C=O group, their enantiomers and diastereoisomers and addition salts thereof with a pharmaceutically acceptable acid or base.

3. Compounds of formula (I) according to claim 1 wherein n and n' represent 1, their enantiomers and diastereoisomers and addition salts thereof with a pharmaceutically acceptable acid or base.

4. Compounds of formula (I) according to claim 1 wherein R₄ represents a NO₂ or SO₂-CF₃ group, their enantiomers and diastereoisomers and addition salts thereof with a pharmaceutically acceptable acid or base.

5. Compounds of formula (I) according to claim 1 wherein X-R₃ represents a ([1,1'-biphenyl]-2-yl)methyl group optionally substituted by one or more groups selected from halogen, cyano, amino, aminomethyl and trifluoromethyl, their enantiomers and diastereoisomers and addition salts thereof with a pharmaceutically acceptable acid or base.

6. Compounds of formula (I) according to claim 1 wherein R₅ represents a hydrogen atom, their enantiomers and diastereoisomers and addition salts thereof with a pharmaceutically acceptable acid or base.

7. Compounds of formula (I) according to claim 1 wherein R₇ represents the 1-(*N,N-*dimethylamino)-4-(phenylsulphanyl)-butan-3-yl group, their enantiomers and diastereoisomers and addition salts thereof with a pharmaceutically acceptable acid or base.

8. Compounds of formula (I) according to claim 1 wherein R₇ represents a 1-(NR₁₀R'₁₀)-4-(phenylsulphanyl)-butan-3-yl group, R₁₀ and R'₁₀ being such that they form a saturated or unsaturated cyclic or bicyclic group, optionally substituted by a hetero atom selected from oxygen, nitrogen and sulphur, their enantiomers and diastereoisomers and addition salts thereof with a pharmaceutically acceptable acid or base.

9. Compounds of formula (I) according to claim 1 wherein R'₇ represents a hydrogen atom, their enantiomers and diastereoisomers and addition salts thereof with a pharmaceutically acceptable acid or base.

10. Compounds of formula (I) which are :
• N-({(4a*R*)-3-[(4'-chloro-[1,1'-biphenyl]-2-yl)methyl]-2,3,4,4a,5,6-hexahydro-1*H-*pyrazino[1,2-*a*]quinolin-8-yl}carbonyl)-4-({(1*R*)-3-(dimethylamino)-1-[(phenylsulphanyl)methyl]propyl}amino)-3-nitrobenzenesulphonamide,
• N-({(10aα)-2-[(4'-chloro-[1,1'-biphenyl]-2-yl)methyl]-1,2,3,4,10,10a-hexahydropyrazino[1,2-*a*]indol-8-yl}carbonyl)-4-({(1*R*)-3-(dimethylamino)-1-[(phenylsulphanyl)methyl]propyl}amino)-3-nitrobenzenesulphonamide,
• N-({(10aβ)-2-[(4'-chloro-[1,1'-biphenyl]-2-yl)methyl]-1,2,3,4,10,10a-hexahydropyrazino[1,2-*a*]indol-8-yl}carbonyl)-4-({(1*R*)-3-(dimethylamino)-1-[(phenylsulphanyl)methyl]propyl}amino)-3-nitrobenzenesulphonamide,
• N-({(10aα)-2-[(4'-chloro-[1,1'-biphenyl]-2-yl)methyl]-1,2,3,4,10,10a-hexahydropyrazino[1,2-*a*]indol-8-yl}carbonyl)-4-({(1*R*)-3-(4-morpholinyl)-1-[(phenylsulphanyl)methyl]propyl}amino)-3-nitrobenzenesulphonamide,
• N-({(10aα)-2-[(4'-chloro-[1,1'-biphenyl]-2-yl)methyl]-1,2,3,4,10,10a-hexahydropyrazino[1,2-*a*]indol-8-yl}carbonyl)-4-({(1*R*)-3-(4-morpholinyl)-1-[(phenylsulphanyl)methyl]propyl}amino)-3-[(trifluoromethyl)sulphonyl]benzenesulphonamide,
• N-[((4a*R*)-3-{[2-(4-chlorophenyl)-5,5-dimethyl-1-cyclohexen-1-yl]methyl}-2,3,4,4a,5,6-hexahydro-1*H*-pyrazino[1,2-*a*]quinolin-8-yl)carbonyl]-4-({(1*R*)-3-(4-morpholinyl)-1-[(phenylsulphanyl)methyl]propyl} amino)-3-[(trifluoromethyl)-sulphonyl]benzenesulphonamide,
• N-[((10aβ)-2-{[2-(4-chlorophenyl)-5,5-dimethyl-1-cyclohexen-1-yl]methyl}-1,2,3,4,10,10a-hexahydropyrazino[1,2-*a*]indol-8-yl)carbonyl]-4-({(1*R*)-3-(4-morpholinyl)-1-[(phenylsulphanyl)methyl]propyl}amino)-3-[(trifluoromethyl)-sulphonyl]benzenesulphonamide,
• N-[((4a*R*)-3-{[4-(4-chlorophenyl)-3-pyridyl]methyl}-2,3,4,4a,5,6-hexahydro-1*H-*pyrazino[1,2-*a*]quinolin-8-yl)carbonyl]-4-({(1*R*)-2-(dimethylamino)-1-[(phenylsulphanyl)methyl]ethyl}amino)-3-nitrobenzenesulphonamide,
• N-({(4a*R*)-3-[(4-amino-4'-chloro-[1,1'-biphenyl]-2-yl)methyl]-2,3,4,4a,5,6-hexahydro-1*H*-pyrazino[1,2-*a*]quinolin-8-yl}carbonyl)-4-({(1*R*)-3-(dimethylamino)-1-[(phenylsulphanyl)methyl]propyl}amino)-3-nitrobenzenesulphonamide,
• N-[((4a*R*)-3-{[4-(aminomethyl)-4'-chloro-[1,1'-biphenyl]-2-yl]methyl}-2,3,4,4a,5,6-hexahydro-1*H*-pyrazino[1,2-*a*]quinolin-8-yl)carbonyl]-4-({(1*R*)-3-(dimethylamino)-1-[(phenylsulphanyl)methyl]propyl}amino)-3-nitrobenzenesulphonamide trihydrochloride,
• N-[((4a*R*)-3-{[3'-fluoro-4'-chloro-[1,1'-biphenyl]-2-yl]methyl}-2,3,4,4a,5,6-hexahydro-1*H*-pyrazino[1,2-*a*]quinolin-8-yl)carbonyl]-4-({(1*R*)-3-(dimethylamino)-1-[(phenylsulphanyl)methyl]propyl}amino)-3-nitrobenzenesulphonamide,
• N-[((4a*R*)-3-{[4'-(trifluoromethyl)-[1,1'-biphenyl]-2-yl]methyl}-2,3,4,4a,5,6-hexahydro-1*H*-pyrazino[1,2-*a*]quinolin-8-yl)carbonyl]-4-({(1*R*)-3-(dimethylamino)-1-[(phenylsulphanyl)methyl]propyl}amino)-3-nitrobenzenesulphonamide,
• N-[((4a*R*)-3-{[4'-cyano-[1,1'-biphenyl]-2-yl]methyl}-2,3,4,4a,5,6-hexahydro-1*H-*pyrazino[1,2-*a*]quinolin-8-yl)carbonyl]-4-({(1*R*)-3-(dimethylamino)-1-[(phenylsulphanyl)methyl]propyl}amino)-3-nitrobenzenesulphonamide,
• N-({(4a*R*)-3-[2-(1,3-benzodioxol-5-yl)benzyl]-2,3,4,4a,5,6-hexahydro-1*H-*pyrazino[1,2-*a*]quinolin-8-yl}carbonyl)-4-({(1*R*)-3-(dimethylamino)-1-[(phenylsulphanyl)methyl]propyl}amino)-3-nitrobenzenesulphonamide,
• N-({(4a*R*)-3-[(4'-chloro-[1,1'-biphenyl]-2-yl)methyl]-2,3,4,4a,5,6-hexahydro-1*H-*pyrazino[1,2-*a*]quinolin-8-yl}carbonyl)-4-({(1*R*)-3-(4-morpholinyl)-1-[(phenylsulphanyl)methyl]propyl}amino)-3-nitrobenzenesulphonamide,
• N-({(4a*R*)-3-[(4'-chloro-[1,1'-biphenyl]-2-yl)methyl]-2,3,4,4a,5,6-hexahydro-1*H-*pyrazino[1,2-*a*]quinolin-8-yl}carbonyl)-4-({(1*R*)-3-(4-morpholinyl)-1-[(phenylsulphanyl)methyl]propyl}amino)-3-[(trifluoromethyl)sulphonyl]-benzenesulphonamide,
• N-({(4a*R*)-3-[(4'-chloro-[1,1'-biphenyl]-2-yl)methyl]-2,3,4,4a,5,6-hexahydro-1*H-*pyrazino[1,2-*a*]quinolin-8-yl}carbonyl)-4-({(1*R*)-3-(4-methyl-1-piperazinyl)-1-[(phenylsulphanyl)methyl]propyl} amino)-3-nitrobenzenesulphonamide,
• N-({(4a*R*)-3-[(4'-chloro-[1,1'-biphenyl]-2-yl)methyl]-2,3,4,4a,5,6-hexahydro-1*H-*pyrazino[1,2-*a*]quinolin-8-yl}carbonyl)-4-({(1*R*)-3-(1-piperidyl)-1-[(phenylsulphanyl)methyl]propyl} amino)-3-nitrobenzenesulphonamide,
• N-({(4a*R*)-3-[(4'-chloro-[1,1'-biphenyl]-2-yl)methyl]-2,3,4,4a,5,6-hexahydro-1*H-*pyrazino[1,2-*a*]quinolin-8-yl}carbonyl)-4-({(1*R*)-3-(1-pyrrolidinyl)-1-[(phenylsulphanyl)methyl]propyl}amino)-3-nitrobenzenesulphonamide,
• N-({(4a*R*)-3-[(4'-chloro-[1,1'-biphenyl]-2-yl)methyl]-2,3,4,4a,5,6-hexahydro-1*H-*pyrazino[1,2-*a*]quinolin-8-yl}carbonyl)-4-({(1*R*)-3-(3,6-dihydro-1(2*H*)-pyridyl)-1-[(phenylsulphanyl)methyl]propyl}amino)-3-nitrobenzenesulphonamide,
• N-({(4a*R*)-3-[(4'-chloro-[1,1'-biphenyl]-2-yl)methyl]-2,3,4,4a,5,6-hexahydro-1*H-*pyrazino[1,2-*a*]quinolin-8-yl}carbonyl)-4-({(1*R*)-3-(1-azepanyl)-1-[(phenylsulphanyl)methyl]propyl}amino)-3-nitrobenzenesulphonamide,
• N-({(4a*R*)-3-[(4'-chloro-[1,1'-biphenyl]-2-yl)methyl]-2,3,4,4a,5,6-hexahydro-1*H-*pyrazino[1,2-*a*]quinolin-8-yl}carbonyl)-4-({(1*R*)-3-((1*R*,5*S*)-3-azabicyclo-[3.1.0]hex-3-yl)-1-[(phenylsulphanyl)methyl]propyl}amino)-3-nitrobenzenesulphonamide,
and addition salts thereof with a pharmaceutically acceptable acid or base.

11. Compound of formula (I) according to claim 1 which is *N*-({(4a*R*)-3-[(4'-chloro-[1,1'-biphenyl]-2-yl)methyl]-2,3,4,4a,5,6-hexahydro-1*H*-pyrazino[1,2-*a*]quinolin-8-yl}-carbonyl)-4-({(1*R*)-3-(dimethylamino)-1-[(phenylsulphanyl)methyl]propyl}amino)-3-nitrobenzenesulphonamide, and addition salts thereof with a pharmaceutically acceptable acid or base.

12. Compound of formula (I) according to claim 1 which is *N*-({(4a*R*)-3-[(4'-chloro-[1,1'-biphenyl]-2-yl)methyl]-2,3,4,4a,5,6-hexahydro-1*H*-pyrazino[1,2-*a*]quinolin-8-yl}-carbonyl)-4-({(1*R*)-3-(dimethylamino)-1-[(phenylsulphanyl)methyl]propyl}amino)-3-nitrobenzenesulphonamide bis(hydrochloride).

13. Compound of formula (I) according to claim 1 which is sodium *N*-({(4a*R*)-3-[(4'-chloro-[1,1'-biphenyl]-2-yl)methyl]-2,3,4,4a,5,6-hexahydro-1*H*-pyrazino[1,2-*a*]quinolin-8-yl}-carbonyl)-4-({(1*R*)-3-(dimethylamino)-1-[(phenylsulphanyl)methyl]propyl}amino)-3-nitrobenzenesulphonamide.

14. Process for the preparation of compounds of formula (I), **characterised in that** there is used as starting material a compound of formula (III) : 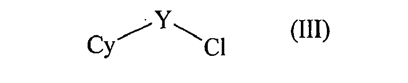 wherein Y is as defined for formula (I) and Cy represents a fused tricyclic system of formula (IV) : 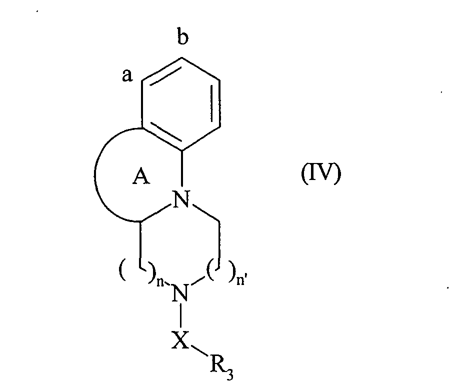 wherein A, X, n, n' and R₃ are as defined for formula (I), the -Y-Cl group being attached in the a or b position of the tricyclic system so defined,
which compound of formula (III) is condensed in a basic medium in the presence or absence of a coupling agent with a compound of formula (V) : 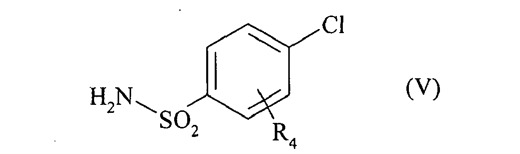 wherein R₄ is as defined for formula (I),
to obtain a compound of formula (VI) : 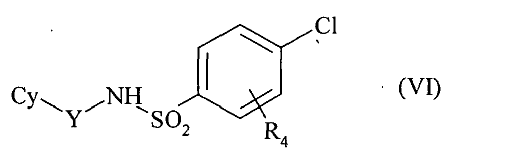 wherein Cy, Y and R₄ are as defined hereinbefore,
which is condensed with a compound of formula HNR₇R'₇ wherein R₇ and R'₇ are as defined for formula (I) to yield a compound of formula (I/a), a particular case of the compounds of formula (I) : 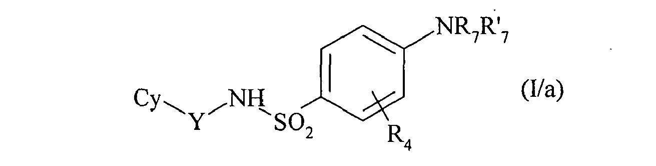 wherein Cy, Y, R₄, R₇ and R'₇ are as defined hereinbefore,
which may be purified according to a conventional separation technique, which is converted, if desired, into addition salts thereof with a pharmaceutically acceptable acid or base and which is optionally separated into its isomers according to a conventional separation technique.

15. Process for the preparation of compounds of formula (I), **characterised in that** there is used as starting material a compound of formula (III') : 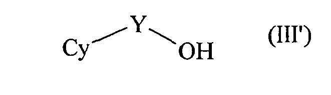 wherein Y is as defined for formula (I) and Cy represents a fused tricyclic system of formula (IV) : 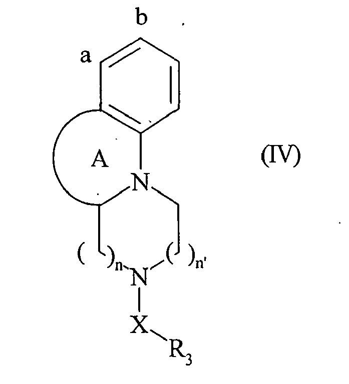 wherein A, X, R₃, n and n' are as defined for formula (I), the -Y-OH group being attached in the a or b position of the tricyclic system so defined,
which compound of formula (III') is condensed in a basic medium in the presence of a coupling agent with a compound of formula (VII) : 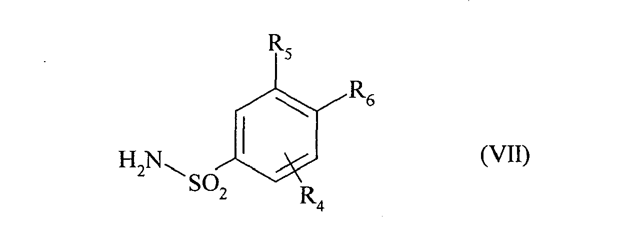 wherein R₄, R₅ and R₆ are as defined for formula (I),
to yield a compound of formula (I) which may be purified according to a conventional separation technique, which is converted, if desired, into addition salts thereof with a pharmaceutically acceptable acid or base and which is optionally separated into its isomers according to a conventional separation technique.

16. Pharmaceutical compositions comprising compounds of formula (I) according to any one of claims 1 to 13 or an addition salt thereof with a pharmaceutically acceptable acid or base in combination with one or more pharmaceutically acceptable excipients.

17. Pharmaceutical compositions according to claim 16 for use in the manufacture of medicaments as pro-apoptotic agents.

18. Pharmaceutical compositions according to claim 16 for use in the manufacture of medicaments for use in the treatment of cancers.

19. Pharmaceutical compositions according to claim 16 for use in the manufacture of medicaments for use in the treatment of cancers of the bladder, brain, breast and uterus, chronic lymphoid leukaemias, cancers of the colon, oesophagus and liver, lymphoblastic leukaemias, follicular lymphomas, melanomas, malignant haemopathies, myelomas, ovarian cancers, non-small-cell lung cancers, prostate cancers and small-cell lung cancers.

20. Combination of a compound of formula (I) according to any one of claims 1 to 13 with an anti-cancer agent selected from genotoxic agents, mitotic poisons, anti-metabolites, proteasome inhibitors and kinase inhibitors.

21. Use of a combination according to claim 20 in the manufacture of medicaments for use in the treatment of cancers.

22. Compound of formula (I) according to any one of claims 1 to 13 in combination with radiotherapy for use in the treatment of cancers.

## Patentansprüche

1. Verbindungen der Formel (I): 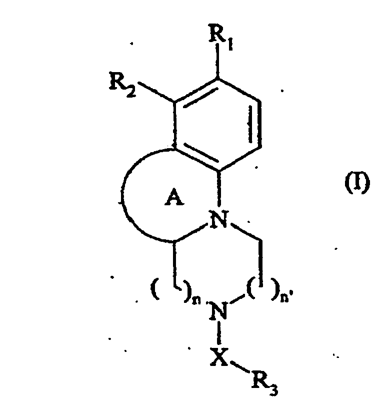 in der:
◆ A einen aromatischen oder nichtaromatischen Ring mit 5, 6 oder 7 Kettengliedern bedeutet, der 1 oder 2 Heteroatome ausgewählt aus Sauerstoff, Schwefel und Stickstoff enthalten kann, wobei letzteres durch eine geradkettige oder verzweigte (C₁-C₆)-Alkylgruppe substituiert sein kann,
wobei es sich versteht, dass der in dieser Weise definierte Ring A nicht 2 Schwefelatome noch 2 Sauerstoffatome enthalten kann und dass eines der Kettenglieder eine Gruppe C=O darstellen kann,
◆ n und n', die gleichartig oder verschieden sind, 0, 1 oder 2 mit der Maßgabe bedeuten, dass 0 < n + n' < 4 ist,
◆ R₃ eine Aryl- oder Heteroarylgruppe bedeutet,
◆ X eine geradkettige oder verzweigte Alkylenkette, die 1 bis 6 Kohlenstoffatome enthält, wobei ein oder zwei der Kohlenstoffatome durch ein Sauerstoffatom ersetzt sein können, eine Cycloalkylengruppe, eine Arylengruppe, eine Heteroarylengruppe oder eine Gruppe SO₂ bedeutet,
◆ eine der Gruppen R₁ oder R₂ ein Wasserstoffatom bedeutet und das andere eine Gruppe der Formel (II) bedeutet: 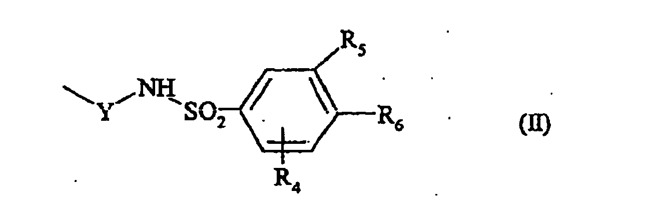
in der:
- Y eine Gruppe C=O oder CH₂ darstellt,
- R₅ ein Wasserstoffatom bedeutet und in diesem Fall R₆ ein Wasserstoffatom oder eine Gruppe -NR₇R'₇ oder -CH₂-NR₇R'₇ bedeutet, worin R₇ und R'₇, die gleichartig oder verschieden sind, jeweils unabhängig voneinander ein Wasserstoffatom oder eine geradkettige oder verzweigte (C₁-C₆)-Alkylgruppe darstellen, die durch ein oder mehrere Aryl-, Heteroaryl-, Aryloxy-, Heteroaryloxy-, Arylthio-, Heteroarylthio-, Heterocycloalkylgruppen oder Gruppen -NR₁₀R'₁₀ subsituiert ist, worin:
* R₁₀ und R'₁₀, die gleichartig oder verschieden sind, ausgewählt sind aus Wasserstoff, geradkettigem oder verzweigtem (C₁-C₆)-Alkyl, geradkettigem oder verzweigtem (C₁-C₆)-Alkoxy, Aryl und Heteroaryl,
* oder R₁₀ und R'₁₀ eine cyclische oder bicyclische, gesättigte oder ungesättigte Gruppe bilden, die gegebenenfalls durch ein Heteroatom ausgewählt aus Sauerstoff, Stickstoff oder Schwefel substituiert ist, wobei es sich versteht, dass eines oder mehrere der Kettenglieder eine Gruppe C=O darstellen kann (können) oder substituiert sein kann (können), wie es die obige Definition des Heterocyclus vorsieht.
oder R₅ und R₆ zusammen mit den sie tragenden beiden Kohlenstoffatomen einen aromatischen oder nichtaromatischen Ring bilden, der 5 oder 6 Kettenglieder enthält, darunter ein Stickstoffatom in der para-Stellung der Gruppe SO₂, und der zusätzlich zu dem Stickstoffatom ein weiteres Stickstoffatom und/oder eine Gruppe SO₂ enthalten kann, wobei der in dieser Weise definierte Ring durch eine Gruppe R₇ substituiert ist, wie sie oben definiert worden ist,
- R₄ ein Halogenatom oder eine Gruppe NO₂, R₈, SO₂-R₉, eine geradkettige oder verzweigte (C₁-C₆)-Alkylgruppe oder eine geradkettige oder verzweigte (C₁-C₆)-Alkoxygruppe bedeutet, worin R₈ sämtliche Bedeutungen von R₇ annehmen kann, wie sie oben definiert worden sind,
- R₉ eine Aminogruppe oder eine geradkettige oder verzweigte (C₁-C₆)-Alkylgruppe bedeutet, die gegebenenfalls durch ein oder mehrere Halogenatome substituiert ist,
mit der Maßgabe, dass man:
- unter "Aryl" eine Phenyl-, Naphthyl- oder Biphenylgruppe versteht,
- unter "Heteroaryl" jede mono- oder bicyclische Gruppe versteht, die mindestens einen aromatischen Teil aufweist und 5 bis 10 Kettenglieder enthält und 1 bis 3 Heteroatome ausgewählt aus Sauerstoff, Schwefel oder Stickstoff enthalten kann, wie die Furan-, Thiophen-, Pyrrol-, Imidazolin-, Pyridin-, Chinolin-, Isochinolin-, Chroman-, Indol-, Benzothiophen-, Benzofuran-, 1,3-Benzodioxol- und 2,3-Dihydro-1,4-benzodioxingruppen,
- unter "Heterocycloalkyl" jede nichtaromatische mono- oder bicyclische Gruppe versteht, die 4 bis 10 Kettenglieder enthält und 1 bis 3 Heteroatome ausgewählt aus Sauerstoff, Schwefel oder Stickstoff enthalten kann,
- unter "Cycloalkyl" jede nichtaromatische mono- oder bicyclische Gruppe mit 4 bis 10 Kettengliedern versteht,
wobei die in dieser Weise definierten Aryl-, Heteroaryl-, Heterocycloalkyl- und Cycloalkylgruppen durch 1 bis 3 Gruppen substituiert sein können ausgewählt aus geradkettigem oder verzweigtem (C₁-C₆)-Alkyl, gegebenenfalls substituiert durch eine Hydroxy- oder Aminogruppe, geradkettigem oder verzweigtem (C₁-C₆)-Alkoxy, Hydroxy, Carboxy, Formyl, Nitro, Cyano, Amino, geradkettigem oder verzweigtem (C₁-C₆)-Polyhalogenalkyl, Alkoxycarbonyl oder Halogenatomen,
- unter "Arylen", "Heteroarylen" und "Cycloalkylen" man eine Aryl-, Heteroaryl- bzw. Cycloalkylgruppe versteht, wie sie oben definiert worden sind, die anstelle eines Kohlenstoffatoms in die Alkylenkette eingefügt sind,
deren Enantiomere und Diastereoisomere sowie deren Additionssalze mit einer pharmazeutisch annehmbaren Säure oder Base.

2. Verbindungen der Formel (I) nach Anspruch 1, worin Y eine Gruppe C=O bedeutet, deren Enantiomere und Diastereoisomere sowie deren Additionssalze mit einer pharmazeutisch annehmbaren Säure oder Base.

3. Verbindungen der Formel (I) nach Anspruch 1, worin n und n' 1 bedeuten, deren Enantiomere und Diastereoisomere sowie deren Additionssalze mit einer pharmazeutisch annehmbaren Säure oder Base.

4. Verbindungen der Formel (I) nach Anspruch 1, worin R₄ eine Gruppe NO₂ oder SO₂-CF₃ bedeutet, deren Enantiomere und Diastereoisomere sowie deren Additionssalze mit einer pharmazeutisch annehmbaren Säure oder Base.

5. Verbindungen der Formel (I) nach Anspruch 1, worin X-R₃ eine ([1,1'-Biphenyl]-2-yl)-methyl-gruppe bedeutet, die gegebenenfalls durch eine oder mehrere Gruppen substituiert ist ausgewählt aus Halogen, Cyano, Amino, Aminomethyl und Trifluormethyl, deren Enantiomere und Diastereoisomere sowie deren Additionssalze mit einer pharmazeutisch annehmbaren Säure oder Base.

6. Verbindungen der Formel (I) nach Anspruch 1, worin R₅ ein Wasserstoffatom bedeutet, deren Enantiomere und Diastereoisomere sowie deren Additionssalze mit einer pharmazeutisch annehmbaren Säure oder Base.

7. Verbindungen der Formel (I) nach Anspruch 1, worin R₇ die 1-(*N,N*-Dimethylamino)-4-(phenylsulfanyl)-butan-3-yl-gruppe bedeutet, deren Enantiomere und Diastereoisomere sowie deren Additionssalze mit einer pharmazeutisch annehmbaren Säure oder Base.

8. Verbindungen der Formel (I) nach Anspruch 1, worin R₇ eine 1-(NR₁₀R'₁₀)-4-(Phenylsulfanyl)-butan-3-yl-gruppe bedeutet, worin R₁₀ und R'₁₀ eine gesättigte oder ungesättigte cyclische oder bicyclische Gruppe bilden, die gegebenenfalls durch ein Heteroatom ausgewählt aus Sauerstoff, Stickstoff oder Schwefel substituiert ist, deren Enantiomere und Diastereoisomere sowie deren Additionssalze mit einer pharmazeutisch annehmbaren Säure oder Base.

9. Verbindungen der Formel (I) nach Anspruch 1, worin R'₇ ein Wasserstoffatom bedeutet, deren Enantiomere und Diastereoisomere sowie deren Additionssalze mit einer pharmazeutisch annehmbaren Säure oder Base.

10. Verbindungen der Formel (I), nämlich:
• *N*-({(4a*R*)-3-[(4'-Chlor-[1,1'-biphenyl]-2-yl)-methyl]-2,3,4,4a,5,6-hexahydro-1*H-*pyrazino[1,2-*a*]chinolin-8-yl}-carbonyl)-4-({(1*R*)-3-(dimethylamino)-1-[(phenylsulfanyl)-methyl]-propyl}-amino)-3-nitrobenzolsulfonamid,
• *N*-({(10aα)-2-[(4'-Chlor-[1,1'-biphenyl]-2-yl)-methyl]-1,2,3,4,10,10a-hexahydropyrazino[1,2-*a*]indol-8-yl}-carbonyl)-4-({(1*R*)-3-(dimethylamino)-1-[(phenylsulfanyl)-methyl]-propyl}-amino)-3-nitrobenzolsulfonamid,
• *N*-({(10aβ)-2-[(4'-Chlor-[1,1'-biphenyl]-2-yl)-methyl]-1,2,3,4,10,10a-hexahydropyrazino[1,2-*a*]indol-8-yl}-carbonyl)-4-({(1*R*)-3-(dimethylamino)-1-[(phenylsulfanyl)-methyl]-propyl}-amino)-3-nitrobenzolsulfonamid,
• *N*-({(10aα)-2-[(4'-Chlor-[1,1'-biphenyl]-2-yl)-methyl]-1,2,3,4,10,10a-hexahydropyrazino[1,2-*a*]indol-8-yl}-carbonyl)-4-({(1*R*)-3-(4-morpholinyl)-1-[(phenylsulfanyl)-methyl]-propyl}-amino)-3-nitrobenzolsulfonamid,
• *N*-({(10aα)-2-[(4'-Chlor-[1,1'-biphenyl]-2-yl)-methyl]-1,2,3,4,10,10a-hexahydropyrazino[1,2-*a*]indol-8-yl}-carbonyl)-4-({(1*R*)-3-(4-morpholinyl)-1-[(phenylsulfanyl)-methyl]-propyl}-amino)-3-[(trifluormethyl)-sulfonyl]-benzolsulfonamid,
• *N*-[((4a*R*)-3-{[2-(4-Chlorphenyl)-5,5-dimethyl-1-cyclohexen-1-yl]-methyl}-2,3,4,4a,5,6-hexahydro-1*H*-pyrazino[1,2-*a*]chinolin-8-yl)-carbonyl]-4-({(1*R*)-3-(4-morpholinyl)-1-[(phenylsulfanyl)-methyl]-propyl}-amino)-3-[(trifluormethyl)-sulfonyl]-benzolsulfonamid,
• *N*-[((10aβ)-2-{[2-(4-Chlorphenyl-5,5-dimethyl-1-cyclohexen-1-yl]-methyl}-1,2,3,4,10,10a-hexahydropyrazino[1,2-*a*]indol-8-yl)-carbonyl]-4-({(1*R*)-3-(4-morpholinyl)-1-[(phenylsulfanyl)-methyl]-propyl}-amino)-3-[(trifluormethyl)-sulfonyl]-benzolsulfonamid,
• *N*-[((4a*R*)-3-{[4-(4-Chlorphenyl)-3-pyridyl]-methyl}-2,3,4,4a,5,6-hexahydro-1*H-*pyrazino[1,2-*a*]chinolin-8-yl)-carbonyl]-4-({(1*R*)-2-(dimethylamino)-1-[(phenylsulfanyl)-methyl]-ethyl}-amino)-3-nitrobenzolsulfonamid,
• *N*-({(4a*R*)-3-[(4-Amino-4'chlor-[1,1'-biphenyl]-2-yl)-methyl]-2,3,4,4a,5,6-hexahydro-1*H*-pyrazino[1,2-*a*]chinolin-8-yl}-carbonyl)-4-({(1*R*)-3-(dimethylamino)-1-[(phenylsulfanyl)-methyl]-propyl}-amino)-3-nitrobenzolsulfonamid,
• *N*-[((4a*R*)-3-{[4-(Aminomethyl)-4'-chlor-[1,1'-biphenyl]-2-yl]-methyl}-2,3,4,4a,5,6-hexahydro-1*H*-pyrazino[1,2-*a*]chinolin-8-yl)-carbonyl]-4-({(1*R*)-3-(dimethylamino)-1-[(phenylsulfanyl)-methyl]-propyl}-amino)-3-nitrobenzolsulfonamid, Trihydrochlorid,
• *N*-[((4a*R*)-3-{[3'-Fluor-4'-chlor-[1,1'-biphenyl]-2-yl]-methyl}-2,3,4,4a,5,6-hexahydro-1*H*-pyrazino[1,2-*a*]chinolin-8-yl)-carbonyl]-4-({(1*R*)-3-(dimethylamino)-1-[(phenylsulfanyl)-methyl]-propyl}-amino)-3-nitrobenzolsulfonamid,
• *N*-[((4a*R*)-3-{[4'-(Trifluormethyl)-[1,1'-biphenyl]-2-yl]-methyl}-2,3,4,4a,5,6-hexahydro-1*H*-pyrazino[1,2-*a*]chinolin-8-yl)-carbonyl]-4-({(1*R*)-3-(dimethylamino)-1-[(phenylsulfanyl)-methyl]-propyl}-amino)-3-nitrobenzolsulfonamid,
• *N*-[((4a*R*)-3-{[4'-Cyano-[1,1'-biphenyl]-2-yl]-methyl}-2,3,4,4a,5,6-hexahydro-1*H-*pyrazino[1,2-*a*]chinolin-8-yl)-carbonyl]-4-({(1*R*)-3-(dimethylamino)-1-[(phenylsulfanyl)-methyl]-propyl}-amino)-3-nitrobenzolsulfonamid,
• *N*-({(4a*R*)-3-[2-(1,3-Benzodioxol-5-yl)-benzyl]-2,3,4,4a,5,6-hexahydro-1*H-*pyrazino[1,2-*a*]chinolin-8-yl}-carbonyl)-4-({(1*R*)-3-(dimethylamino)-1-[(phenylsulfanyl)-methyl]-propyl}-amino)-3-nitrobenzolsulfonamid,
• *N*-[[(4a*R*)-3-[4'Chlor-[1,1'-biphenyl]-2-yl)-methyl]-2,3,4,4a,5,6-hexahydro-1*H-*pyrazino[1,2-*a*]chinolin-8-yl}-carbonyl)-4-({(1*R*)-3-(4-morpholinyl)-1-[(phenylsulfanyl)-methyl]-propyl}-amino)-3-nitrobenzolsulfonamid,
• *N*-({(4a*R*)-3-[4'Chlor-[1,1'-biphenyl]-2-yl)-methyl]-2,3,4,4a,5,6-hexahydro-1*H-*pyrazino[1,2-*a*]chinolin-8-yl}-carbonyl)-4-({(1*R*)-3-(4-morpholinyl)-1-[(phenylsulfanyl)-methyl]-propyl}-amino)-3-[(trifluormethyl)₋sulfonyl]-benzolsulfonamid,
• *N*-{((4a*R*)-3-[(4'-Chlor-[1,1'-biphenyl]-2-yl)-methyl]-2,3,4,4a,5,6-hexahydro-1*H-*pyrazino[1,2-*a*]chinolin-8-yl)-carbonyl)-4-({(1*R*)-3-(4-methyl-1-piperazinyl)-1-[(phenylsulfanyl)-methyl]-propyl}-amino)-3-nitrobenzolsulfonamid,
• *N*-({(4a*R*)-3-[4'-Chlor-[1,1'-biphenyl]-2-yl)-methyl]-2,3,4,4a,5,6-hexahydro-1*H-*pyrazino[1,2-*a*]chinolin-8-yl}-carbonyl)-4-({(1*R*)-3-(1-piperidyl)-1-[(phenylsulfanyl)-methyl]-propyl}-amino)-3-nitrobenzolsulfonamid,
• *N*-({(4a*R*)-3-[(4'-Chlor-[1,1'-biphenyl]-2-yl]-methyl]-2,3,4,4a,5,6-hexahydro-1*H-*pyrazino[1,2-*a*]chinolin-8-yl}-carbonyl)-4-({(1*R*)-3-(pyrrolidinyl)-1-[(phenylsulfanyl)-methyl]-propyl}-amino)-3-nitrobenzolsulfonamid,
• *N*-({(4a*R*)-3-[(4'-Chlor-[1,1'-biphenyl]-2-yl)-methyl]-2,3,4,4a,5,6-hexahydro-1*H-*pyrazino[1,2-*a*]chinolin-8-yl}-carbonyl)-4-({(1*R*)-3-(3,6-dihydro-1(2*H*)-pyridyl)-1-[(phenylsulfanyl)-methyl]-propyl}-amino)-3-nitrobenzolsulfonamid,
• *N*-({(4a*R*)-3-[(4'-Chlor-[1,1'-biphenyl]-2-yl)-methyl]-2,3,4,4a,5,6-hexahydro-1*H-*pyrazino[1,2-*a*]chinolin-8-yl}-carbonyl)-4-({(1*R*)-3-(1-azepanyl)-1-[(phenylsulfanyl)-methyl]-propyl}-amino)-3-nitrobenzolsulfonamid,
• *N*-({(4a*R*)-3-[(4'-Chlor-[1,1'-biphenyl]-2-yl)-methyl]-2,3,4,4a,5,6-hexahydro-1*H-*pyrazino[1,2-a]chinolin-8-yl}-carbonyl)-4-({(1*R*)-3-((1*R*,5*S*)-3-azabicyclo[3.1.0]-hex-3-yl)-1-[(phenylsulfanyl)-methyl]-propyl}-amino)-3-nitrobenzolsulfonamid
sowie deren Additionssalze mit einer pharmazeutisch annehmbaren Säure oder Base.

11. Verbindung der Formel (I) nach Anspruch 1, nämlich N-({(4a*R*)-3-[(4'-Chlor-[1,1'-biphenyl]-2-yl)-methyl]-2,3,4,4a,5,6-hexahydro-1*H*-pyrazino[1,2-*a*]chinolin-8-yl}-carbonyl)-4-({(1*R*)-3-(dimethylamino)-1-[(phenylsulfanyl)-methyl]-propyl}-amino)-3-nitrobenzolsulfonamid sowie dessen Additionssalze mit einer pharmazeutisch annehmbaren Säure oder Base.

12. Verbindung der Formel (I) nach Anspruch 1, nämlich N-({(4a*R*)-3-[(4'-Chlor-[1,1'-biphenyl]-2-yl)-methyl]-2,3,4,4a,5,6-hexahydro-1*H*-pyrazino[1,2-*a*]chinolin-8-yl}-carbonyl)-4-({(1*R*)-3-(dimethylamino)-1-[(phenylsulfanyl)-methyl]-propyl}-amino)-3-nitrobenzolsulfonamid, Bis-Hydrochlorid.

13. Verbindung der Formel (I) nach Anspruch 1, nämlich N-({(4a*R*)-3-[(4'-Chlor-[1,1'-biphenyl]-2-yl)-methyl]-2,3,4,4a,5,6-hexahydro-1*H*-pyrazino[1,2-*a*]chinolin-8-yl}-carbonyl)-4-({(1*R*)-3-(dimethylamino)-1-[(phenylsulfanyl)-methyl]-propyl}-amino)-3-nitrobenzolsulfonamid, Natriumsalz.

14. Verfahren zur Herstellung der Verbindungen der Formel (I), **dadurch gekennzeichnet, dass** man als Ausgangsprodukt die Verbindung der Formel (III) verwendet: 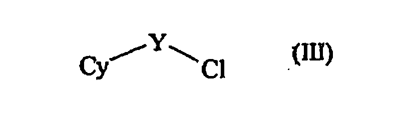 in der Y die bezüglich der Formel (I) angegebenen Bedeutungen besitzt und Cy ein kondensiertes tricyclisches System der Formel (IV) bedeutet: 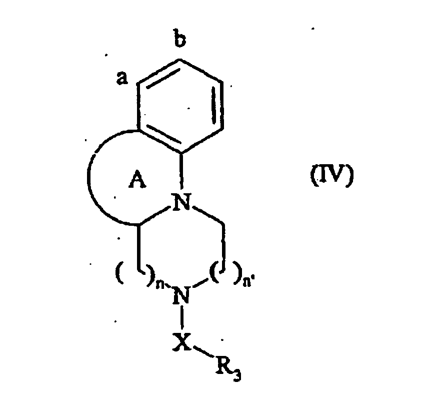 in der A, X, n, n' und R₃ die bezüglich der Formel (I) angegebenen Bedeutungen besitzen, wobei die Gruppe -Y-Cl an der Position a oder b des in dieser Weise definierten tricyclischen Systems gebunden ist,
welche Verbindung der Formel (III) man in basischem Medium und gegebenenfalls in Gegenwart eines Kupplungsmittels mit der Verbindung der Formel (V) kondensiert: 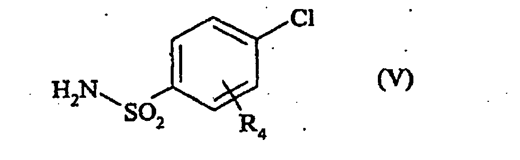 in der R₄ die bezüglich der Formel (I) angegebenen Bedeutungen besitzt,
zur Bildung der Verbindung der Formel (VI): 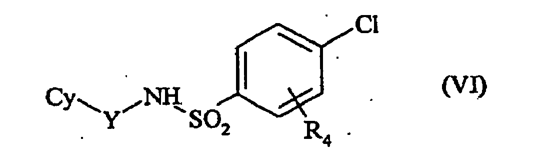 in der Cy, Y und R₄ die oben angegebenen Bedeutungen besitzen,
welche man mit der Verbindung der Formel HNR₇R'₇, in der R₇ und R'₇ die bezüglich der Formel (I) angegebenen Bedeutungen besitzen, kondensiert, zur Bildung der Verbindung der Formel (I/a), einem Sonderfall der Verbindungen der Formel (I): 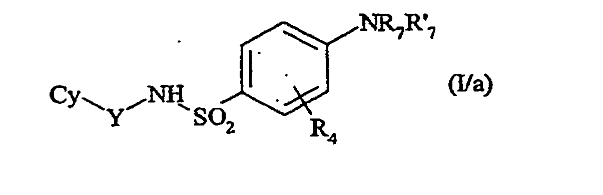 in der Cy, Y, R₄, R₇ und R'₇ die oben angegebenen Bedeutungen besitzen,
welche mit Hilfe einer klassischen Trennmethode gereinigt werden kann, welche man gewünschtenfalls in ihre Additionssalze mit einer pharmazeutisch annehmbaren Säure oder Base überführt und deren Isomere man gegebenenfalls mit Hilfe einer klassischen Trennungsmethode trennt.

15. Verfahren zur Herstellung der Verbindungen der Formel (I), **dadurch gekennzeichnet, dass** man als Ausgangsprodukt die Verbindung der Formel (III') verwendet: 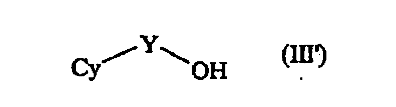 in der Y die bezüglich der Formel (I) angegebenen Bedeutungen besitzt und Cy ein kondensiertes tricyclisches System der Formel (IV) bedeutet: 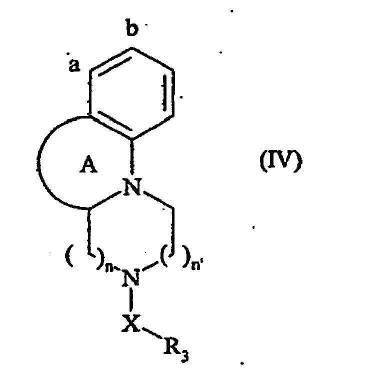 in der A, X, R₃, n und n' die bezüglich der Formel (I) angegebenen Bedeutungen besitzen, wobei die Gruppe -Y-OH in der Position a oder b des in dieser Weise definierten tricyclischen Systems gebunden ist,
welche Verbindung der Formel (III') man in basischem Medium und in Gegenwart eines Kupplungsmittels mit der Verbindung der Formel (VII) kondensiert: 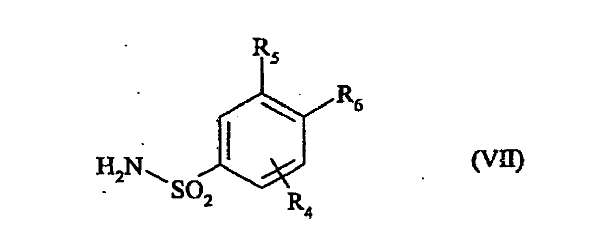 in der R₄, R₅ und R₆ die bezüglich der Formel (I) angegebenen Bedeutungen besitzen,
zur Bildung der Verbindung der Formel (I), welche mit Hilfe einer klassischen Trennungsmethode gereinigt werden kann, welche man gewünschtenfalls in ihre Additionssalze mit einer pharmazeutisch annehmbaren Säure oder Base überführt und deren Isomere man gegebenenfalls mit Hilfe einer klassischen Trennmethode trennt.

16. Pharmazeutische Zubereitungen enthalten die Verbindungen der Formel (I) gemäß einem der Ansprüche 1 bis 13 oder eines ihrer Additionssalze mit einer pharmazeutisch annehmbaren Säure oder Base in Kombination mit einem oder mehreren pharmazeutisch annehmbaren Hilfsstoffen.

17. Pharmazeutische Zubereitungen nach Anspruch 16, nützlich zur Herstellung von Arzneimitteln, die als pro-apoptotische Mittel wirken.

18. Pharmazeutische Zubereitungen nach Anspruch 16, nützlich für die Herstellung von Arzneimitteln, die zur Behandlung von Krebs nützlich sind.

19. Pharmazeutische Zubereitungen nach Anspruch 16, nützlich für die Herstellung von Arzneimitteln, die geeignet sind für die Behandlung von Blasenkrebs, Gehirnkrebs, Brustkrebs, Gebärmutterkrebs, chronischer lymphoider Leukämien, Darmkrebs, Speiseröhrenkrebs, Leberkrebs, lymphoblastischer Leukämien, Follikellymphome, Melanome, bösartige Hämopathien, Myelome, Eierstockkrebs, Lungenkrebs nicht mit kleinen Zellen, Prostatakrebs und Lungenkrebs mit kleinen Zellen.

20. Kombination einer Verbindung der Formel (I) nach einem der Ansprüche 1 bis 13 mit einem Antikrebsmittel ausgewählt aus genotoxischen, mitotoxischen Giften, Anti-metaboliten, Proteasom-Inhibitoren oder Kinase-Inhibitoren.

21. Verwendung einer Kombination nach Anspruch 20 für die Herstellung von Arzneimitteln, die nützlich sind bei der Behandlung von Krebs.

22. Verbindung der Formel (I) nach einem der Ansprüche 1 bis 13 in Kombination mit einer Strahlentherapie für die Anwendung bei der Behandlung von Krebs.
